# EUROPEAN PATENT SPECIFICATION

(11) **EP 1 082 287 B1**
(45) Date of publication and mention of the grant of the patent: **17.03.2004**
(21) Application number: 99917040.0
(22) Date of filing: 17.05.1999
(51) Int. Cl.: C07C 69/76, C07C 69/716, C07C 69/738, C11B 9/00, A61K 7/46

(54) **SLOW RELEASE OF FRAGRANT COMPOUNDS IN PERFUMERY USING 2-BENZOYL BENZOATES, 2-ALKANOYL BENZOATES OR ALPHA-KETO ESTERS**
LANGSAME FREISETZUNG VON RIECHSTOFFVERBINDUNGEN IN PARFÜMEN UNTER ANWENDUNG VON 2-BENZOYLBENZOATEN,2-ALKANOYLBENZOATEN ODER ALPHA-KETOESTERN
COMPOSES DE PARFUMERIE ODORANTS A LIBERATION LENTE CONTENANT DES BENZOATES DE 2-BENZOYLE, DES BENZOATES DE 2-ALCANOYLE OU DES ALPHA-CETOESTERS

(30) Priority: 28.05.1998 US 85593
(43) Date of publication of application: 14.03.2001
(73) Proprietor: FIRMENICH SA, 1211 Genève 8 (CH)
(72) Inventor: PIKA, Jana, Princeton, NJ 08540 (US); HERRMANN, Andreas, CH-1205 Geneva (CH); VIAL, Christian, CH-1202 Geneva (CH)
(74) Representative: Salvaterra-Garcia, Maria de Lurdes, Dr.
(86) International application number: PCT/IB1999/000890
(87) International publication number: WO 1999/060990

(56) References cited:
- PAUL B. JONES ET AL.: "2-Benzoylbenzoic Acid:A Photolabile Mask for Alcohols and Thiols" JOURNAL OF ORGANIC CHEMISTRY., vol. 61, no. 26, 27 December 1996 (1996-12-27), pages 9455-9461, XP002110642 AMERICAN CHEMICAL SOCIETY. EASTON., US ISSN: 0022-3263
- SHENGKUI HU ET AL.: "Photochemical Reactions of Alkenyl Phenylglyoxylates" JOURNAL OF ORGANIC CHEMISTRY., vol. 62, no. 20, 3 October 1997 (1997-10-03), pages 6820-6826, XP002122283 AMERICAN CHEMICAL SOCIETY. EASTON., US ISSN: 0022-3263
- PATENT ABSTRACTS OF JAPAN vol. 18, no. 410 (C-1232), 2 August 1994 (1994-08-02) & JP 06 121822 A (TANIGUSHI KORYO KK), 6 May 1994 (1994-05-06)
- GEORGE A. KRAUS ET AL.: "1,5 and 1,9-Hydrogen Atom Abstractions.Photochemical Strategies for Radical Cyclizations" JOURNAL OF THE AMERICAN CHEMICAL SOCIETY., vol. 114, no. 22, 21 October 1992 (1992-10-21), pages 8705-8707, XP002122284 AMERICAN CHEMICAL SOCIETY, WASHINGTON, DC., US ISSN: 0002-7863
- CHEMICAL ABSTRACTS, vol. 71, no. 23, 8 December 1969 (1969-12-08) Columbus, Ohio, US; abstract no. 112473d, KLIMOVA E.I. ET AL.: "Catalytic enol synthesis with carbonyl compounds.Addition of cyclohexene to n-butyl glyoxylate" page 324; column r; XP002122285 & KLIMOVA E.I. ET AL.: ZH.ORG.KHIM., vol. 5, no. 8, 1969, pages 1345-1348, Moscow, USSR

## Description

The present invention relates to the field of perfumery. It relates, more particularly, to perfuming compositions or perfumed products containing a class of aliphatic or aromatic keto esters of fragrant alcohols, as defined below, which are capable of releasing said fragrant alcohol upon exposure to light, more particularly daylight. The present invention also relates to α-keto esters, as defined below, of alcohols which are precursors of fragrant aldehydes and ketones and which are capable of releasing said fragrant ketone or aldehyde upon exposure to light, more particularly daylight. Said α-keto esters may furthermore contain, in α-position to the keto group, an alkyl group which may contain various substituents and which alkyl group is derived from a fragrant molecule possessing an olefinic unsaturation. The unsaturated molecule and/or the aldehyde or ketone are released upon exposure to light, in particular daylight, of the α-keto ester.

Some compounds of the invention, namely some 2-benzoylbenzoate esters as well as some α-keto esters are known to be photolabile compounds. Therefore, it has been suggested in the prior art to use 2-benzoylbentoate esters as protective groups for alcohols in organic synthesis and subsequently release the alcohol present in the ester function by photolysis (see Porter et al., J. Org. Chem **1996**, *61*, 9455-9461). The authors conducted experiments with different alcohols, and they described the elimination of geraniol from the geranyl 2-benzoyl benzoate (R₁=R₂=R₃=R₄=R₅=H). Moreover, S. Hu and D.C. Neckers in J. Org. Chem. **1997**, 62, 6820-6826, and G.A. Kraus and Y. Wu in J. Am. Chem. Soc. **1992**, 114, 8705-8707 disclose some α-keto ester derivatives within the scope of photolysis studies. On the other hand, some pyruvate esters are known to be active ingredients to enable the removal of amine and mercaptan type odors (Patent Abstracts of Japan, **1994**, 18, 410). However, it has not been described or suggested in the prior art to use the said esters in perfumery, as fragrance delivery systems capable of releasing the fragrant alcohol over a prolonged period of time and thus provide a slow release fragrance effect.

There exists, in perfumery, a particular interest in compounds which are capable of "fixing" fragrant molecules, for example by chemical bonding or intramolecular forces like absorption, and releasing said fragrant molecules over a prolonged period of time, for example by the action of heat, enzymes, or even sunlight. Fragrant molecules have to be volatile in order to be perceived. Although many fragrant compounds are known which show a good substantivity, i.e. they will remain on a surface to which they have been applied for several days and can hence be perceived over such a period of time, a great number of fragrant compounds are very volatile, and their characteristic smell can no longer be perceived several hours after their application.

It is thus desirable to dispose of fragrance delivery systems which are capable of releasing the fragrant compound or compounds in a controlled manner, maintaining a desired smell over a prolonged period of time.

### Description of the Invention

We have now developed a fragrance delivery system which is capable of releasing fragrant alcohols upon exposure to light, and in particular daylight. One object of the present invention is a delivery system which comprises 2-benzoyl benzoates and 2-alkanoyl benzoates of formulae 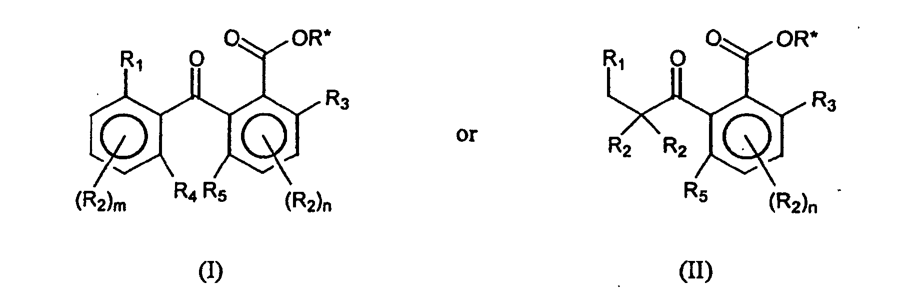 in which
R₁ represents hydrogen or a group of formula 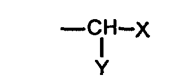 in which X and Y can be identical or different and represent, independently from each other, hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group or a group of formula 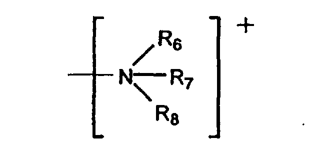 R₂ can be identical to R₁ or different from it and represents hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group, a group of formula 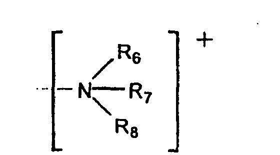 or a polyalcohol or polyether group ;
R₃ represents hydrogen, an alkyl or alkoxy group from C₁ to C₄, linear or branched, a OH group or a NH₂ group ;
R₄ and R₅, taken separately, have the meaning given above for R₁ and can be identical to or different from R₁ or from each other; or
R₄ and R₅, taken together, form a bridging group between the two aromatic rings, which bridging group can be a methylene or a keto group ;
m is an integer from 0 to 3 and n is an integer from 0 to 2 ; R₆ and R₇, taken separately, each represent hydrogen, an alkyl group from C₁ to C₄, an alcohol group having an alkyl chain from C₁ to C₁₂, or a phenyl group, or, R₆ and R₇, taken together with the nitrogen atom form a 5-membered or six-membered ring possibly containing another hetero atom ;
R₈ represents hydrogen, an alkyl group from C₁ to C₄, an alcohol group having an alkyl chain from C₁ to C₁₂ or a phenyl group ;
M represents hydrogen or an alkali metal ; and
R* is the organic part derived from a primary or secondary fragrant alcohol R*OH.

In the above definition, when reference is made to a fragrant alcohol, there is always meant an alcohol which not only has an odor, but which is also known to a person skilled in the art as being useful as perfuming ingredient for the formulation of perfumes or perfumed articles. The criteria a useful perfuming ingredient has to fulfil are known to a person skilled in the art and include, amongst others, a certain originality of the odoriferous note, stability and a certain price/performance ratio. Non-limiting examples for fragrant alcohols which can be used with the benzoates of the invention will be mentioned below.

From the above, it is clear that when reference is made to the organic part R* of a fragrant alcohol R*OH, R* is the hydrocarbyl rest of said alcohol, e.g. a geranyl radical in case R*OH is geraniol.

The advantage of the fragrance delivery system of the present invention lies in its capacity to slowly release the fragrant alcohols R*OH from which the benzoyl benzoate esters of formula (I) or the alkanoyl benzoate esters of formula (II) are derived. The release occurs when said esters are exposed to daylight in particular. Upon absorption of energy from said light, the ester undergoes a photoreaction in the course of which the fragrant alcohol is released from the molecule into the surroundings. Said release occurs in a controlled manner, i.e. a more or less constant amount of alcohol. R*OH is formed over a period of time, without an initial burst of very intense odor which becomes rapidly imperceptible as is the case with volatile alcohols. Because the release of the alcohol R*OH can occur over several days or weeks, the use of the system of the present invention obviates the drawbacks of many fragrant alcohols R*OH which are of pleasant smell but also very volatile. Good examples are citronellol and geraniol which can only be perceived over a short period of, say, one or two hours, when applied to the surface of, for example, tiles and windows in the course of a cleaning procedure using liquid cleaners ; even in solution, the typical smell of said alcohols disappears within several hours. It goes without saying that the concentration of the alcohols in the application plays an important role in the time during which the fragrant molecules can be perceived.

With the system of the present invention, the typical odor of the alcohol R*OH is perceived over a considerably prolonged period of time, as the 2-benzoyl benzoate or the 2-alkanoyl benzoate of the fragrance delivery system, which are not or few volatile, remain as such on the surface to which they are applied or in the solution into which they are incorporated, and it is only upon exposure to light, that the fragrant alcohol R*OH is released. It is clear that this reaction can provide perceptible amounts of the alcohol over days or weeks, depending, amongst others, on the amount or the concentration of the fragrance delivery system, the time of exposure to light, its intensity and its wavelength.

As fragrant alcohol R*OH derived radical R* in the above formula (I), in principle a group derived from any fragrant alcohol which is known in the art can be used. Primary and secondary alcohols are shown to be useful in the present invention as they are liberated when exposed to daylight.

As non-limiting examples of alcohols which can be used in the present invention in the form of the 2-benzoyl benzoate esters, one can cite anisic alcohol, cinnamic alcohol, fenchylic alcohol, 9-decen-1-ol, phenethylol, citronellol, 3-methyl-5-phenyl-1-pentanol (origin : Firmenich SA, Geneva, Switzerland), Mayol ® (7-p-menthan-1-ol ; origin: Firmenich SA, Geneva, Switzerland), geraniol (3,7-dimethyl-2,6-octadien-1-ol), (Z)-3-hexen-1-ol, 1-hexanol, 2-hexanol, 5-ethyl-2-nonanol, 2,6-nonadien-1-ol, borneol, 1-octen-3-ol, cyclomethyl citronellol, decanol, dihydroeugenol, 8-p-menthanol, 3,7-dimethyl-1-octanol, dodecanol, eugenol, isoeugenol, Tarragol ® (2-methoxy-4-propyl-1-cylohexanol; origin: Firmenich SA, Geneva, Switzerland), Polysantol ® [(E)-3,3-dimethyl-5-(2',2',3'-trimethyl-3'-cyclopenten-1'-yl)-4-penten-2-ol; origin : Firmenich SA, Geneva, Switzerland] and Limbanol ® [1-(2',2',3',6'-tetramethyl-cyclohex-1-yl)-3-hexanol ; origin : Firmenich SA, Geneva, Switzerland].

It is quite obvious, however, that the process of the invention is perfectly general and can relate to many other alcohols which the skilled person is quite able to choose from the general knowledge in the art and as a function of the olfactive effect it is desired to achieve. The above list therefore is more illustrative for fragrant alcohols which are known to a person skilled in the art, and whose delivery can be improved, but it is clearly quite impossible to cite in an exhaustive manner all alcohols of formula R*OH which have a pleasant odor and the 2-benzoyl or 2-alkanoyl benzoate esters of which can be used in the fragrance delivery system of the present invention.

From the foregoing, it is evident that the fragrance delivery system is particularly appropriate for delivering fragrant alcohols R*OH which are very volatile, or which have a low perception threshold, like geraniol, citronellol or phenethylol. The benzoyl and alkanoyl benzoate esters (I) of the latter are thus preferred according to the present invention.

The chemical reaction which releases the fragrant alcohol can only occur when a source of a hydrogen radical H• is present in the fragrance delivery system. It is believed that in the first reaction step, said hydrogen radical is transferred to the oxygen of the keto-function. Such a source can be intramolecular, i.e. the hydrogen radical comes from the 2-benzoyl benzoates of formula (I) or the 2-alkanoyl benzoates of formula (II) themselves, or intermolecular, i.e. the hydrogen radical comes from another, different source which is present in the medium in which the ester is incorporated. The intramolecular pathway or mechanism is a universal mechanism which can occur in every possible application medium, thus in the liquid or solid state. The intermolecular mechanism, however, is only possible in solution, but not in the solid state. Non-limiting examples of liquid state application media are liquid air-fresheners which release the fragrant alcohol upon exposure to light. Examples of release of the fragrant alcohol in the solid state are surfaces, like those of tiles or windows, which are cleaned with a cleaner containing the fragrance delivery system of the invention, the system being thus deposited on the surface after cleaning and remaining on it as a solid film after evaporation of the liquids present in the cleaner. However, it has to be understood that the term "solid" as used beforehand is used to designate the benzoates in the neat state in which they may be a real solid, crystalline or non-crystalline, or be in the form of a more or less viscous oil.

For the 2-benzoyl benzoates of the above formula (I) or the 2-alkanoyl benzoates of the above formula (II) in which R₁, R₄ and R₅ are hydrogen, an external hydrogen radical source is necessary. In general, the hydrogen radical will be abstracted from the solvent in which the 2-benzoyl or the 2-alkanoyl benzoate is dissolved or provided by a solvent which is added to the solution containing the said compound. Suitable sources are known to a person skilled in the art. The most important criterion a suitable hydrogen radical source has to fulfil is that a stable radical is formed after abstraction of the hydrogen. For a given compound, and independently from other functional groups or structural elements present in the same, the presence of hydrocarbon groups which are not methyl or tert-butyl is very favorable towards the formation of a stable radical after hydrogen abstraction. Suitable groups include ethyl or n-propyl. Even better are branched secondary alkyl groups, like isopropyl or sec-butyl. It is preferred when the solvent contains an isopropyl group or is a primary or secondary alcohol. Non-limiting examples for classes of solvents are the following: aliphatic and aromatic alcohols, like methanol, ethanol, propanol, decanol or benzyl alcohol, in particular isopropanol; diols and polyols, like ethyleneglycol, glycerol, polyethyleneglycol, propyleneglycol or polypropyleneglycol ; ketones, such as diisopropylketone ; esters, such as isopropylacetate ; aromatic solvents, such as ethylbenzene, cyclohexylbenzene or isopropylbenzene (cumene), di- or triisopropylbenzene; ethers, such as diisopropylether, tetrahydrofuran, mono-, di- or triethyleneglycoldimethylether, diethyleneglycolmonoether or polyethyleneglycol-dimethylether ; aminoalcohols, such as mono-, di- or triethanolamine ; hydrocarbons, in particular branched hydrocarbons, including limonene.

Preferred solvents include primary and secondary alcohols, in particular isopropanol, 1-dodecanol, 2-tridecanol, butanol or amyl alcohol.

All the above-mentioned solvents can, of course, also be used for benzoyl and alkanoyl benzoate esters which react in an intramolecular pathway to release the fragrant alcohol. In such case, R₁, R₄ or R₅ are the intramolecular hydrogen radical source, as will be described below.

The mentioned solvents will be chosen according to their ability to release hydrogen radicals.

We have found that the intramolecular pathway for the release of the fragrant alcohol only occurs when at least one of the groups R₁, R₄ or R₅ of formula (I) or (II), which is in 2-position relative to the keto function, is a group of formula 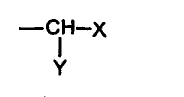 from which the hydrogen radical is easily transferred to the keto function, due to the vicinity of the group R₁ to the keto function by which an energetically favorable transition state is possible. X and Y are chosen to stabilize the resulting radical 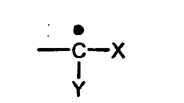 which remains after abstraction of the hydrogen radical and its transfer to the keto function. Suitable groups X and Y which can stabilize radicals are known to a person skilled in the art, and X and Y, which can be the same or different, will be chosen according to the respective benzoyl benzoate and the fragrant alcohol R*OH used in a given fragrance delivery system in order to give the best results, i.e. the desired release rate for the fragrant alcohol. Preferably X and Y are, independently from each other, a group as defined above with respect to formulae (I) and (II).

The compounds of formula (I) can contain, in addition to the substituent R₁ in 2-position of the cycle relative to the keto function, a further substituent R₄ in 6-position. It is evident that this substituent R₄ can also function as a hydrogen radical source, after a rotation around the single bond between the keto function and the phenyl ring. Moreover, the same applies to the group R₅ of the above formula (I) or (II), which is optionally present in the phenyl ring which carries the ester function. R₅, after a rotation of the phenyl group, can also serve as a hydrogen radical source. R₄ and R₅ thus have the same meaning as R₁, which has been defined above, and R₄ and R₅ can be identical to R₁, or they can be different from R₁ and, respectively, from each other.

The two phenyl groups of the 2-benzoyl benzoates or formula (I) can furthermore be bridged by a methylene or keto group.

We have furthermore found that it can be advantageous with respect to the release of the fragrant alcohol when the respective benzoyl benzoate of formula (I) or the respective alkanoyl benzoate of formula (II) carries a substituent R₃ other than hydrogen in the ortho-position to the -COOR*- function. The purpose of this substituent is to establish a favorable conformation of the -COOR*- function relative to the keto group, or respectively to the reduced keto-group, in order to facilitate the cyclization to the lactone which occurs after release of the alcohol. This reaction leads to the release of the fragrant alcohol R*OH. Practically, any group which is inert towards the -COOR*-function can be used, and they are known to a person skilled in the art. The groups defined in the above formula (I) and (II), namely linear or branched alkyl or alkoxy from C₁ to C₄, OH or NH₂ have revealed themselves as being appropriate from the point of view of effectiveness, and, of course, synthetic access.

The benzoyl benzoates of formula (I) and the alkanoyl benzoate of formula (II) can furthermore carry one or more substituents R₂ in the positions indicated and defined above. Substituents R₂, however, seem to be of less importance to the reactivity and the performance of the fragrance delivery system of the present invention, although it is often preferred, for reasons of easy accessibility of the corresponding 2-benzoyl and 2 -alkanoyl benzoates of the invention, to use an ester wherein R₂ is a group other than hydrogen. It is however possible to adapt e.g. the stability of the 2-benzoyl and 2 -alkanoyl benzoates of the present invention to the respective application desired. The 2-benzoyl benzoates can e.g. be rendered more hydrophilic by one or more groups R₂ which are a quaternary amine group, a polyalcohol group or a polyether group. Specific examples for said functional groups are known to a person skilled in the art, and the groups will be chosen according to the effect desired.

Preferred 2-benzoyl benzoate esters of the present invention are those of formula 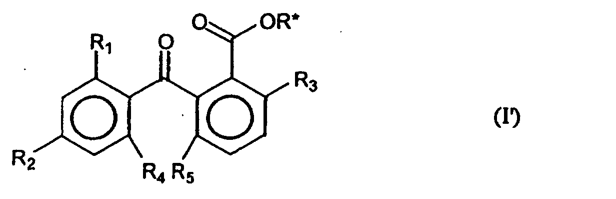 in which
R₁ is a branched alkyl group from C₃ to C₄ containing a secondary hydrocarbon group ;
R₂ is a branched alkyl group from C₃ to C₄ and is identical to R₁ ;
R₃ is hydrogen or a linear or branched alkyl group from C₁ to C₄;
R₄ is hydrogen or a linear or branched alkyl group from C₁ to C₄;
R₅ is hydrogen or a linear or branched alkyl group from C₁ to C₄ ;
R* is the organic part derived from a primary or secondary fragrant alcohol R*OH.

Generally, with respect to the above formulae (I) and (I'), it can be said that it is preferred when R₁, R₄ or R₅ which are responsible for the transfer of the hydrogen radical towards the keto function, is an isopropyl group, irrespective of the other substituents which may be present in the molecule. The isopropyl group was found to be the substituent which is most easily available, from a synthetic point of view, and which readily transfers hydrogen to the keto function, which we attribute to its ability to form a stable radical after abstraction of hydrogen.

The most preferred compounds according to the above formula (I') are geranyl 2-(2'-isopropylbenzoyl)benzoate, geranyl 2-(2',4'-diisopropyl-benzoyl)benzoate and 3,3-dimethyl-5-(2',2',3'-trimethyl-3'-cyclopenten-1'-yl)-4-penten-2-yl 2-(2',4'-diisopropylbenzoyl)benzoate [(E)-3,3-dimethyl-S-(2',2',3'-trimethyl-3'-cyclopenten-1'-yl)-4-penten-2-ol is a secondary alcohol sold under the name Polysantol ® by Firmenich SA, Geneva, Switzerland].

The 2-benzoyl and 2-alkanoyl benzoates of the present invention are synthesized by esterification of the respective 2-benzoyl and 2-alkanoyl benzoic acids with the desired alcohol, in a way known to a person skilled in the art, preferably using 4-dimethylaminopyridine in pyridine and 1,3-dicyclohexylcarbodiimide. The above-mentioned benzoic acids are obtained from the respective phthalic anhydride. This latter is brought to reaction, for example, with the desired substituted or unsubstituted benzene in a Friedel-Crafts reaction. If necessary, the respective phthalic anhydride can also be reacted with the Grignard reagent, the organolithium compound or another appropriate organometallic compound of the desired substituted or unsubstituted benzene or alkane, respectively.

A further object of the present invention is a fragrance delivery system comprising α-keto esters of formula 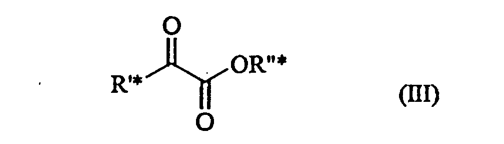 in which
R'* is hydrogen or a linear or branched, unsubstituted or substituted alkyl group or alkylene group from C₁ to C₃₅, an unsubstituted or substituted cycloalkyl group from C₃ to C₈, an unsubstituted or substituted phenyl group, wherein said alkyl, alkylene, cycloalkyl and phenyl groups may comprise one or several hetero atoms not directly linked to the α-keto group and selected from the group consisting of oxygen, nitrogen, phosphorous and sulfur, or
R'* is a substituted or unsubstituted, linear or branched alkyl group carrying an abstractable hydrogen in γ-position relative to the α-keto function and comprising a moiety from which is derived a fragrant compound containing an olefin function, such that said fragrant compound containing an olefin function is eliminated after abstraction of said γ-hydrogen atom ;
R"* is hydrogen or a methyl, ethyl or tert-butyl group or is the organic part of a primary or secondary alcohol from which is derived a fragrant aldehyde or ketone, and
at least one of the groups R'* and R"* being a group which is derived from a fragrant compound.

In the above definition, when reference is made to a fragrant compound, aldehyde or ketone, it is always meant a compound which not only has an odor, but which is also known to a person skilled in the art as being useful as a perfuming ingredient for the formulation of perfumes or perfumed articles. The criteria a useful perfuming ingredient has to fulfil are known to a person skilled in the art and include, amongst others, a certain originality of the odoriferous note, stability and a certain price/performance ratio. Non-limiting examples for fragrant compounds which can be used with the α-keto esters of the invention will be mentioned below.

Like the above-described 2-benzoyl benzoates and 2-alkanoyl benzoates, the α-keto esters of the above formula (III) release fragrant compounds upon exposure to light, in particular daylight. The α-keto esters of formula (III), however, are capable of releasing a fragrant compound containing an olefin function from the group R'* in 1-position relative to the keto function, or a fragrant aldehyde or ketone which is derived from the alcohol R"*OH from which the organic part R"* is present in the ester function of the keto esters of the present invention, or even both.

From the above, it is clear that when reference is made to the organic part R"* of a fragrant alcohol R"*OH, R"* is the hydrocarbyl rest of said alcohol, e.g. a menthyl radical in case R"*OH is menthol.

The release of the fragrant compound from the keto esters occurs in an elimination reaction after an intramolecular transfer of an abstractable hydrogen radical, in γ-position to the α-keto function, to said keto function. The respective part of the molecule from which the hydrogen radical has been abstracted is subsequently released from the reduced keto ester, with concomitant formation of a double bond. The above is illustrated in the scheme below in which possible substituents in the respective parts of the molecules have been omitted for reasons of clarity. The double bonds which will be formed after elimination are indicated by dotted lines.

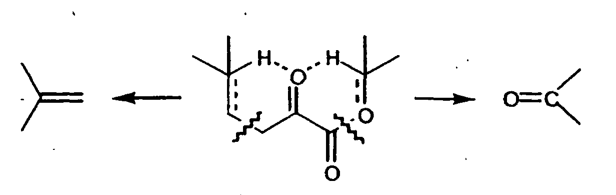

It is to be understood that the α-keto esters of the present invention can release only one or both molecules of fragrant compound per molecule of α-keto ester. When the hydrogen transfer to the α-keto function is able to occur from the one or the other side of said function, as illustrated above, a certain part of the molecules will release a ketone or aldehyde and a certain part will release the olefin compound. The proportions of the two products released depend on the relative rate of each hydrogen transfer reaction. According to the effect desired, the α-keto esters of the invention can be tailored to release exclusively a fragrant ketone or aldehyde, or exclusively a fragrant compound containing an olefin group, or both. When only one of the two classes of fragrant compounds is to be released from the α-keto esters of the invention, the part of the molecule from which no release shall occur does not contain an abstractable hydrogen atom in γ-position to the keto function, i.e. either no hydrogen atom at all is present in the said position, or it is one which is not abstracted.

It is also clear that the α-keto esters according to the invention can, in a first step, release the olefin compound under formation of a molecule which does not any longer contain an abstractable hydrogen atom in γ-position to the keto function (left side of the molecule as designed above) ; in a second step, this molecule can then release the ketone or aldehyde from the ester function.

It is evident that a fragrance delivery system which contains the α-keto esters of the above formula (III) has all the advantages described above for the 2-benzoyl and 2-alkanoyl benzoates of formula (I) and (II), i.e. the release of the fragrant compound occurs in a more or less constant amount. No initial burst of very intensive odor which becomes imperceptible after a relatively short period of time occurs, as is often observed with volatile aldehydes or ketones or fragrant compounds containing an olefin group. With the α-keto esters of the present invention, such disadvantages are obviated because the esters will remain on a surface to which they have been applied or in the solution into which they have been incorporated. Upon exposure to light, the fragrant compound or compounds are released, and this reaction can provide perceptible amounts of the compound over days or weeks, depending, amongst others, on the amount or the concentration of the α-keto esters, the time of exposure to light and its intensity.

A further advantage of the α-keto esters according to formula (III) is the protection of the reactive, unstable aldehyde or keto function in the molecules to be released against degradation which may occur during storage.

Additionally, the α-keto esters of the present invention allow for the generation of mixtures of two different fragrant compounds, and in different proportions, if desired.

In principle, any fragrant aldehyde or ketone which is known in the art can be released from the α-keto esters of the invention in which they are chemically bound in the form of the ester of their corresponding secondary or primary alcohol.

Non-limiting examples for fragrant aldehydes which can be released from the α-keto esters include saturated and unsaturated linear and branched aldehydes from C₆ to C₁₃, citral, citronellal, campholenic aldehyde, cinnamic aldehyde, hexylcinnamic aldehyde, formyl pinane, hydroxycitronellal, cuminic aldehyde, vanillin, ethylvanillin, Lilial® [3-(4-tert-butylphenyl)-2-methylpropanal ; origin : Givaudan-Roure SA, Vernier, Switzerland], Lyral ® [4- and 3-(4-hydroxy-4-methylpentyl)-3-cyclohexene-1-carbaldehyde ; origin : International Flavors and Fragrances, USA], Bourgeonal ® [3-(4-tert-butylphenyl)propanal ; origin : Quest International, Naarden, Netherlands], heliopropanal [3-(1,3-benzodioxol-5-yl)-2-methylpropanal ; origin : Firmenich SA, Geneva, Switzerland], Zestover (2,4-dimethyl-3-cyclohexene-1-carbaldehyde ; origin : Firmenich SA, Geneva, Switzerland), Trifernal ® (3-phenylbutanal ; origin : Firmenich SA, Geneva, Switzerland), α-sinensal, (4-methylphenoxy)acetaldehyde, 1,3-benzodioxol-5-carboxaldehyde (heliotropine), Scentenal ® [8(9)-methoxy-tricyclo[5.2.1.0.(2,6)]decane-3(4)-carbaldehyde ; origin: Firmenich SA, Geneva, Switzerland], Liminal ® [(4R)-1-p-menthene-9-carbaldehyde ; origin : Firmenich SA, Geneva, Switzerland], Cyclosal [3-(4-isopropylphenyl)-2-methylpropanal ; origin : Firmenich SA, Geneva, Switzerland], ortho- and para-anisaldehyde, 3-methyl-5-phenylpentanal, Acropal ® [4-(4-methyl-3-pentenyl)-3-cyclohexene-1-carbaldehyde ; origin : Givaudan-Roure SA., Vernier, Switzerland], Intreleven ® aldehyde (mixture of 10-undecenal and 9-undecenal ; origin : International Flavors & Fragrances, USA), muguet aldehyde [(3,7-dimethyl-6-octenyl)acetaldehyde ; origin : International Flavors & Fragrances, USA], 2,6-dimethyl-5-heptanal, Precyclemone ® B [1-methyl-4-(4-methyl-3-pentenyl)-3-cyclohexen-1-carbaldehyde ; origin: International Flavors & Fragrances, USA] and Isocyclocitral ® (2,4,6-trimethyl-3-cyclohexene-1-carbaldehyde ; origin : International Flavors & Fragrances, USA).

Non-limiting examples for ketones which can be released from the α-keto esters include camphor, carvone, menthone, ionones, irones, damascenones and damacones, benzyl acetone (4-phenyl-2-butanone), 1-carvone, 4-(4-hydroxy-1-phenyl)-2-butanone (raspberry ketone), Hedione ® (methyl dihydrojasmonate ; origin : Firmenich SA, Geneva, Switzerland), Neobutenone [1-(5,5-dimethyl-1-cyclohexen-1-yl)-4-penten-1-one ; origin : Firmenich SA, Geneva, Switzerland], Calone ® (7-methyl-2H,4H-1,5-benzodioxepin-3-one ; origin : C.A.L. SA, Grasse, France), Sulfox [(1R,4R)-8-mercapto-3-p-menthanone ; origin: Firmenich SA, Geneva, Switzerland], Orivone ® [4-(1,1-dimethylpropyl)-1-cyclohexanone ; origin : International Flavors & Fragrances, USA], Delphone (2-pentyl-1-cyclopentanone ; origin: Firmenich SA, Geneva, Switzerland), 2-naphthalenyl-1-ethanone, Veloutone (2,2,5-trimethyl-5-pentyl-1-cyclopentanone ; origin: Firmenich SA, Geneva, Switzerland), 4-isopropyl-2-cyclohexen-1-one, Iso E Super ® [isomer mixture of 1-(octahydro-2,3,8,8-tetrame-2-naphthalenyl)-1-ethanone ; origin : International Flavors & Fragrances, USA], Plicatone [5-methyl-exo-tricyclo[6.2.1.0(2,7)]undecan-4-one ; origin : Firmenich SA, Geneva, Switzerland] ; and macrocyclic ketones such as, for example Exaltone ® (cyclopentadecanone), Delta Muscenone (mixture of 3-methyl-4-cyclopentadecen-1-one and 3-methyl-5-cyclopentadecen-1-one) and Muscone (3-methyl-1-cyclopentadecanone), all from Firmenich SA, Geneva, Switzerland.

With respect to the fragrant compounds carrying an olefin group, in principle any compound containing such olefin group and, in addition, any osmophoric group known in perfumery can be used. As non-limiting examples for osmophoric groups, one can cite alcohol, ether, ester, aldehyde and keto groups, the thio analogues of the said groups, nitrile, nitro and olefin groups.

As non-limiting examples for fragrant compounds which carry an olefin group, there can be cited linalool, 1,3,5-undecatrienes, myrcene, myrcenol, dihydromyrcenol, nerolidol, sinensals, limonene, carvone, farnesenes, isopentyrate (1,3-dimethyl-3-butenyl isobutyrate ; origin : Firmenich SA, Geneva, Switzerland), allyl 3-methylbutoxyacetate, eugenol, Rosalva ® (9-decen-1-ol ; origin : International Flavors & Fragrances, USA), and allyl heptanoate.

It is quite obvious, however, that the invention is perfectly general and can relate to many other aldehydes, ketones and olefins which are useful as fragrant compounds. The person skilled in the art is quite able to choose these compounds from the general knowledge in the art and from the olfactive effect it is desired to achieve. The above list is therefore more illustrative for the compounds which are known to a person skilled in the art, and whose delivery can be improved. It is clearly quite impossible to cite in an exhaustive manner all aldehydes, ketones and olefins which have a pleasant odor and which can be used in the form of derivatives in the α-keto esters of formula (III) from which they are released upon exposure to light.

The α-keto esters of the present invention are in particular appropriate for delivering fragrant aldehydes, ketones and fragrant compounds containing an olefin group which are very volatile or which have a low perception threshold. Preferred aldehydes and ketones include citronellal, citral, hydroxycitronellal, Hedione ®, Lilial ®, raspberry ketone, anisaldehyde, menthone, Delphone, Orivone ®, 2-naphthalenyl-1-ethanone, and aldehydes from C₆ to C₁₃, saturated or unsaturated linear or branched. Preferred fragrant compounds containing an olefin group include linalool, myrcene, myrcenol and Rosalva ®.

In case the α-keto esters of the present invention are used to release exclusively aldehydes or ketones, the group R'* is hydrogen, phenyl, cyclohexyl or cyclopentyl, methyl, ethyl, n-propyl, isopropyl, sec-butyl, isobutyl or tert-butyl, i.e. groups which do not provide an abstractable hydrogen atom in γ-position to the α-keto function or which do not form a stable radical when a hydrogen radical is abstracted from them. In the latter case, small amounts of olefin may be formed which however do not interfere with the aldehyde or ketone released.

Likewise, when the α-keto esters of the present invention are used to release a fragrant compound containing an olefin group only, then the group R"* will be hydrogen or a methyl, ethyl or tert-butyl group, thus a group which does not provide an abstractable proton in γ-position to the α-keto function or which do not form a stable radical when a hydrogen radical is abstracted from them.

It is preferred when the fragrance delivery system of the present invention contains α-keto esters of formula (III) in which R"* is the organic part of a primary or secondary alcohol from which is derived a fragrant aldehyde or ketone and in which R'* is a phenyl, cyclohexyl or cyclopentyl group or a linear or branched alkyl group from C₁ to C₄.

A fragrance delivery system containing the α-keto esters of formula (III) does not need an external hydrogen radical source. A fragrance delivery system containing α-keto esters of the present invention may thus comprise a solvent the choice of which is not supposed to be critical. Suitable classes of solvents include alcohols, ethers, esters, ketones, amines and aminoalcohols.

Depending on the general application conditions or on the product into which the α-keto esters according to the present invention are incorporated, one can sometimes also observe the release of alcohols R"*OH, due to saponification of the ester function, or due to reduction of the aldehyde or ketone formed by irradiation.

The α-keto esters of formula (III) can be prepared, on the one hand, by esterification of the respective α-ketoacids with the primary or secondary alcohols which are the precursors of the fragrant aldehydes and ketones to be releasead. Another way for the preparation of the α-keto esters of the present invention is the reaction of the bis(oxalyl) ester of the primary or secondary precursor alcohol R"*OH with the Grignard compound of the appropriate group R'* as defined in formula (III). The reaction is illustrated in the scheme I below.

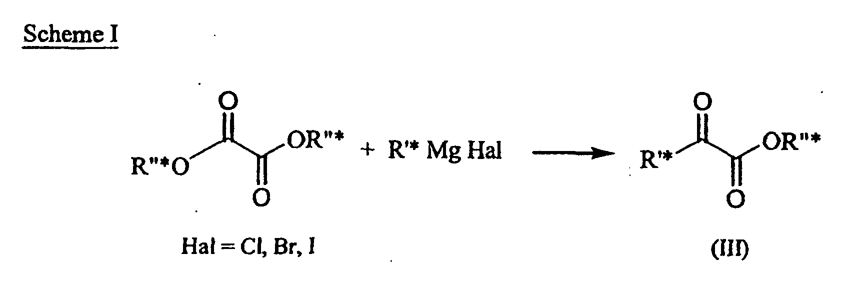

The bis(oxalyl) ester is prepared from oxalyl chloride and the desired alcohol, see Synth. Commun. **1981**, (11), 943-946 and Org. Synth. Coll. Vol. II **1943**, 425-427.

Another synthetic route leading to the desired α-keto esters of formula (III) is the Grignard reaction of the readily available bis(oxalyl)esters of lower aliphatic alcohols such as, for example, methanol, ethanol or propanol, with the Grignard compound of the respective group R'*, resulting in the intermediate ester (IV). This said ester (IV) is then submitted to a transesterification reaction with the respective precursor alcohol R"*OH, to give the desired α-keto ester. This reaction is outlined in the following scheme II in which R'* and R"* have the meaning defined in formula (III). Hal is Cl, Br or I and R is a lower alkyl group such as, for example, methyl, ethyl, propyl or butyl.

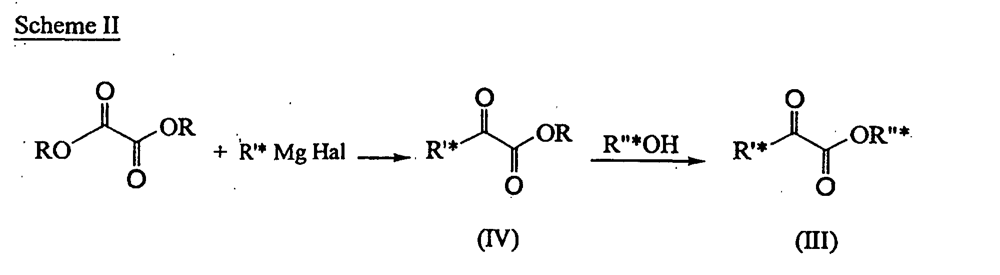

Various α-keto esters of formula (III) in which R'* is hydrogen or a phenyl or methyl group and R"* is derived from the alcohol precursor of a fragrant aldehyde are described in the literature.

Also known is hexyl (cyclohexyl)oxoacetate (see DE-OS 29 09 951 to Bayer AG, describing the use of the said compound as starting product for the synthesis of catalysts for the polymerisation of olefins), which would release n-hexanal upon irradiation.

In Biochem. Z. 1935, (277), p 426-436, there is described the synthesis of the (-)-bornyl ester of (4-methylphenyl)oxoacetic acid, i.e. (-) (1S,2R), 1,7,7-trimethylbicyclo[2.2.1]heptan-2-yl (4-methylphenyl)oxoacetate. The compound is characterized by its physical data.

There are furthermore known, from the chemical literature, various compounds according to the above formula (III) wherein OR"* is a menthyl or a benzyl group, with the groups R'* being various alkyl, alkenyl, cycloalkyl or phenyl groups as defined above.

There is nowhere found, however, any description or hint concerning the value of the compounds according to formula (III) in perfumery as a photosensitive molecule which will release a fragrant compound upon irradiation.

In the book of S. Arctander, Perfume and Flavors Chemicals, 1969, Montclair, New Jersey, USA, there are mentioned decyl 2-oxopropanoate, (Z)-3-hexenyl 2-oxopropanoate and 2-ethyl-3-methylbutyl 2-oxopropanoate, with a short description of their odor and their synthesis. It is not mentioned that the said molecules release fragrant compounds upon irradiation.

The release of the above-mentioned fragrant compounds from the delivery system occurs upon the exposure to light, e.g. the normal daylight which can penetrate through ordinary windows in houses and which is not particularly rich in UV-radiation. It goes without saying that upon exposure to bright sunlight, in particular outdoors, the release of the fragrant alcohol, aldehyde, ketone or alkene will occur faster and to a greater extent than upon exposure to the light in a room inside a building. Of course, the reaction which releases the fragrant compound from the delivery system can also be initiated by an appropriate artificial lamp.

The fragrance delivery systems of the present invention can be used in any application in which a prolonged, defined release of the above-mentioned fragrant compounds is desired. They therefore mostly find use in functional perfumery, in articles which are exposed to daylight when in use or which are applied to other articles which thereafter are exposed to daylight. Suitable examples include air-fresheners in liquid and solid form which, with the delivery system of the present invention, still can release a fragrance when conventional air-fresheners, i.e. those not containing the system of the present invention, are exhausted. Other examples, are various cleaners for the cleaning of surfaces of all kinds, e.g. window and household cleaners, all purpose-cleaners and furniture polish. The surfaces which have been cleaned with such cleaners will diffuse the smell of the perfume much longer than when cleaned with conventional cleaners. Other representative examples include detergents for fabric wash, fabric conditioners and fabric softeners which can also contain the delivery system of the present invention and which products can be in the form of powders, liquids or tablets. The fabrics and clothes washed or treated with such detergents or softeners will diffuse the fragrant compound even after having been stored for weeks, or even months, in a dark place, like a wardrobe.

The release of the fragrant compound occurs in all the above-mentioned application examples. All possible types of window, household, all-purpose cleaners, air-fresheners, detergent, fabric wash and fabric softeners can be used with the fragrance delivery system of the present invention, which has revealed itself to be useful in all types of these above-mentioned application examples.

In the field of body care, the delivery systems according to the present invention have shown themselves to be particularly appropriate for an application in the hair care area, and specific examples include shampoos, hair conditioners, in particular leave- in conditioners, hairspray and other hair care products.

It can be said that generally all products which can be applied to a surface which is exposable to light may contain the system of the present invention. Examples include surfaces which belong to the human body, like skin or hair, surfaces in buildings and apartments, like floors, windows, tiles or furniture, or surfaces of fabrics, e.g. clothes. It is clear that the system of the invention can also be used to release fragrances from liquids, like in liquid air-fresheners. The possible applications of this type, however, appear to be less general than the application on the various surfaces mentioned.

Of course, the above examples are only illustrative and non-limiting as referring to preferred embodiments. All other current articles in functional and fine perfumery may contain the system of the present invention, and these articles include soaps, bath or shower gels, cosmetic preparations, body deodorants, and even perfumes or colognes.

In the above-cited applications, the device of the present invention can be used alone or with other perfuming ingredients, solvents and adjuvants of current use in the art. The nature and variety of these co-ingredients does not require a detailed description which, moreover could not be exhaustive, and a person skilled in the art will be able to choose said coingredients by his general knowledge and in function of the nature of the product to be perfumed and the olfactive effect sought. These perfuming ingredients belong to such varied chemical classes as alcohols, aldehydes, ketones, esters, ethers, acetates, nitriles, terpene hydrocarbons, nitrogen- or sulfur- containing. heterocyclic compounds, as well as essential oils of natural or synthetic origin. By way of example, embodiments of compounds can be found in standard reference works, such as the book of S. Arctander, Perfume and Flavor Chemicals, 1969, Montclair, New Jersey, USA, or more recent versions thereof, or in other works of similar nature.

The proportions in which the system of the present invention can be incorporated in the various above-mentioned products vary within a wide range of values. These values depend on the nature of the fragrant compound to be released, the nature of the article or product which is to be perfumed and the desired olfactive effect, as well as on the nature of the co-ingredients in a given composition when the system of the present invention is used in admixture with perfuming co-ingredients, solvents or adjuvants of current use in the art.

By way of example, one can cite typical concentrations of the order of 0.01 to 5%, or even 10% by weight relative to the weight of the consumer products cited above into which it is incorporated. Higher concentrations than those mentioned above can be used when the system is applied in perfuming compositions, perfumes or colognes.

The invention will now be described in greater detail in the following examples in which the temperatures are indicated in degrees centigrade and the abbreviations have the usual meaning in the art.

### Embodiments of the invention

### General

The following chemicals were obtained from commercial sources : geraniol, Polysantol ®, 2-benzoylbenzoate, dicyclohexylcarbodiimide (DCC), diisopropylcarbodiimide (DIC), 4-dimethylamino-pyridine, magnesium turnings, 2-iodoisopropyl benzene, 1,3-diisopropylbenzene, AlCl₃, 1,2-dichloroethane, 1,2-dibromoethane, 2-norbornyl bromide, bromocyclopentane, citronellol, decanol, 4-methoxybenzyl alcohol, Lilial®, (-)-menthol, 2-pentylcyclopentanol, 4-(1,1-dimethylpropyl)-1-cyclohexanol, 1-(2-naphthalenyl)ethanol, oxalyl chloride, diethyl oxalate, 3-methyl-2-oxo-pentanoic acid, 2-oxopropionic acid, 2-oxobutanoic acid, bromocyclohexane, bromobenzene, 2-oxo-pentanoic acid, 4-bromo acetophenone, ethylene glycol, 2-bromo-tetradecane, 1-bromo-tetradecane.
Geranyl 2-benzoylbenzoate (**1**) was prepared as described by Porter et al., J. Org, Chem. **1996**, *61*, 9455-9461.

### A. Execution of photorelease assays and analysis for 2-benzoyl benzoates and 2-alkanoyl benzoates

### Photorelease Assays

Photorelease assays were conducted on solutions (typical concentrations = 0.005 to 0.01 M) or films of the respective esters in 10 ml borosilicate glass volumetric flasks (*Pyrex* ®) unless otherwise stated. The films were prepared by dissolving the ester in a small (<1 ml) volume of pentane or acetone, transferring to a 10 ml volumetric flask and drying under a stream of nitrogen or reduced pressure while rotating the flask to evenly disperse the ester on the surface of the glass. The samples were not degassed.
The Fadeometer assays were done using an Atlas Ci35 Fadeometer, equipped with a borosilicate glass inner filter and a soda lime outer filter, set at 0.35 W/m² at 340 nm. Natural light assays were done by putting the samples in a metal rack outdoors during daylight hours. Natural light conditions could also be mimicked by using a 8W 366 nm UV lamp with an intensity of 500 µW/cm² (VWR Scientific Products).

### Analysis

After photolysis, the quantity of alcohol released was measured by GC analysis of duplicate samples using the alcohol as the external standard. The presence of photoreleased alcohol was checked using GC retention times, GC-MS and also by smelling the samples. The ester solutions were injected neat while the solid films were dissolved and diluted volumetrically to 10 ml in acetone. Samples (1 µl split 54:1, injector at 250°C) were injected as acetone solutions. Gas chromatography-flame ionization detection (GC-FID) was carried out using an SPB-1 capillary column (30 m, 250 µm id, 0,25 µm film, He carrier gas, 1.0 ml/min). Gas chromatography-mass spectrometry (GC-MS) was performed using an HP-5890 GC coupled to an HP 5989A mass spectrometer. The GC separation utilized an SPB-1 capillary column (30 m, 0,25 µm id, 0.25 µm film, He carrier gas, 1 ml/min). An SPB-1 column (30 m, 0,32 µm id, 0.25 µm film, He carrier gas, 1.3 ml/min) was used for the GC separation with the same temperature program used for the GC-MS. The samples (1 µl, split 16:1, injector at 250°C) were injected as acetone solutions.

### B. Execution of photorelease assays and analysis for α-keto esters

### Photorelease Assays

Photorelease assays were conducted on solutions or on films of the respective ester and will be described below in each of the examples referring to the respective mode of irradiation.
All samples were irradiated using a xenon lamp (Heraeus Suntest CPS at 460W/m2), a UV lamp (UVP Model UVL-28, 8W at 360 nm) or exposed to outdoor sunlight, as will be indicated for each sample in the respective examples.

### Analysis

The mode of analysis for each sample which had been irradiated will be indicated in each respective example.
*Analytical HPLC* was carried out on a *Spectra Physics* instrument composed from a *SP 8800* ternary pump, *a SP 5750* injection valve, *a SP 8780* autosampler, a *Waters 490E* UV detector and *a Spectra Physics ChromJet* integrator*Macherey-Nagel Nucleosil 5 C*_{*18*} reversed phase column (125 x 4 mm i.d.) eluted with a gradient from acetonitrile/water 1:1 to pure acetonitrile during 20 min. The injection volume was 50 µl and the UV detector wavelength fixed at 220 nm.
*Analytical GC* for analysis of all-purpose/window cleaner applications : the on-column injections were carried out on a *Carlo Erba MFC 500* using a precolumn (30 cm) and a *Suppelco SPB-1* capillary column (30 m) at 115°C for 8 min, then to 28.0°C, helium pressure 75 kPa, injection volume 2 µl. All other GC analyses were carried out on the same instrument equiped with a *Fisons AS 800* autosampler using a *J& W Scientific DBI* capillary column (15 m) at 70 or 80°C for 10 min, then to 260°C, helium pressure 50 kPa, injection volume 0.5 µl.
*Analytical GC* for dynamic headspace analysis : *Tenax* cartridges were thermally desorbed in *a PE ATD400* or a *TDAS 5000* desorber. The volatiles were then analysed either with a *Carlo Erba HRGC 5300* gas chromatograph coupled to *Finnigan ITD-800* mass spectrometers using a *Supelco SPB-1* capillary column (60 m, 0.75 mm i.d., film 1 micron) at 60°C for 5 min then to 120°C (3°C/min) and 280°C (5°C/min) for the citronellal analysis, and at 100°C then to 250°C (5°C/min) for the menthone quantification or, alternatively, with a *Carlo Erba Vega 6000* gas chromatograph using a *Supelco SPB-1* capillary column (30 m, 0.53 mm i.d., film 1.5 micron) from 110°C to 200°C (6°C/min) using He as carrier gas in both cases.

### Example 1

### Preparation of substituted 2-benzoyl benzoates

### a) Geranyl 2-(2'-isopropylbenzoyl)benzoate (2)

Magnesium (0.46 g, 19 mmol) and a crystal of iodine were placed in a dry round bottom flask which was heated to activate the magnesium. Diethyl ether was added to cover the magnesium (50 ml) and several drops of 2-iodoisopropyl benzene in diethyl ether were added to start the preparation of the Grignard reagent. When the latter was underway, a solution of 2-iodoisopropyl benzene (4.18 g, 17 mmol) in diethyl ether (20 ml) was added over 20 minutes. The reaction mixture was stirred for another 15 minutes and then refluxed for 20 minutes. Phthalic anhydride (3.11 g, 21 mmol) in toluene (50 ml) was added dropwise to the Grignard reagent at room temperature. The reaction temperature was raised to 60°C and the diethyl ether removed by evaporation. The reaction was allowed to stir at 60°C for 6 hours. The reaction mixture was poured on ice and 10% HCl (100 ml) and extracted twice with diethyl ether. The organic phase was washed twice with a 10% Na₂CO₃ solution (200 ml). The aqueous phase was acidified with acetic acid (120 ml) and extracted twice with diethyl ether (200 ml). The organic phase was washed three times with NaHCO₃ (100 ml) and then twice with water. The ether phase was dried over Na₂SO₄, filtered and concentrated. The yield was 1.43 g (purity : 94.6%, isolated yield : 31%) of 2-(2-isopropylbenzoyl)benzoic acid.
For esterification, a solution of the thus obtained acid (3.77 g, 10 mmol), geraniol (1.4 g, 9 mmol) and 4-dimethylaminopyridine (DMAP, 0.244 g, 2 mmol) in pyridine (15 ml) was prepared under anhydrous conditions. 1,3-Dicyclohexylcarbodiimide (DCC, 2.06 g, 10 mmol) was added and the reaction was stirred under a stream of nitrogen gas for 52 hours. The reaction mixture was partitioned between 1M HCl and ethyl acetate. The organic extract was dried over Na₂SO₄, filtered and concentrated under vacuum. The ester product was purified by flash column chromatography (SiO₂, 7:1 cyclohexane:ethyl acetate ; isolated yield : 0.7 g, 48%) to give the following analytical data:
UV (cyclohexane) 240 (ε 13 000), 280 (ε 5 000) ;
¹H-NMR (360MHz, CDCl₃) δ (ppm) : 7.85 (m, 1H) ; 7.49 (m, 4H) ; 7.38 (m, 1H) ; 7.23 (dd, 1H, J=1, 8Hz) ; 7.12 (m, 1H) ; 5.21 (1H, m) ; 5.05 (1H, m) ; 4.65 (1H, d, J=7Hz) ; 3.70 (1H, m) ; 2.00 (4H, m) ; 1.66 (3H, br s) ; 1.63 (3H, br s); 1.58 (3H, br s) ; 1.28 (6H, d, J=7Hz)
¹³C NMR (90MHz, CDCl₃) δ (ppm) : 198.7(s), 167.2(s), 150.1(s), 142.5(s), 142.1 (s), 136.7(s), 131.6(d), 131.1(d), 130.6(d), 130.3(d), 129.5(d), 129.0(d), 126.4(d), 124.9(d), 123.8(d), 117.8(d), 62.4(t), 39.5(t), 29.3(d), 26.3(t), 24.1(q), 17.7(q), 16.5(q).

### b) Geranyl 2-(2',4'-diisopropylbenzoyl)benzoate (3)

Phthalic anhydride (19.3 g, 0.13 mol) was placed in a flame-dried three-neck round-bottom flask under nitrogen. 1,2-Dibromoethane (100 ml) and aluminum chloride (36.0 g, 0.27 mol) were added. The reaction solution was stirred at room temperature while 1,3-diisopropylbenzene (20.4 g, 0.126 mol) was added dropwise over the space of an hour. The reaction mixture was stirred at 100°C for two hours. Upon completion, the reaction mixture was cooled to room temperature and poured over ice/hydrochloric acid (1:1). The solution was extracted twice with dichloromethane. The organic extract was washed with saturated aqueous sodium chloride solution to neutrality, dried over anhydrous sodium sulfate, filtered and concentrated under vacuum to yield a heavy brown oil of 80% purity (isolated yield = 36 g, % yield = 74%). The thus obtained 2-(2',4'-diisopropylbenzoyl) benzoic acid showed the following analytical characteristics:
IR: (neat), 2965, 1695, 1670, 1605 cm⁻¹,
¹H NMR (360 MHz, CDCl₃) δ ppm : 7.98 (1H, dd, *J* = 1, 8 Hz), 7.59 (1H, m), 7.52 (1H, m), 7.37 (1H, dd, *J* = 1, 8 Hz), 7.31 (1H, d, *J* = 1.2 Hz), 7.09 (1H, d, *J* = 8 Hz), 6.94 (1H, dd, *J* = 2, 8 Hz), 3.82 (1H, m), 2.91 (1H, m), 1.25 (12H, m);
13C NMR (90 MHz, CDCl₃) δ ppm : 198.6 (s), 170.9 (s), 153.2 (s), 151.0 (s), 143.8 (s), 133.9 (s), 132.3 (d), 131.7 (d), 130.6 (d), 129.8 (d), 128.9 (s), 128.7 (d), 125.0 (d), 122.7 (d), 34.3 (d), 29.0 (d), 24.1 (q), 24.1 (q), 23.7 (q), 23.7 (q);
LREIMS: *m*/*z* (relative abundance) 310 (5, M⁺), 265 (43), 249 (45), 221 (100), 149 (32), 84 (41), 49 (35).

The thus obtained product (1.15 g, 3.7 mmol) was dissolved in dry pyridine (10 ml) in a flame-dried three-neck round-bottom flask. To the solution were added geraniol (freshly dialled, 0.55 g, 3.6 mmol), 4-dimethylaminopyridine (DMAP, 0.10 g, 0.8 mmol) and 1,3-dicyclohexylcarbodiimide (DCC, 0.76 g, 3.7 mmol). The reaction mixture was stirred at room temperature overnight. When complete, the reaction mixture was poured onto shaved ice (20 g), 32% hydrochloric acid (24 g) and ethyl acetate (30 ml) and stirred vigorously for 10 minutes. The solution was extracted twice with diethyl ether and the organic phase was washed twice with saturated aqueous sodium bicarbonate solution and twice with water. The organic phase was dried over anhydrous sodium sulfate and concentrated under vacuum. The product was purified by redissolving in pentane, crystallizing at 4°C, and filtering though Celite. The filtered solution was concentrated under vacuum and purified further by normal phase silica gel chromatography (20% diethyl ether/heptane). Geranyl 2-(2'-4'-diisopropylbenzoyl)benzoate was isolated as a pale yellow oil (isolated yield = 1.08 g, % yield = 74.5%), having the following analytical data :
¹H NMR (360 MHz, CDCl₃) δ ppm : 7.76 (1H, dd, *J* = 3, 6 Hz), 7.51 (2H, m), 7.37 (1H, dd, *J =* 3, 6 Hz), 7.31 (1H, d, *J =* 2 Hz), 7.18 (1H, d, *J* = 8 Hz), 6.97 (1H, dd, *J =* 2, 8 Hz), 5.22 (1H, m), 5.04 (1H, m), 4.64 (2H, d, *J =* 7 Hz), 3.79 (1H, m), 2.92 (1H, m), 2.1-1.9 (4H, m), 1.74 (3H, br s), 1.62 (3H, br s), 1.58 (3H, br s), 1.28 (6H, d, *J* = 7 Hz), 1.25 (6H, d, *J* = 7 Hz);
¹³C NMR (90 MHz, CDCl₃) δ ppm : 198.5 (s), 167.2 (s), 152.9 (s), 150.6 (s), 142.7 (s), 142.4 (s), 134.1 (s), 131.7 (s), 131.2 (s), 131.6 (d), 131.1 (d), 129.9 (d), 129.6 (d), 128.7 (d), 124.7 (d), 123.8 (d), 122.8 (d), 117.9 (d), 62.3 (t), 39.5 (t), 34.3 (d), 29.2 (d), 26.3 (t), 25.7 (q), 24.1 (q), 24.1 (q), 23.7 (q), 23.7 (q), 17.7 (q), 16.4 (q);
LREIMS: *m*/*z* (relative abundance) 446 (M⁺, <0.5), 309 (100), 265 (29), 249 (52), 231 (28), 221 (49), 149 (52), 93 (34), 69 (55), 41 (53).

### c) (E)-3,3-dimethyl-5-(2',2',3'-trimethyl-3'-cyclopenten-1'-yl)-4-penten-2-yl 2-(2',4'-diisopropylbenzoyl)benzoate (4)

2-(2',4'-Diisopropylbenzoyl)benzoic acid (0.3114 g, 1.0 mmol) was dissolved in dry pyridine (2 ml) in a flame-dried round bottom flask. To the solution were added Polysantol ® (0.2113 g, 0.95 mmol), 4-dimethylaminopyridine (DMAP) on polystyrene resin (0,168 g, 0.34 mmol) and 1,3-diisopropylcarbodiimide (DIC, 120 µl, 1.4 mmol). The reaction mixture was stirred at room temperature, under an atmosphere of dry nitrogen for 68 hours. The reaction mixture was filtered, and partitioned between 0.5M aqueous hydrochloric acid and ethyl acetate. The organic phase was washed a second time with 0.5M hydrochloric acid, then once with 10% aqueous sodium carbonate solution. The ethyl acetate solution was washed with saturated, aqueous sodium bicarbonate solution and finally with water. The organic phase was dried over anhydrous sodium sulfate, filtered and concentrated under vacuum. The resulting ester was purified by normal phase silica gel chromatography (2% ethyl acetate/cyclohexane) to yield a 1:1 mixture of two stereoisomers in the form of an oil (isolated yield = 0.14 g, % yield = 27%) which showed the following analytical data :
IR: (neat) 2960, 1720, 1675 cm⁻¹,
¹H NMR (360 MHz, CDCl₃) δ ppm : 7.83 (m, 1H), 7.52 (m, 2H), 7.39 (m, 1H), 7.30 (d, 1H, *J* = 1 Hz), 7.12 (dd, 1H, *J* = 2, 8 Hz), 6.94 (dd, 1H, *J* = 2, 8 Hz), 5.39 (2H, m), 5.21 (1H, m), 4.82 (1H, m), 3.83 (1H, m), 2.90 (1H, m), 2.26 (1H, m), 2.17 (1H, m), 2.03 (1H, m), 1.59 (3H, br d, *J* = 1 Hz), 1.30 (6H, d, *J* = 7 Hz), 1.24 (6H, d, *J* = 7 Hz), 0.99, 0.99 (3H, d, *J* = 6 Hz), 0.97, 0.95 (6H, br s), 0.90, 0.87 (3H, s), 0.69, 0.69 (3H, s);
¹³C NMR (90 MHz, CDCl₃) δ ppm : 198.5 (s), 166.5 (s), 152.8 (s), 150.8 (s), 148.1 (s), 143.0 (s), 136.7 (s), 136.6 (s), 134.3 (s), 131.9 (s), 131.5 (d), 131.0 (d), 129.8 (d), 129.5 (d), 129.5 (d), 129.3 (d), 128.8 (d), 124.8 (d), 122.6 (d), 121.5 (d), 78.3 (d), 78.2 (d), 54.3 (d), 48.1 (s), 48.1 (s), 39.9 (s), 35.5 (t), 34.4 (d), 29.1 (d), 25.4 (q), 24:2 (q), 24.2 (q), 23.7 (q), 23.7 (q), 23.4 (q), 23.2 (q), 20.5 (q), 14.8 (q), 14.7 (q), 12.7 (q);
Nanospray MS: *m*/*z* (relative abundance) 537.4 ([M + Na]⁺, 100), 515.2 ([M + H]⁺, 2).

### Example 2

### Preparation of α-keto esters

The bis(3,7-dimethyl-6-octenyl)oxalate which was used for the synthesis of some of the α-keto esters described below was prepared as follows.
Oxalyl chloride (10 ml, 116 mmol) was added dropwise to a stirred solution of 36.37 g (233 mmol) of citronellol in 300 ml of pyridine at 0°C over a period of 30 min. The formation of a white precipitate was observed. The solution was allowed to warm up at room temperature over night and was quenched with water, extracted with diethyl ether (2x), H₂SO₄ (10%) (2x), NaHCO₃ (10%) and saturated NaCl. The organic layer was dried over Na₂SO₄, concentrated at reduced pressure and filtered over a short plug (SiO₂, heptane/diethyl ether). Column chromatography (SiO₂, heptane/diethyl ether) gave 18.55 g (43%) of a colorless oil.
IR (neat): 2965*s*, 2925*s*, 2873*m*, 2856*m*, 1770*s*, 1745*s*, 1457*m*, 1380*m*, 1347*w*, 1312*m*, 1250*w*, 1170*s*, 1122*w*, 1044*w*, 941*m*, 886*w*, 831*w*, 792*w*, 756*w*, 742*w*.
¹H NMR (360 MHz, CDCl₃): 5.13-5.04 (*m*, 1 H); 4.40-4.23 (*m*, 2 H); 2.08-1.87 (*m*, 2 H); 1.85- .71 (*m*, 1 H); 1.70-1.50 (*m*, 2 H); 1.68 (*s*, 3 H); 1.60 (*s*, 3 H); 1.43-1.29 (m, 1 H); 1.29-1.13 (m, 1 H); 0.94 (*d*, *J* = 6.3, 3 H).
¹³C NMR (90.6 MHz, CDCl₃): 158.04 (*s*); 131.45 (*s*); 124.42 (*d*); 65.59 (*t*); 36.91 (*t*); 35.08 (*t*); 29.42 (*d*); 25.70 (*q*); 25.36 (*t*); 19.36 (*q*); 17.65 (*q*).
MS (EI): 336 (M⁺, 0.1); 228 (0.1); 183 (0.1); 165 (0.1); 138 (18); 123 (30); 109 (16); 95 (38); 81 (51); 69 (100); 55 (30); 41 (46); 29 (5).

### a) 3,7-Dimethyl-6-octenyl-2-oxopropanoate (5)

A stirred solution of 5.56 g (63 mmol) of 2-oxo propionic acid and 19.68 g (126 mmol) of citronellol in 150 ml of toluene was heated for 35 h under reflux with azeotropic removal of water. After cooling to room temperature the reaction mixture was extracted with diethyl ether (2x), 10% NaHCO₃, sat. NaCl, dried (Na₂SO₄) and concentrated *in vacuo*. Column chromatography (SiO₂, pentane/ether 9:1) afforded 2.81 g (20%) of a colorless oil.
UV/Vis (hexane): 388 (sh, 3); 378 (sh, 5); 369 (sh, 8); 360 (sh, 10); 345 (14); 334 (14); 319 (sh, 12); 284 (sh, 9).
IR (neat): 2961*m*, 2915*m*, 2873*m*, 2856*m*, 1728*s*, 1454*m*, 1378*m*, 1357*m*, 1297*m*, 1266*m*, 1203*w*, 1134*s*, 1051*m*, 1024*w*, 982*m*, 937*m*, 830*m*, 771*w*, 720*m*, 663*w*.
¹H NMR (360 MHz, CDCl₃): 5.15-5.03 (*m*, 1 H); 4.37-4.18 (*m*, 2 H); 2.47 (*s*, 3 H); 2.10-1.88 (m, 2 H); 1.87-1.71 (m, 1 H); 1.71-1.47 (*m*, 2 H); 1.68 (*s*; 3 H); 1.60 (*s*, 3 H); 1.46-1.28 (m, 1 H); 1.28-1.12 (m, 1 H); 0.94 (*d*, *J* = 6.3, 3 H).
¹³C NMR (90.6 MHz, CDCl₃): 191.96 (*s*); 160.92 (*s*); 131.52 (*s*); 124.37 (*d*); 65.06 (*t*); 36.89 (*t*); 35.14 (*t*); 29.39 (*d*); 26.73 (*q*); 25.71 (*q*); 25.33 (*t*); 19.36 (*q*); 17.6 (*q*).
MS (EI): 226 (M⁺, 3); 209 (1); 208 (5); 198 (1), 184 81); 183 (9); 165 (2); 156 (1); 155 (14); 139 (1); 138 (15); 137 (20); 136 (1); 124 (3); 123 (29); 121 (3); 111 (1); 110 (5); 109 (20); 99 (1); 97 (2); 96 (8); 95 (45); 94 (2); 93 (1); 91 (1); 90 (1); 84 (1); 83 (15); 82 (28); 81 (51); 80 (2); 79 (2); 77 (1); 71 (1); 70 (10); 69 (100); 68 (14); 67 (23); 66 (1); 65 (2); 57 (5); 56 (8); 55 (34); 54 (2); 53 (7); 44 (1); 43 (41); 42 (5); 41 (40); 40 (2); 39 (6); 29 (4); 27 (3).

### b) 3,7-Dimethyl-6-octenyl-2-oxobutanoate (6)

The synthesis was carried out as described above under a) with 6.43 g (63 mmol) of 2-oxo butyric acid, 19.68 g (126 mmol) of citronellol and 150 ml of toluene (24 h). Column chromatography (SiO₂, pentane/ether 9:1) afforded 7.80 g (52%) of a colorless oil.
UV/Vis (hexane): 397 (sh, 1); 383 (sh, 3); 373 (sh, 6); 356 (sh, 12); 341 (16); 330 (16); 318 (sh, 14); 268 (sh, 12).
IR (neat): 2961*m*, 2914*m*, 2879*m*, 2857*m*, 1725*s*, 1456*m*, 1404*w*, 1379*m*, 1351*w*, 1273*m*, 1242*m*, 1173*w*, 1144*m*, 1097*s*, 1041*m*, 982*m*, 946*w*, 881*w*, 830*m*, 760*w*, 737*w*, 700*m*, 678*m*.
¹H NMR (360 MHz, CDCl₃): 5.14-5.02 (*m*, 1 H); 4.40-4.20 (*m*, 2 H); 2.86 (*q*, *J* = 7.3, 2 H); 2.09-1.88 (*m*, 2 H); 1.87-1.68 (*m*, 1 H); 1.68 (*s*, 3 H); 1.68-1.45 (*m*, 2 H); 1.60 (*s*, 3 H); 1.45-1.29 (*m*, 1 H); 1.29-1.15 (*m*, 1 H); 1.13 (*t*, *J =* 7.1, 3 H); 0.94 *(d, J* = 6.3, 3 H).
¹³C NMR (90.6 MHz, CDCl₃): 195.09 (*s*); 161.32 (*s*); 131.51 (*s*); 124.40 (*d*); 64.87 (*t*); 36.90 (*t*); 35.17 (*t*); 32.89 (*t*); 29.40 (*d*); 25.71 (*q*); 25.34 (*t*); 19.37 (*q*); 17.66 (*q*); 6.97 (*q*).
MS (EI): 240 (M⁺, 1); 222 (3); 212 (2); 184 (1); 183 (8); 165 (1); 156 (1); 155 (12); 139 (3); 138 (20); 137 (15); 136 (1); 124 (3); 123 (31); 121 (3); 111 (2); 110 (4); 109 (16); 104 (2); 99 (1); 97 (3); 96 (9); 95 (43); 94 (3); 93 (2); 91 (1); 85 (1); 84 (2); 83 (17); 82 (31); 81 (51); 80 (3); 79 (2); 77 (1), 71 (1); 70 (8); 69 (100); 68 (13); 67 (19); 66 (1); 65 (2); 58 (2); 57 (63); 56 (7); 55 (30); 54 (2); 53 (6); 43 (6); 42 (4); 41 (38); 40 (1); 39 (5); 29 (17); 28 (2); 27 (5).

### c) 3,7-Dimethyl-6-octenyl 3-methyl-2-oxopentanoate (7)

The synthesis was carried out as described above under a), using 4.85 g (38 mmol) of 3-methyl-2-oxo pentanoic acid and 11.66 g (74 mmol) of citronellol in 130 ml of toluene, for 72 h. Column chromatography (SiO₂, toluene/EtOAc) afforded 10 g of crude product, which was fractionally distilled to give 3.65 g (36%) of a colorless oil.
B.p. 94°C/2x10¹ Pa.
UV/Vis (hexane): 394 (sh, 4), 382 (sh, 10), 374 (sh, 10), 365 (sh, 10), 350 (sh, 20), 336 (20), 268 (sh, 30), 241 (sh, 180).
IR (neat): 2966*s*, 2929*s*, 2877*m*, 1749*m*, 1728*s*, 1460*m*, 1380*m*, 1267*m*, 1254*m*, 1165*m*, 1115*w*, 1087*w*, 1051*m*, 1001*w*, 961*w*, 829*w*.
¹H NMR (360 MHz, CDCl₃): 5.12-5.04 (*m*, 1 H); 4.36-4.24 (*m*, 2 H); 3.18-3.06 (*m*, 1 H); 2.08-1.88 (*m*, 2 H); 1.86-1.67 (*m*, 2 H); 1.68 (*s*, 3 H); 1.65-1.10 (*m*, 5, H); 1.60 (*s*, 3 H); 1.28 (*d*, *J* = 6.8, 3 H); 0.94 (*d*, *J* = 6.4, 3 H); 0.92 (*t*, *J* = 7.6, 3 H).
¹³C NMR (90.6 MHz, CDCl₃): 198.22 (*s*); 162.21 (*s*); 131.51 (*s*); 124.40 (*d*); 64.74 (*t*); 43.64 (*d*); 36.92 (*t*); 35.23 (*t*); 29.43 (*d*); 25.71 (*q*); 25.36 (*t*); 24.93 (*t*); 19.35 (*q*); 17.66 (*q*); 14.55 (*q*); 11.35 (*q*).
MS (EI): 268 (M⁺, 1); 250 (1); 240 (1); 207 (1); 183 (2); 155 (2); 138 (10); 123 (14); 109 (7); 95 (18); 85 (32); 81 (26); 69 (51); 57 (100); 41 (53); 29 (18).

### d) 3,7-Dimethyl-6-octenyl 2-oxopentanoate (8)

The synthesis was carried out as described above under a), using 4.33 g (37 mmol) of 2-oxo pentanoic acid and 11.65 g (75 mmol) of citronellol. Column chromatography (SiO₂, toluene/EtOAc and SiO₂, heptane/diethyl ether) afforded 3.79 g of crude product, which was distilled (Kugelrohr) to give 2.52 g (27%) of a colorless oil.
UV/Vis (hexane): 398 (sh, 1), 376 (sh, 10), 357 (sh, 10), 342 (sh, 20), 331 (20), 281 (sh, 20), 268 (sh, 30), 241 (sh, 280).
IR (neat): 2965*s*, 2931*s*, 2877*m*, 1750*m*, 1728*s*, 1457*m*, 1380*m*, 1287*w*, 1261*m*, 1178*w*, 1146*w*, 1118*m*, 1055*m*, 1037*w*, 943*w*, 832*w*.
¹H NMR (360 MHz, CDCl₃): 5.13-5.03 (*m*, 1 H); 4.36-4.21 (*m*, 2 H); 2.80 (*t, J =* 7.1, 2 H); 2.10-1.89 (*m*, 2 H); 1.83-1.70 (*m*, 1 H); 1.68 (*s*, 3 H); 1.67 (*q, J =* 7.3, 2 H); 1.63-1.47 (m, 2 H); 1.60 (*s*, 3 H); 1.45-1.29 (m, 1 H); 1.28-1.12 (*m*, 1 H); 0.96 (*t, J =* 6.9, 3 H); 0.94 (*d, J =* 6.3, 3 H).
¹³C NMR (90.6 MHz, CDCl₃): 194.63 (*s*); 161.44 (*s*); 131.52 (*s*); 124.40 (*d*); 64.88 (*t*); 41.21 (*t*); 36.91 (*t*); 35.19 (*t*); 29.43 (*d*); 25.71 (*q*); 25.35 (*t*); 19.37 (*q*); 17.67 (*q*); 16.54 (*t*); 13.52 (*q*).
MS (EI): 254 (M⁺, 1); 236 (2); 226 (1); 193 (1); 183 (6); 165 (1); 155 (7); 138 (15); 137 (10); 123 (26); 118 (3); 109 (17); 95 (41); 83 (15); 82 (32); 81 (54); 71 (87); 69 (100); 67 (23); 55 (34); 43 (66); 41 (72); 27 (14).

### e) 3,7-Dimethyl-6-octenyl oxo(phenyl)acetate (9)

A Grignard reagent prepared from 3.14 g of 1-bromobenzene (20 mmol) and 0.55 g of magnesium (22 mmol) in THF was added dropwise to a stirred solution of 8.0 g (22 mmol) of bis(3,7-dimethyl-6-octenyl)oxalate in 50 ml of THF at -78°C. The mixture was slowly warmed to -10°C, quenched with 25-30 ml of a saturated solution of NH₄Cl and left stirring for 30 min. The reaction mixture was extracted with diethyl ether and water (3x) and the organic phase dried over Na₂SO₄. MPLC on a *Lobar* column (SiO₂ *Merck*, heptane/diethyl ether) afforded 3.5 g (61%) of the pure product as a bright yellow oil.
UV/Vis (hexane): 370 (sh, 30), 352 (40), 340 (sh, 40), 294 (sh, 1020), 252 (10350), 248 (10360).
IR (neat): 3065*w*, 2962*s*, 2926*s*, 2872*m*, 2855*m*, 1738*s*, 1693*s*, 1597*m*, 1581*m*, 1451*m*, 1379*m*, 1322*m*, 1313*m*, 1300*m*, 1246*w*, 1198*s*, 1175*s*, 1122*w*, 1042*w*, 1030*w*, 1003*m*, 998*m*, 941*w*, 831*w*.
¹H NMR (360 MHz, CDCl₃): 8.04-7.97 (*m*, 2 H); 7.69.7.62 (*m*, 1 H); 7.55-7.45 (*m*, 2 H); 5.12-5.03 (*m*, 1 H); 4.50-4.36 (*m*, 2 H); 2.15-1.90 (*m*, 2 H); 1.90-1.75 (*m*, 1 H); 1.75-1.50 (*m*, 2 H); 1.66 (*s*, 3 H); 1.59 (*s*, 3 H); 1.45-1.32 (*m*, 1 H); 1.32-1.15 (*m*, 1 H); 0.96 (*d*, *J* = 6.3, 3 H).
¹³C NMR (90.6 MHz, CDCl₃): 186.50 (*s*); 164.02 (*s*); 134.87 (*d*); 132.56 (*s*); 131.51 (*s*); 130.02 (*d*); 128.90 (*d*); 124.40 (*d*); 64.85 (*t*); 36.93, (*t*); 35.30 (*t*); 29.44 (*d*); 25.69 (*q*); 25.38 (*t*); 19.38 (*q*); 17.66 (*q*).
MS (EI): 288 (M⁺, 1); 270 (4); 260 (1); 227 (1); 215 (1); 187 (1); 183 (1); 174 (1); 165 (1); 155 (4); 152 (3); 138 (9); 137 (10); 134 (2); 123 (11); 109 (8); 106 (10); 105 (100); 96 (3); 95 (20); 83 (3); 82 (12); 81 (24); 80 (2); 78 (3); 77 (36); 70 (3); 69 (26); 68 (5); 67 (10); 57 (3); 56 (3); 55 (11); 53 (3); 51 (10); 43 (4); 42 (3); 41 (28); 39 (5); 29 (4); 27 (4).

### f) 3,7-Dimethyl-6-octenyl (4-acetylphenyl)oxoacetate (10)

In the first step, 2-(4-bromomethyl)-2-methyl-1,3-dioxolane was prepared as follows. 10.0 g (50 mmol) of 4-bromo acetophenone, 7.0 g (112 mmol) of ethylene glycol and a few crystals of *p*-toluene sulphonic acid were dissolved in 100 ml of toluene and heated overnight under reflux with azeotropic removal of water. After cooling to room temperature the reaction mixture was concentrated *in vacuo*. Column chromatography (SiO₂, heptane/diethyl ether) afforded 11.4 g (93%) of a colorless oil which easily crystallized.
UV/Vis (hexane): 287 (sh, 400), 274 (sh, 1300), 270 (sh, 1800), 259 (sh, 6700), 252 (7800), 227 (sh, 61800), 220 (75600), 217 (sh, 75000).
IR (neat): 3084*w*, 3060*w*, 2990*m*, 2957*s*, 2928*s*, 2890*s*, 2856*m*, 2670*w*, 1911*w*, 1691*m*, 1657*w*, 1591*m*, 1575*w*, 1482*m*, 1470*w*, 1443*m*, 1393*m*, 1373*m*, 1249*m*, 1222*w*, 1196*s*, 1144*m*, 1118*m*, 1092*m*, 1079*m*, 1040*s*, 1010*s*, 947*m*, 873*s*, 826*s*.
¹H NMR (360 MHz, CDCl₃): 7.49-7.42 (*m*, 2 H); 7.39-7.32 (*m*, 2 H); 4.08-3.96 (*m*, 2 H); 3.80-3.69 (*m*, 2 H); 1.62 (*s*, 3 H).
¹³C NMR (90.6 MHz, CDCl₃): 142.49 (*s*); 131.30 (*d*); 127.17 (*d*); 121.86 (*s*); 108.43 (*s*); 64.47 (*t*); 27.52 (*q*).
MS (EI): 244, 242 (M⁺, 1, 1); 230 (14); 229 (97); 227 (100); 213 (5); 211 (5); 186 (4); 185, 183 (51, 53); 171 (2); 169 (2); 157, 155 (14, 14); 148 (4); 133 (5); 105 (2); 104 (8); 103 (9); 102 (8); 101 (2); 89 (3); 87 (26); 78 (2); 77 (12); 76 (16); 75 (14); 74 (7); 73 (2); 63 (4); 62 (2); 51 (7); 50 (13); 43 (41); 39 (3); 29 (7).

The thus obtained compound was then used as starting product for the synthesis of 3,7-dimethyl-6-octenyl [4-(2-methyl-1,3-dioxolan-2-yl)phenyl]oxoacetate. The synthesis was carried out as described above under e), using 4.66 g (20 mmol) of the above-prepared dioxolane, 0.54 g (22 mmol) of magnesium and 8.0 g (22 mmol) of bis(3,7-dimethyl-6-octenyl)oxalate. Column chromatography (SiO₂, heptane/diethyl ether) afforded 4.35 g (58%) of the product as a slightly yellow oil.
UV/Vis (hexane): 370 (sh, 40), 353 (60), 340 (sh, 60), 296 (sh, 1300), 258 (13890).
IR (neat): 2963*s*, 2926*s*, 1736*s*, 1690*s*, 1607*s*, 1573*m*, 1505*w*, 1455*m*, 1407*m*, 1374*m*, 1347*w*, 1314*m*, 1294*w*, 1250*m*, 1199*s*, 1175*s*, 1146*w*, 1122*w*, 1100*w*, 1078*m*, 1039*m*, 1018*w*, 989*m*, 948*w*, 890*w*, 876*m*, 861*m*, 833*w*.
¹H NMR (360 MHz, CDCl₃): 7.98 (*d*, *J* = 8.3, 2 H); 7.62 (*d*, *J* = 8.7, 2 H); 5.12-5.04 (*m*, 1 H); 4.50-4.36 (*m*, 2 H); 4.13-4.00 (*m*, 2 H); 3.82-3.70 (*m*, 2 H); 2.10-1.90 (*m*; 2 H); 1.90-1.75 (*m*, 1 H); 1.72-1.54 (*m*, 2 H); 1.67 (*s*, 3 H); 1.65 (*s*, 3 H); 1.60 (*s*, 3 H); 1.45-1.32 (*m*, 1 H); 1.30-1.16 (*m*, 1 H); 0.96 (*d*, *J*= 6.3, 3 H).
¹³C NMR (90.6 MHz, CDCl₃): 186.04 (*s*); 163.97 (*s*); 150.64 (*s*); 132.12 (*s*); 131.53 (*s*); 130.15 (*d*); 125.97 (*d*); 124.39 (*d*); 108.39 (*s*); 64.89 (*t*); 64.65 (2x) (*t*); 36.93 (*t*); 35.30 (*t*); 29.44 (*d*); 27.38 (*q*); 25.70 (*q*); 25.37 (*t*); 19.38 (*q*); 17.66 (*q*).
MS (EI): 374 (M⁺, 7); 359 (8); 356 (3); 289 (1); 220 (2); 205 (1); 192 (32); 191 (100); 176 (2); 160 (2); 155 (2); 148 (24); 138 (16); 133 (6); 123 (14); 119 (76); 109 (9); 104 (15); 95 (22); 91 (8); 87 (18); 81 (30); 69 (26); 55 (10); 43 (12); 41 (21); 29 (3).

### 3,7-Dimethyl-6-octenyl (4-acetylphenyl)oxoacetate (10)

5 ml of H₂SO₄ (50%) were added to a solution of 4.2 g (13 mmol) of the product obtained in the above step in 30 ml of THF. The reaction mixture was heated at 40°C for 5 h, then extracted with diethyl ether (2x), and saturated solutions of NaHCO₃ (2x) and NaCl (2x). The organic layer was dried over Na₂SO₄ and concentrated. Column chromatography (SiO₂, heptane/diethyl ether) yielded 2.0 g (47%) of a yellow oil.
UV/Vis (hexane): 384 (sh, 60), 367 (sh, 100), 343 (sh, 150), 310 (sh, 1230), 301 (sh, 1660), 266 (17910), 260 (18440).
IR (neat): 3051*w*, 2964*s*, 2926*s*, 2872*m*, 2856*m*, 1736*s*, 1693*s*, 1607*w*, 1570*m*, 1500*m*, 1457*m*, 1434*m*, 1407*m*, 1379*m*, 1359*m*, 1318*m*, 1307*m*, 1260*s*, 1199*s*, 1176*s*, 1117*w*, 1075*m*, 992*s*, 959*m*, 861*m*, 832*m*.
¹H NMR (360 MHz, CDCl₃): 8.17-8.02 (*m*, 4 H); 5.12-5.04 (*m*, 1 H); 4.53-4.37 (*m*, 2 H); 2.66 (*s*, 3 H); 2.14-1.90 (*m*, 2 H); 1.90-1.75 (*m*, 1 H); 1.73-1.53 (*m*, 2 H); 1.67 (*s*, 3 H); 1.60 (*s*, 3 H); 1.46-1.32 (*m*, 1 H); 1.32-1.12 (*m,* 1 H); 0.96 (*d*, *J=* 6.3, 3 H).
¹³C NMR (90.6 MHz, CDCl₃): 197.19 (*s*); 185.55 (*s*); 163.25 (*s*); 141.33 (*s*); 135.67 (*s*); 131.57 (*s*); 130.28 (*d*); 128:56 (*d*); 124.34 (*d*); 65.19 (*t*); 36.91 (*t*); 35.26 (*t*); 29.43 (*d*); 26.94 (*q*); 25.70 (*q*); 25.35 (*t*); 19.37 (*q*); 17.67 (*q*).
MS (EI): 330 (M⁺, 4); 312 (1); 302 (1); 281 (1); 269 (1); 194 (4); 193 (2); 183 (1); 176 (2); 165 (1); 161 (1); 155 (2); 149 (5); 148 (43); 147 (100); 138 (4); 137 (11); 133 (1); 132 (2); 123 (10); 120 (4); 119 (11); 110 (2); 109 (10);105 (2); 104 (12); 96 (4); 95 (21); 91 (15); 83 (5); 82 (13); 81 (29); 77 (6); 76 (8); 69 (38); 68 (5); 67 (11); 65 (3); 57 (3); 56 (3); 55 (12); 53 (3); 50 (3); 43 (15); 41 (30); 39 (5); 29 (4); 27 (3).

### g) 3,7-Dimethyl-6-octenyl 3-methyl-2-oxopentadecanoate (11)

The compound was prepared as described above under e), using 5.0 g (18 mmol) of 2-bromotetradecane, 0.58 g (24 mmol) of magnesium and 7.32 g (20 mmol) of bis(3,7-dimethyl-6-octenyl)oxalate. Column chromatography (SiO₂, heptane/diethyl ether) afforded 2.52 g (34%) of a colorless oil.
UV/Vis (hexane): 394 (sh, 4), 383 (sh, 10), 373 (sh, 10), 365 (sh, 20), 349 (sh, 20), 336 (20), 284 (sh, 10), 269 (sh, 20), 241 (sh, 140).
IR (neat): 3440*w*, 2958*s*, 2924*s*, 2854*s*, 2730*w*, 1749*s*, 1725*s*, 1460*m*, 1378*m*, 1350*w*, 1266*m*, 1173*w*, 1146*w*, 1112*w*, 1053*m*, 1032*m*, 943*w*, 887*w*, 830*w*.
¹H NMR (360 MHz, CDCl₃): 5.13-5.04 (*m*, 1 H); 4.36-4.23 (*m*, 2 H); 3.23-3.10 (*m*, 1 H); 2.10-1.87 (*m*, 2 H); 1.87-1.64 (*m*, 1 H); 1.68 (*s*, 3 H); 1.64-1.47 (*m*, 2 H); 1.60 (*s*, 3 H); 1.46-1.16 (*m*, 24 H); 1.13 (*d*, *J* = 6.7, 3 H); 0.94 (*d*, *J*= 6.3, 3 H); 0.88 (*t*, *J*= 6.9, 3 H).
13C NMR (90.6 MHz, CDCl₃): 198.33 (*s*); 162.20 (*s*); 131.50 (*s*); 124.40 (*d*); 64.75 (*t*); 42.21 (*d*); 36.93 (*t*); 35.23 (*t*); 31.92 (*t*); 29.68 (*t*); 29.66 (2x) (*t*); 29.59 (2x) (*t*); 29.45 (2x) (*t*); 29.37 (*t*); 27.01 (*t*); 25.71 (*q*); 25.37 (*t*); 22.70 (*t*); 19.35 (*q*); 17.66 (*q*); 15.01 (*q*); 14.12 (*q*).
MS (EI): 408 (M⁺, 1); 390 (1); 380 (1); 347 (1); 294 (1); 272 (1); 255 (4); 205 (1); 197 (3); 184 (2); 183 (12); 165 (1); 155 (8); 141 (4); 139 (9); 138 (76); 137 (21); 127 (7); 123 (46); 113 (9); 109 (19); 99 (15); 96 (15); 95 (57); 94 (8); 85 (47); 83 (25); 82 (52); 81 (89); 80 (14); 71 (65); 70 (10); 69 (100); 68 (10); 67 (18); 57 (94);56 (17); 55 (51); 43 (61); 41 (69); 39 (7); 29 (15); 27 (6).

### h) 3,7-Dimethyl-6-octenyl 2-oxohexadecanoate (12)

The compound was prepared as described above under e), using 5.54 g (20 mmol) of 1-bromotetradecane, 0.54 g (22.5 mmol) of magnesium and 8.0 g (22 mmol) of bis(3,7-dimethyl-6-octenyl)oxalate. Column chromatography (SiO₂, heptane/diethyl ether) afforded 3.21 g (39%) of a colorless oil.
UV/Vis (hexane): 376 (sh, 10), 359 (sh, 20), 343 (sh, 20), 279 (260), 272 (sh, 250), 242 (530).
IR (neat): 2958*m*, 2924*s*, 2854*s*, 1728*s*, 1465*m*, 1458*m*, 1400*w*, 1378*m*, 1271*m*, 1128*w*, 1088*w*, 1062*m*, 945*w*, 831*w*.
¹H NMR (360 MHz, CDCl₃): 5.12-5.03 (m, 1 H); 4.35-4.21 (m, 2 H); 2.81 (t, J = 7.3, 2 H); 2.09-1.88 (*m*, 2 H); 1.87-1.69 (*m*, 1 H); 1.68 (*s*, 3 H); 1.69-1.47 (*m*, 2 H); 1.60 (*s*, 3 H); 1.45-1.14 (*m*, 26 H); 0.94 (*d*, *J* = 6.3, 3 H); 0.88 (*t*, *J* = 6.9, 3 H).
¹³C NMR (90.6 MHz, CDCl₃): 194.77 (*s*); 161.48 (*s*); 131.49 (*s*); 124.41 (*d*); 64.86 (*t*); 39.38 (*t*); 36.93 (*t*); 35.20 (*t*); 31.96 (*t*); 29.68 (3x) (*t*); 29.61 (*t*); 29.45 (2x) (*t*); 29.39 (*t*); 29.33 (*t*); 29.01 (*t*); 25.71 (*q*); 25.37 (*t*); 23.05 (*t*); 22.71 (*t*); 19.38 (*q*); 17.66 (*q*); 14.12 (*q*).
MS (EI): 390 (1), 225 (11), 183 (14), 165 (1), 155 (8), 139 (7), 138 (55), 137 (28), 124 (6), 123 (52), 121 (5), 111 (4), 110 (7), 109 (27), 97 (9), 96 (16), 95 (70), 94 (8), 85 (16), 83 (28), 82 (50), 81 (97), 80 (10), 71 (26), 70 (11), 69 (100), 68 (11), 67 (21), 57 (54), 56 (12), 55 (47), 43 (48), 42 (10), 41 (55), 39 (7), 29 (12).

### i) 3,7-Dimethyl-6-octenyl (cyclohexyl)oxoacetate (13)

The compound was prepared as described above under e), using 3.24 g (20 mmol) of freshly distilled 1-bromocyclohexane, 0.55 g (22 mmol) of magnesium and 8.0 g (22 mmol) of bis(3,7-dimethyl-6-octenyl)oxalate. MPLC on a *Lobar* column (SiO₂ *Merck,* heptane/diethyl ether) finally afforded 1.69 g (29%) of the pure product as a colorless oil.
UV/Vis (hexane): 394 (sh, 4), 375 (sh, 11), 366 (sh, 14), 350 (sh, 18), 338 (19).
IR (neat): 2932*s*, 2856*m*, 1747*m*, 1727*s*, 1451*m*, 1379*m*, 1311*w*, 1276*m*, 1230*m*, 1183*w*, 1173*w*, 1140*m*, 1118*w*, 1082*m*, 1067*m*, 1050*w*, 1029*w*, 997*m*, 942*w*, 895*w*, 837*w*.
¹H NMR (360 MHz, CDCl₃): 5.12-5.04 (m, 1 H); 4.36-4.22 (m, 2 H); 3.07-2.95 (m, 1H); 2.09-1.85 (m, 4 H); 1.85-1.64 (m, 3 H); 1.68 (s, 3 H); 1.64-1.47 (m, 2 H); 1.60 (s, 3 H); 1.43-1.13 (m, 8 H); 0.93 (d, J = 6.3, 3 H).
¹³C NMR (90.6 MHz, CDCl₃): 197.65 (*s*); 162.17 *(s);* 131.51 (*s*); 124.39 (d); 64.71 (*t*); 46.34 (*d*); 36.91 (*t*); 35.21 (*t*); 29.44(*d*); 27.46 (*t*); 25.72 (*t*); 25.36 (*t*); 25.30 (*t*); 19.35 (*q*); 17.66 (*q*).
MS (EI): 294 (M⁺, 1); 276 (1); 266 (1); 233 (1); 193 (1); 183 (4); 165 (1); 155 (2); 139 (2); 138 (13); 137 (4); 123 (14); 112 (2); 111 (16); 110 (3); 109 (6); 96 (4); 95 (16); 94 (2); 84 (7); 83 (100); 82 (15); 81 (22); 80 (3); 70 (2); 69 (29); 68 (4); 67 (11); 56 (4); 55 (42); 54 (3); 53 (5); 43 (4); 42 (4); 41 (38); 39 (8); 29 (6); 27 (4).

### k) (E)-3,7-Dimethyl-2,6-octadienyl(cyclohexyl)oxoacetate (14)

In the first step, ethyl (cyclohexyl)oxoacetate was prepared as follows.
A Grignard reagent prepared from 24.45 g of 1-bromocyclohexane (0.18 mol) and 4.32 g of magnesium (0.15 mol) in 70 ml THF was added dropwise (during a period of 40 min) to a stirred solution of 14.6 g (0.10 mol) of diethyl oxalate in 150 ml of THF at -70°C. The formation of a precipitate was observed and another 100 ml of THF were added. The mixture was slowly warmed to -10°C and poured onto ice, saturated with NaCl, extracted with diethyl ether (2x) and washed with a sat. solution of NH₄Cl (2x) and water (pH ≈ 7). The organic phase was dried over Na₂SO₄ and concentrated. Fractional distillation gave 9.86 g (54%) of a colorless oil.
B.p. 54°C/0.1-150 Pa (0.1-1.5 mbar).
UV/Vis (hexane): 394 (sh, 5); 375 (sh, 10); 366 (sh, 15); 350 (sh, 20); 337 (20); 285 (sh, 7).
IR (neat): 2982*w*, 2930*m*, 2854*m*, 1722*s*, 1449*m*, 1366*w*, 1272*m*, 1229*m*, 1184*w*, 1140*m*, 1112*w*, 1081*m*, 1066*s*, 1014*m*, 991*m*, 923*w*, 894*w*, 855*w*.
¹H NMR (360 MHz, CDCl₃): 4.32 (*q*, *J*= 7.1, 2 H); 3.1-2.97 (*m*, 1 H); 1.97-1.85 (*m*, 2 H); 1.85-1.74 (*m*, 2 H); 1.74-1.64 (*m*, 1 H); 1.45-1.13 (*m*, 5 H); 1.37 (*t*, *J* = 7.1, 3 H).
¹³C NMR (90.6 MHz, CDCl₃): 197.65 (*s*); 162.03 (*s*); 62.19 (*t*); 46.29 (*d*); 27.51 (*t*); 25.73 (*t*); 25.32 (*t*); 14.06 (*q*).
MS (EI): 184 (M⁺, 2); 112 (3); 111 (33); 110 (3); 84 (6); 83 (100); 81 (3); 79 (2); 77 (1); 68 (1); 67 (5); 65 (1); 56 (3); 55 (54); 54 (5); 53 (5); 51 (1); 43 (2); 42 (3); 41 (23); 40 (2); 39 (12); 30 (1); 29 (20); 28 (3); 27 (13); 26 (1).

### (E)-3,7-Dimethyl-2,6-octadienyl (cyclohexyl)oxoacetate (14)

A solution of 25.20 g (137 mmol) of the product obtained above, 25.56 g (166 mmol) of geraniol and 1 ml of NaOCH₃ (30% in methanol) in 150 ml of cyclohexane was heated under reflux overnight. After cooling to room temperature the reaction mixture was taken up in ether, washed with a sat. solution of NaCl (pH ≈ 7), dried (Na₂SO₄), filtered and concentrated. Column chromatography (SiO₂, heptane/ether 9:1) and fractional distillation afforded 23.36 g (58%) of a colorless oil.
B.p. 130°C/10 Pa (0.1 mbar).
UV/Vis (hexane): 394 (sh, 5); 384 (sh, 8); 375 (sh, 14); 366 (sh, 17); 358 (sh, 20); 350 (sh, 22); 336 (24).
IR (neat): 2926*m*, 2853*m*, 1743*m*, 1721*s*, 1670*w*, 1449*m*, 1376*m*, 1341*w*, 1331*w*, 1309*w*, 1273*m*, 1267*m*, 1227*m*, 1183*w*, 1139*m*, 1111*w*, 1080*m*, 1063*s*, 1027*w*, 993*s*, 915*m*, 895*w*, 830*w*, 805*w*, 787*w*, 739*w*, 729*w*, 718*w*.
¹H NMR (360 MHz, CDCl₃): 5.45-5.35 (*m*, 1 H); 5.12-5.03 (*m*, 1 H); 4.76 (*d*, *J* = 7.1, 2 H); 3.09-2.95 (*m*, 1 H); 2.17-1.98 (*m*, 4 H); 1.98-1.85 (*m*, 2 H); 1.84-1.75 (*m*, 2 H); 1.74 (*s*, 3 H); 1.73-1.62 (*m*, 1 H); 1.68 (*s*, 3 H); 1.60 (*s*, 3 H); 1.43-1.14 (*m*, 5 H).
¹³C NMR (90.6 MHz, CDCl₃): 197.70 (*s*); 162.08 (*s*); 143.97 (*s*); 131.97 (*s*); 123.59 (*d*); 117.16 (*d*); 62.90 (*t*); 46.38 (*d*); 39.55 (*t*); 27.49 (*t*); 26.23 (*t*); 25.73 (*t*); 25.67 (*q*); 25.31 (*t*); 17.69 (*q*); 16.58 (*q*).
MS (EI): 292 (M⁺, 1); 205 (1); 179 (1); 138 (3); 137 (24); 136 (4); 135 (3); 123 (1); 122 (1); 121 (2); 112 (1); 111 (9); 107 (2); 105 (1); 96 (1); 95 (9); 94 (1); 93 (9); 92 (2); 91 (3); 84 (4); 83 (54); 82 (4); 81 (55); 80 (2); 79 (4); 77 (3); 70 (6); 69 (100); 68 (12); 67 (12); 65 (1); 56 (1); 55 (24); 54(2); 53 (6); 43 (2); 42 (2); 41 (25); 40 (1); 39 (5); 29 (2); 27 (2).

### l) Decyl (cyclohexyl)oxoacetate (15)

The synthesis was carried out as described above under k), using 6.21 g (33.4 mmol) of ethyl (cyclohexyl)oxoacetate, 5.75 g (36.4 mmol) of decanol, 0.5 ml of NaOCH₃ (30% in methanol) and 50 ml of cyclohexane. Fractional distillation afforded 3.85 g (39%) of a colorless oil.
B.p. 118-126°C/20 Pa (0.2 mbar).
UV/Vis (hexane): 394 (sh, 4); 382 (sh, 8); 376 (sh, 11); 367 (sh, 14); 358 (sh, 17); 350 (sh, 19); 336 (19); 314 (sh, 17); 302 (sh, 15).
IR (neat): 2924*s*, 2852*m*, 1745*m*, 1723*s*, 1466*m*, 1450*m*, 1377*w*, 1330*w*, 1310*w*, 1290*w*, 1274*m*, 1229*m*, 1183*w*, 1139*m*, 1117*w*, 1082*m*, 1065*m*, 1028*w*, 995*m*, 929*w*, 895*w*, 867*w*, 802*w*, 785*w*, 720*m*, 662*w*.
¹H NMR (360 MHz, CDCl₃): 4.24 (*t*, *J* = 6.7, 2 H); 3.07-2.96 (*m*, 1 H); 1.98-1.85 (*m*, 2 H); 1.85-1.60 (*m*, 5 H); 1.44-1.14 (*m*, 19 H); 0.88 (*t*, *J =* 6.9, 3 H).
¹³C NMR (90.6 MHz, CDCl₃): 197.70 (*s*); 162.22 (*s*); 66.27 (*t*); 46.37 (*d*); 31.90 (*t*); 29.51 (*t*); 29.49 (*t*); 29.30 (*t*); 29.17 (*t*); 28.42 (*t*); 27.48 (*t*); 25.80 (*t*); 25.74 (*t*); 25.32 (*t*); 22.69 (t); 14.11 (*q*).
MS (EI): 296 (M⁺, 2); 185 (1); 158 (1); 156 (1); 112 (7); 111 (88); 110 (3); 85 (2); 84 (7); 83 (100); 81 (1); 79 (1); 71 82); 70 (1); 69 (2); 68 (1); 67 (3); 57 (5); 56 (3); 55 (23); 54 (1); 53 (1); 43 (7); 42 (2); 41 (10); 39 (2); 29 (2); 27 (1).

### m) 4-Methoxybenzyl (cyclohexyl)oxoacetate (16)

The synthesis was carried out as described above under k), using 6.62 g (35.9 mmol) of ethyl (cyclohexyl)oxoacetate, 6.06 g (43.9 mmol) of 4-methoxybenzyl alcohol, 0.5 ml of NaOCH₃ (30% in methanol) and 50 ml of cyclohexane. Column chromatography (SiO₂, heptane/ether 7:3) afforded one fraction of the pure product together with another fraction of lower purity. The latter was rechromatographed (SiO₂, heptane/ether 8:2) to yield a total of 1.15 g (12%) of pure product as a slightly yellow oil.
UV/Vis (hexane): 395 (sh, 5); 375 (sh, 15); 367 (sh, 18); 360 (sh, 21); 352 (sh, 24); 337 (26); 324 (sh, 25); 312 (sh, 24); 288 (sh, 230); 280 (1520); 274 (1790); 268 (sh, 1590); 265 (sh, 1520); 259 (sh, 1170).
IR (neat): 3001*w*, 2929*m*, 2853*m*, 1806*w*, 1721*s*, 1612*m*, 1586*m*, 1514*s*, 1461*m*, 1449*m*, 1424*w*, 1369*w*, 1303*m*, 1271*m*, 1246*s*, 1225*s*, 1174*s*, 1138*s*, 1112*m*, 1080*m*, 1063*s*, 1031*s*, 996*s*, 984*s*, 946*w*, 916*w*, 895*m*, 849*w*, 821*s*, 755*w*, 719*w*.
¹H NMR (360 MHz, CDCl₃): 7.38-7.30 (*m*, 2 H); 6.94-6.85 (*m*, 2 H); 5.21 (*s*, 2 H); 3.81 (*s*, 3 H); 3.08-2.94 (m, 1 H); 1.98-1.83 (*m*, 2 H); 1.83-1.71 (*m*, 2 H); 1.71-1.56 (*m*, 1 H); 1.41-1.10 (*m*, 5 H).
¹³C NMR (90.6 MHz, CDCl₃): 197.39 (*s*); 161.94 (*s*); 160.04 (*s*); 130.51 (*d*); 126.81 (*s*); 114.08 (*d*); 67.58 (*t*); 55.31 (*q*); 46.41 (*d*); 27.46 (*t*); 25.70 (*t*); 25.27 (*t*).
MS (EI): 276 (M⁺, 1); 135 (1); 123 (1); 122 (10); 121 (100); 111 (2); 107 (1); 106 (2); 94 (1); 92 (1); 91 (3); 90 (1); 89 (1); 83 (7); 78 (5); 77 (4); 65 (1); 55 (9); 53 (1); 52 (1); 51 (1); 41 (3); 39 (2). ,

### n) 3-(4-tert-Butylphenyl)-2-methylpropyl cyclohexyl(oxo)acetate (17)

The synthesis was carried out as described above under k), using 4.8 g (26.1 mmol) of ethyl (cyclohexyl)oxoacetate, 4.0 g (21.5 mmol) of 3-(4-*tert*-butylphenyl)-2-methylpropanol (obtained by reduction of (±)-3-(4-*tert*-butylphenyl)-2-methylpropanal (*Lilial®*) with LiAlH₄ in ether), 0.5 ml of NaOCH₃ (30% in methanol) and 40 ml of cyclohexane. Column chromatography (SiO₂, heptane/ether 8:2) afforded 3.43 g (46%) of a colorless oil.
UV/Vis (hexane): 393 (sh, 4); 384 (sh, 7); 375 (sh, 12); 366 (sh, 15); 357 (sh, 18); 351 (sh, 20); 336 (22); 322 (sh, 20); 271 (270); 263 (330); 257 (280); 251 (240); 244 (sh, 240).
IR (neat): 3089*w*, 3055*w*, 3021*w*, 2953*m*, 2928*m*, 2855*m*, 1723*s*, 1512*m*, 1450*m*, 1410*w*, 1387*w*, 1364*w*, 1310*w*, 1270*m*, 1226*m*, 1183*w*, 1139*m*, 1112*w*, 1079*m*, 1064*m*, 998*m*, 963*w*, 954*w*, 919*w*, 892*w*, 843*w*, 800*w*, 718*w*, 674*w*.
¹H NMR (360 MHz, CDCl₃): 7.35-7.27 (*m*, 2 H); 7.12-7.05 (*m*, 2 H); 4.14 (*ABX, J* = 10.7, 5.6, 1 H); 4.07 (*ABX*, *J* = 10.7, 6.7, 1 H); 3.06-2.95 (*m*, 1 H); 2.70 (*ABX, J* = 13.7, 6.5, 1 H); 2.48 (*ABX, J* = 13.7, 7.7, 1 H); 2.28-2.12 (*m*, 1 H); 1.97-1.86 (*m*, 2 H); 1.86-1.74 (*m*, 2 H); 1.74-1.63 (m, 1 H); 1.45-1-15 *(m,* 5 H); 1.31 (*s*, 9 H); 0.98 (*d, J* = 6.7, 3 H).
¹³C NMR (90.6 MHz, CDCl₃): 197.52 (*s*); 162.24 (*s*); 149.01 (*s*); 136.34 (s); 128.75 (*d*); 125.27 (*d*); 70.11 (*t*); 46.44 (*d*); 39.08 (*t*); 34.43 (*d*); 34.38 (*s*); 31.39 (*q*); 27.44. (*t*); 25.71 (*t*); 25.30 (*t*); 16.77 (*q*).
MS (EI): 345 ([M+H]⁺, 1); 344 (M⁺; 6); 330 (1); 329 (6); 234 (9); 233 (52); 231 (4); 217 (2); 190 (1); 189 (10); 188 (27); 178 (2); 177 (13); 175 (2); 174 (7); 173 (31); 161 (1); 160 (1); 159 (5); 148 (6); 147 (45); 146 (1); 145 (8); 133 (3); 132 (23); 131 (29); 130 (1); 129 (2); 128 (2); 127 (1); 119 (4); 118 (3); 117 (19); 116 (3); 115 (5); 112 (3); 111 (40); 110 (1); 105 (5); 104 (2); 103 (1); 91 (9); 84 (7); 83 (100); 81 (1); 79 (1); 77 (1); 67 (1); 65 (1); 57 (14); 55 (20); 54 (1); 53 (1); 41 (9); 39 (2); 29 (2).

### o) (1R,3R,4S)-3-p-Menthanyl (cyclohexyl)oxoacetate (18)

The synthesis was carried out as described above under k), using 25.03 g (136 mmol) of ethyl (cyclohexyl)oxoacetate, 25.70 g (165 mmol) of (-)-menthol and 1 ml of NaOCH₃ (30% in methanol) in 150 ml of cyclohexane. Fractional distillation afforded 23.14 g (58%) of a colorless oil.
B.p. 122°C/33 Pa (0.33 mbar).
UV/Vis (hexane): 394 (sh, 5); 383 (sh, 8); 375 (sh, 12); 366 (sh, 16); 360 (sh, 18); 351 (sh, 20); 337 (22).
IR (neat): 2949*m*, 2928*m*, 2854*m*, 1717*s*, 1450*m*, 1387*w*, 1370*m*, 1332*w*, 1311*w*, 1274*m*, 1230*m*, 1181*w*, 1139*m*, 1111*w*, 1081*m*, 1064*m*, 1037*w*, 1027*w*, 1006*w*, 995*s*, 980*m*, 951*m*, 912*m*, 894*m*, 869*w*, 844*m*, 802*w*, 787*w*, 717*m*.
¹H NMR (360 MHz, CDCl₃): 4.83 (*td*, *J* = 10.9, 4.36, 1 H); 3.05-2.94 (*m*, 1 H); 2.08-1.99 (*m*, 1 H); 1.96-1.62 (*m*, 8 H); 1.59-1.45 (*m*, 2 H); 1.44-0.99 (*m*, 7 H); 0.93 (*d*, *J* = 6.7, 3 H); 0.90 (*d*, *J* = 7.1, 3 H); 0.77 (*d*, *J* = 7.1, 3 H).
¹³C NMR (90.6 MHz, CDCl₃): 198.09 (*s*); 162.16 (*s*); 76.71 (*d*); 46.79 (*d*); 46.32 (*d*); 40.49 (*t*); 34.10 (*t*); 31.50 (*d*); 27.37 (*t*); 26.25 (*d*); 25.76 (*t*);. 25.32 (*t*); 25.26 (*t*); 23.38 (*t*); 21.95 (*q*); 20.67 (*q*); 16.17 (*q*).
MS (EI): 294 (M⁺, 1); 250 (1); 167 (1); 154 (4); 140 (4); 139 (33); 13 8 (8); 137 (1); 123 (2); 112 (1); 111 (9); 110 (1); 109 (1); 98 (1); 97 (16); 96 (1); 95 (5); 84 (7); 83 (100); 82 (2); 81 (12); 80 (1); 79 (2); 71 (3); 70 (1); 69 (19); 68 (1); 67 (5); 57 (13); 56 (2); 55 (33); 54 (2); 53 (2); 43 (5); 42 (1); 41 (11); 39 (2); 29 (2); 27 (1).

### p) 2-Pentyl-1-cyclopentyl (cyclohexyl)oxoacetate (19)

The synthesis was carried out as described above under k), using 6.62 g (36 mmol) of ethyl (cyclohexyl)oxoacetate, 6.80 g (44 mmol) of 2-pentyl cyclopentanol and 1 ml of NaOCH₃ (30% in methanol) in 50 ml of cyclohexane for 24 h. Column chromatography (SiO₂, heptane/ether 8:2) afforded 5.91 g (55%) of a yellow oil (mixture of diastereoisomers). The UV/Vis spectrum indicated. the presence of a colored impurity.
UV/Vis (hexane): 395 (sh, 4); 383 (sh, 7); 374 (sh, 11); 366 (sh, 14); 358 (sh, 16); 349 (sh, 19); 320 (sh, 23); 303 (sh, 34); 289 (sh, 43).
IR (neat): 2924*m*, 2853*m*, 1806*w*, 1719*s*, 1461*w*, 1449*m*, 1376*w*, 1311*w*, 1275*m*, 1254*w*, 1229*m*, 1183*w*, 1139*m*, 1116*w*, 1081*m*, 1064*m*, 1028*w*, 996*m*, 968*w*, 925*w*, 894*w*, 844*w*, 724*w*.
¹H NMR (360 MHz, CDCl₃): 5.35-5.28 (*m*, 1 H); 4.96-4.89 (*m*, 1 H); 3.05-2.88 (*m*, 2 H); 2.10-1.55 (*m*, 10 H); 1.53-1.10 (*m*, 13 H); 0.93-0.80 (*m*, 3 H).
¹³C NMR (90.6 MHz, CDCl₃): 197.99 *(s);* 162.29 (*s*); 162.26 (*s*); 83.72 (*d*); 80.36 (*d*); 46.58 (*d*); 46.42 (*d*); 45.39 (*d*); 44.81 (*d*); 33.49 (*t*); 32.53 (*t*); 32.07 (*t*); 31.94 (*t*); 31.80 (*t*); 30.20 (*t*); 29.61 (*t*); 29.12 (*t*); 28.18 (*t*); 27.60 (*t*); 27.46 (*t*); 27.38 (*t*); 25.32 (*t*); 22.76 (*t*); 22.59 (*t*); 22.03 (*t*); 14.05. (*q*).
MS (EI): 167 (1); 140 (1); 139 (8); 138 (7); 123 (1); 112 (1); 111 (11); 110 (1); 109. (1); 98 (2); 97 (25); 96 (2); 95 (3); 84 (7); 83 (100); 82 (5); 81 (4); 79 (2); 71 (4); 70 (2); 69 (22); 68 (2); 67 (9); 66 (1); 65 (1); 57 (11); 56 (2); 55 (29); 54 (3); 53 (2); 43 (4); 42 (1); 41 (12); 39 (3); 29 (3); 27 (1).

### q) 4-(1,1-Dimethylpropyl)-1-cyclohexyl(cyclohexyl)oxoacetate (20)

The synthesis was carried out as described above under k), using 6.62 g (36 mmol) of ethyl (cyclohexyl)oxoacetate, 7.40 g (43.5 mmol) of 4-(1,1-dimethylpropyl)-1-cyclohexanol and 1 ml of NaOCH₃ (30% in methanol) in 50 ml of cyclohexane. Column chromatography (SiO₂, heptane/ether 8:2) afforded 4.78 g (43%) of a slightly yellow oil as a mixture of *cis*/*trans* isomers (≈38:62).
UV/Vis (hexane): 394 (sh, 4); 385 (sh, 7); 375 (sh, 12); 367 (sh, 15); 339 (sh, 35); 326 (40); 312 (sh, 38); 297 (sh, 34); 283 (33); 272 (sh, 36).
IR (neat): 2929*s*; 2855*m*, 1800*w*, 1719*s*, 1462*w*, 1448*m*, 1387*w*, 1377*w*, 1364*w*, 1323*w*, 1309*w*, 1274*m*, 1254*w*, 1228*m*, 1182*w*, 1160*w*, 1140*m*, 1108*w*, 1081*m*, 1064*m*, 1047*w*, 1005*w*, 995*s*, 948*w*, 928*w*, 906*w*, 894*w*, 875*w*, 830*w*, 805*w*, 780*w*, 745*w*, 719*w*.
¹H NMR (360 MHz, CDCl₃): 5.21-5.14 (*m*, 1 H (*cis*)); 4.85-4.72 (*tt*, *J* = 11.3, 4.6, 1 H (*trans*)); 3.07-2.91 (*m,* 1 H); 2.17-1.04 (*m*, 21 H); 0.83-0.77 (*m*, 9 H).
¹³C NMR (90.6 MHz, CDCl₃): 198.07 (*s*); 161.85 (*s*); 76.16 (*d*); 72.28 (*d*); 46.81 (*d*); 46.35 (*d*); 44.58 (*d*); 44.21 (*d*); 34.82 (*s*); 34.60 (*s*); 32.75 (*t*); 32.49 (*t*); 31.90 (*t*); 30.49 (*t*); 27.47 (*t*); 25.75 (*t*); 25.38 (*t*); 25.31 (*t*); 24.97 (*t*); 24.27 (*q*); 24.17 (*q*); 21.22 (*t*); 8.10 (*q*).
MS (EI): 264 (1); 193 (1); 181 (1); 153 (4); 152 (3); 137 (4); 124 (1); 6); 112 (1); 111 (14); 110 (2); 109 (1); 98 (4); 97 (55); 95 (5); 85 (2); 84 (4); 83 (60); 81 (12); 80 (1); 79 (2); 72 (6); 71 (100); 69 (13); 68 (1); 67 (11); 57 (15); 56 (3); 55 (51); 54 (4); 53 (3); 43 (32); 41 (22); 39 (4); 29 (7); 27 (4).

### r) 1-(2-Naphthalenyl)ethyl (cyclohexyl)oxoacetate (21)

The synthesis was carried out as described above under k), using 6.62 g (24 mmol) of ethyl (cyclohexyl)oxoacetate, 7.5 g (29 mmol) of 1-(2-naphthalenyl)ethanol and 1 ml of NaOCH₃ (30% in methanol) in 70 ml of cyclohexane for 28 h. Column chromatography (SiO₂, heptane/ether 8:2) afforded 2.67 g of a colorless oil still containing about 30% of ethyl (cyclohexyl)oxoacetate.
¹H NMR (360 MHz, CDCl₃): 7.88-7.78 (*m*, 4 H); 7.54-7.44 (*m*, 3 H); 6.16 (*q, J =* 6.6, 1 H); 3.08-2.93 (*m*, 1 H); 1.97-1.60 (*m*, 5 H); 1.72 (*d, J*= 6.7, 3 H); 1.44-1.12 (*m*, 5 H).
¹³C NMR (90.6 MHz, CDCl₃): 197.53 (*s*); 161.49 (*s*); 137.73 (*s*); 133.21 (*s*); 133.13 (*s*); 128.60 (*d*); 128.09 (*d*); 127.71 (*d*); 126.40 (*d*); 126.34 (*d*); 125.38 (*d*); 123.85 (*d*); 74.76 (*d*); 46.41 (*d*); 27.38 (*t*); 25.70 (*t*); 25.26 (*t*); 22.08 (*q*).
MS (EI): 310 (M⁺, 1); 157 (2); 156 (14); 155 (100); 154 (22); 153 (16); 152 (8); 151 (2); 141 (2); 139 (1); 129 (3); 128 (9); 127 (9); 126 (2); 115 (4); 111 (3); 101 (1); 84 (1); 83 (17); 77 (4); 76 (4); 75 (2); 64 (1); 63 (2); 56 (1); 55 (16); 51 (2); 50 (1); 43 (2); 41 (9); 39 (4); 29 (3); 27 (3).

### s) 3,7-Dimethyl-6-octenyl (cyclopentyl)oxoacetate (22)

In the first step, ethyl (cyclopentyl)oxoacetate was prepared as follows.

A Grignard reagent prepared from 64.0 g of freshly distilled bromocyclopentane (0.43 mol) and 11.0 g of magnesium (0.45 mol) in 360 ml of dry ether and filtered under N₂ was added dropwise to a stirred solution of 48.2 g (0.33 mol) of diethyl oxalate in 300 ml of dry ether at -40°C. The mixture was slowly warmed to 0°C and poured onto a sat. solution of NH₄Cl, extracted with ether and washed with water (pH ≈ 7). The organic phase was dried over Na₂SO₄ and concentrated. Fractional distillation gave 27.1 g (48%) of a colorless oil in sufficient purity for further derivatization. Column chromatography (SiO₂, heptane/ether 8:2) of 2.50 g afforded 2.04 g of product at high purity.
B.p. 42°C/10 Pa (0.1 mbar).
UV/Vis (hexane): 389 (sh, 3); 371 (sh, 9); 359 (sh, 13); 345 (sh, 15); 336 (15).
IR (neat): 3483*w*, 2956*m*, 2869*m*, 1723*s*, 1684*m*, 1469*w*, 1449*m*, 1399*w*, 1372*w*, 1318*w*, 1296*m*, 1254*s*, 1194*m*, 1159*m*, 1140*m*, 1091*s*, 1043*s*, 1029*s*, 952*m*, 906*m*, 858*m*, 780*m*, 708*w*.
¹H NMR (360 MHz, CDCl₃): 4.32 (*q*, *J =* 7.1, 2 H); 3.56-3.44 (*m*, 1 H); 1.98-1.75 (*m*, 4 H); 1.75-1.57 (*m*, 4 H); 1.37 (*t*, *J =* 7.1, 3 H).
¹³C NMR (90.6 MHz, CDCl₃): 196.73 (*s*); 161.98 (*s*); 62.24 (*t*); 47.42 (*d*); 28.32 (*t*); 26.05 (*t*); 14.05 (*q*).
MS (EI): 170 (M⁺, 5); 114 (1); 101 (1); 98 (4); 97 (48); 96 (4); 95 (1); 70 (6); 69 (100); 68 (3); 67 (6); 66 (1); 65 (1); 55 (4); 54 (1); 53 (2); 51 (1); 43 (1); 42 (2); 41 (22); 40 (2); 39 (7); 29 (5); 28 (1); 27 (4).

### 3,7-Dimethyl-6-octenyl (cyclopentyl)oxoacetate (22)

The synthesis was carried out as described above under k), using 6.07 g (35.6 mmol) of the product obtained above, 6.80 g (43.6 mmol) of citronellol and 0.5 ml of NaOCH₃ (30% in methanol) in 50 ml of cyclohexane. Column chromatography (SiO₂, heptane/ether 7:3) afforded 5.28 g (53%) of a yellow oil.
UV/Vis (hexane): 389 (sh, 4); 366 (sh, 12); 345 (sh, 17); 336 (17).
IR (neat): 3493*w*, 2957*m*, 2916*m*, 2869*m*, 1798*w*, 1724*s*, 1687*m*, 1451*m*, 1377*m*, 1354*w*, 1259*m*, 1190*m*, 1164*m*, 1144*m*, 1091*m*, 1047*m*, 1027*m*, 984*w*, 945*m*, 829*m*, 782*w*, 739*w*, 717*w*.
¹H NMR (360 MHz, CDCl₃): 5.13-5.03 (*m*, 1 H); 4.40-4.20 (*m*, 2 H); 3.54-3.42 (*m*, 1 H); 2.10-1.71 (*m*, 7 H); 1.71-1.45 (*m*, 6 H); 1.68 (*s*, 3 H); 1.60 (*s*, 3 H); 1.43-1.30 (*m*, 1 H); 1.29-1.13 (*m*, 1 H); 0.94 (*d*, *J* = 6.3, 3 H).
¹³C NMR (90.6 MHz, CDCl₃): 196.66 (*s*); 162.11 (*s*); 131.51 (*s*); 124.40 (*d*); 64.75 (*t*); 47.48 (*d*); 36.90 (*t*); 35.22 (*t*); 29.40 (*d*); 28.27 (*t*); 26.05 (*t*); 25.71 (*q*); 25.35 (*t*); 19.35 (*q*); 17.66 (*q*).
MS (EI): 280 (M⁺, 1); 262 (2); 252 (1); 184 (1); 183 (6); 165 (1); 155 (3); 144 (2); 142 (1); 139 (2); 138 (20); 137 (6); 126 (1); 125 (1); 124 (2); 123 (22); 121 (1); 111 (1); 110 (2); 109 (9); 98 (3); 97 (39); 96 (7); 95 (21); 94 (2); 83 (6); 82 (15); 81 (23); 80 (2); 79 (1); 70 (7); 69 (100); 68 (5); 67 (9); 65 (1); 57 (2); 56 (2); 55 (10); 54 (1); 53 (3); 43 (2); 42 (2); 41 (25); 40 (1); 39 (4); 29 (2); 27 (2).

### t) (E)-3,7-Dimethyl-2,6-octadienyl 3-methyl-2-oxopentanoate (23)

The synthesis was caried out as described above under a), using 4.85 g (38 mmol) of 3-methyl-2-oxo pentanoic acid and 11.5 g (75 mmol) of geraniol in 130 ml of toluene for 24 h. Column chromatography (SiO₂, heptane/EtOAc 95:5) afforded 7.68 g of crude product, which was fractionally distilled to give 4.04 g (40%) of a colorless oil.
B.p. 82°C/20 Pa (0.2 mbar).
UV/Vis (hexane): 393 (sh, 5); 382 (sh, 9); 374 (sh, 13); 364 (sh, 17); 357 (sh, 19); 350 (sh, 21); 335 (23).
IR (neat): 2966*m*, 2929*m*, 2878*m*, 1746*m*, 1723*s*, 1670*w*, 1454*m*, 1377*m*, 1338*w*, 1274*m*, 1244*m*, 1163*m*, 1107*w*, 1085*w*, 1039*s*, 999*m*, 959*m*, 913*m*, 827*w*, 796*w*, 772*w*, 742*w*, 705*w*.
¹H NMR (360 MHz, CDCl₃): 5.46-5.35 (*m*, 1 H); 5.14-5.04 (*m*, 2 H); 4.77 (*d*, *J* = 7.1, 2 H); 3.20-3.07 (*m*, 1 H); 2.20-2.00 (*m*, 4 H); 1.83-1.66 (*m*, 1 H); 1.74 (*s*, 3 H); 1.68 (*s*, 3 H); 1.60 (*s*, 3 H); 1.52-1.36 (*m*, 1 H); 1.13 (*d*, *J* = 7.1, 3 H); 0.92 (*t*, *J* = 7.5, 3 H).
¹³C NMR (90.6 MHz, CDCl₃): 198.29 (*s*); 162.10 (*s*); 144.01 (*s*); 131.97 (*s*); 123.58 (*d*); 117.13 (*d*); 62.94 (*t*); 43.66 (*d*); 39.53 (*t*); 26.22 (*t*); 25.66 (*q*); 24.92 (*t*); 17.69 (*q*); 16.57 (*q*); 14.46 (*q*); 11.35 (*q*).
MS (EI): 266 (M⁺, 1); 181 (1); 179 (1); 153 (1); 138 (3); 137 (28); 13.6 (6); 135 (5); 123 (1); 122 (1); 121 (2); 109 (1); 107 82); 96 (2); 95 (10); 94 (2); 93 (6); 92 (2); 91 (3); 85 (9); 83 (1); 82 (4); 81 (52); 80 (2); 79 (3); 78 (1); 77 (3); 71 (1); 70 (6); 69 (100); 68 (12); 67 (12); 66 (1); 65 (2); 58 (2); 57 (30); 56 (1); 55 (5); 54 (1); 53 (6); 51 (1); 43 (1); 42 (2); 41 (26); 40 (2); 39 (5) 29 (5); 28 (1); 27 (2).

### u) 3,7-Dimethyl-6-octenyl (bicyclo[2.2.1]hept-2-yl)oxoacetate (24)

A Grignard reagent prepared from 4.00 g of 2-norbornyl bromide (23 mmol) and 0.59 g of magnesium (24 mmol) in 30 ml THF was, after filtration under N₂, added dropwise (during 45 min) to a stirred solution of 3.00 g (8 mmol) of bis(3,7-dimethyl-6-octenyl) oxalate in 40 ml of THF at -40°C. The mixture was slowly warmed to 0°C, quenched with 30 ml of a sat. solution of NH₄Cl. The reaction mixture was extracted with diethyl ether and water (2x) and the organic phase dried over Na₂SO₄. Repetitive column chromatography (SiO₂, heptane/ether 9:1 and heptane/ether 95:5) followed by MPLC on a *Lobar* column (SiO₂ *Merck*, heptane/ether 85:15) finally afforded 0.188 g (3%) of the pure product as a colorless oil.
¹H NMR (360 MHz, CDCl₃): 5.13-5.04 (*m*, 1 H); 4.37-4.22 (*m*, 2 H); 3.06 (*m*, 1 H); 2.59-2.48 (*m*, 1 H); 2.36-2.27 (*m*, 1 H); 2.09-1.84 (*m*, 3 H); 1.84-1.69 (*m*, 1 H); 1.68 (*s*, 3 H); 1.66-1.45 (*m*, 4 H); 1.60 (*s*, 3 H); 1.45-1.30 (*m*, 3 H); 1.30-1.08 (*m*, 4 H); 0.94 (*d*, *J* = 6.3, 3 H).
¹³C NMR (90.6 MHz, CDCl₃): 195.33 (*s*); 162.08 (*s*); 131.50 (*s*); 124.39 (*d*); 64.75 (*t*); 50.37 (*d*); 39.82 (*d*); 36.91 (*t*); 36.28 (*d*); 35.84 (t); 35.23 (*t*); 31.86 (*t*); 29.64 (*t*); 29.43 (*d*); 28.78 (*t*); 25.71 (*q*); 25.36 (*t*); 19.34 (*q*); 17.66 (*q*).
MS (EI): 288 (1); 183 (4); 168 (1); 155 (1); 139 (2); 138 (15); 137 (2); 124 (3); 123 (30); 122 (2); 121 (1); 110 (1); 109 (5); 97 (1); 96 (11); 95 (100); 93 (4); 91 (1); 83 (4); 82 (19); 81 (21); 80 (5); 79 (3); 77 (2); 70 (2); 69 (23); 68 (5); 67 (22); 66 (3); 65 (3); 57 (3); 56 (3); 55 (15); 54 (2); 53 (5); 43 (4); 42 (3); 41 (33); 39 (6); 29 (5); 28 (1); 27 (5).

### Example 3

### Release of geraniol from solutions of geranyl 2-benzoyl benzoate

Geranyl 2-benzoyl benzoate was dissolved in a concentration of 3.68g/l in the solvents indicated in Table 1. The samples were then irradiated using a Fadeometer and under the conditions indicated in Table 1, and the amount of released geraniol was measured. The values indicated are the average of duplicate samples.

**Table 1 :**

| Release of geraniol from geranyl 2-benzoyl benzoate in solution upon irradiation with a Fadeometer | | | |
|---|---|---|---|
| Run | Solvent | Irradiation intensity (KJ/m²) | % of geraniol released* |
| 1 | Isopropanol/benzene 1:1 | 33.7 | 24.4 |
| 2 | Isopropanol/benzene 1:1 | 3.4 | 30.4 |
| 3 | Isopropanol/benzene 1:1 | 0** | 0 |
| 4 | Dodecanol/benzene 1:1 | 33.7 | 26.8 |
| 5 | Isopropanol/acetonitrile | 3.4 | 22.6 |
| 6 | Isopropanol/ acetonitrile | 0 ** | 0 |

| | | | |
|---|---|---|---|
| * calculated as weight % of theoretically possible geraniol release | | | |
| ** indicates a control run in which the flask was wrapped with aluminum foil before irradiation | | | |

The following Table 2 indicates the amount of geraniol released from the same ester, but upon exposure to sunlight (New Jersey, USA, typical sunny day of June).

**Table 2 :**

| Release of geraniol from geranyl 2-benzoyl benzoate in solution upon exposure to sunlight | | | |
|---|---|---|---|
| Run | Solvent | Hours of sun exposure | % of geraniol released * |
| 1 | Isopropanol/benzene 1:1 | 5 | 71.3 |
| 2 | Isopropanol/benzene 1:1 | 0** | 0 |

| | | | |
|---|---|---|---|
| * calculated as weight % of theoretically possible geraniol release | | | |
| ** indicates a control run in which the flask was wrapped with aluminum foil before irradiation | | | |

The above results show that it is possible to release geraniol in solution upon exposure to a Fadeometer or to sunlight, while no release occurs when the sample is not exposed to radiation.

### Example 4

### Release of geraniol from geranyl 2-(2'-isopropylbenzoyl)benzoate (solution and film)

Geranyl 2-(2-isopropylbenzoyl)benzoate was dissolved in a concentration of 4.05g/l in benzene and subsequently irradiated, or was deposited as a thin film, by evaporation of the solvent, on the walls of the flask before irradiation. After irradiation, the amount of released geraniol was measured. The results are shown in Table 3. The values indicated are the average of duplicate samples.

**Table 3 :**

| Release of geraniol from geranyl 2-(2'-isopropylbenzoyl)benzoate in solution and as a film upon irradiation with a Fadeometer | | | |
|---|---|---|---|
| Run | Solvent/film | Irradiation intensity (KJ/m²) | % of geraniol released * |
| I | Benzene | 3.4 | 11.2 |
| 2 | Benzene | 0** | 0 |
| 3 | Film (33.5mg) | 3.4 | 9.5 |
| 4 | Film (32.5mg) | 0** | 0 |

| | | | |
|---|---|---|---|
| * calculated as weight % of theoretically possible geraniol release | | | |
| ** indicates a control run in which the flask was wrapped with aluminum foil before irradiation | | | |

Table 4 indicates the results of analogous experiments in which the solutions and films of geranyl 2-(2'-isopropylbenzoyl)benzoate were exposed to sunlight (New Jersey, USA, typical sunny day of June). The values indicated are the average of duplicate samples.

**Table 4 :**

| Release of geraniol from geranyl 2-(2'-isopropylbenzoyl)benzoate (solution and film) upon exposure to sunlight | | | |
|---|---|---|---|
| Run | Solvent/film | Hours of sun exposure | % of geraniol released* |
| 1 | Isopropanol/benzene 1:1 | 5 | 71.3 |
| 2 | Isopropanol/benzene 1:1 | 0** | 0 |
| 3 | Film (14.2mg) | 5 | 27.0 |
| 4 | Film (14.2mg) | 0** | 0 |

| | | | |
|---|---|---|---|
| * calculated as weight % of theoretically possible geraniol release | | | |
| ** indicates a control run in which the flask was wrapped with aluminum foil before irradiation | | | |

The above results show that the introduction of an isopropyl substituent into the geranyl ester allows the release of geraniol from solution and from a solid film, upon exposure to a Fadeometer radiation and to natural sunlight.

### Example 5

### Release of geraniol from geranyl 2-(2',4'-diisopropylbenzoyl)benzoate (solution and film)

Geranyl 2-(2',4'-diisopropylbenzoyl)benzoate was dissolved in benzene in a concentration of 4.48 g/l in benzene and subsequently irradiated, using a Fadeometer. The samples were irradiated with 31.1 KJ/m², and 50 weight% of the theoretical value of geraniol was released.
Similar experiments were conducted in which benzene solutions with the same content in geranyl 2-(2',4'-diisopropylbenzoyl)benzoate and films which were obtained by evaporation of the solvent, were exposed to daylight outdoors (New Jersey, USA, cloudy day in August). Table 5 shows the results of the experiments: The values indicated are the average of duplicate samples.

**Table 5 :**

| Release of geraniol from geranyl 2-(2',4'-diisopropylbenzoyl)benzoate in solution and as film, upon exposure to sunlight | | | |
|---|---|---|---|
| Run | Solvent/film | Hours of sun exposure | % of geraniol released* |
| 1 | Benzene | 6 | 13 |
| 2 | Film | 6 | 18 |

| | | | |
|---|---|---|---|
| * calculated as weight % of theoretically possible geraniol release | | | |

### Example 6

### Release of geraniol from geranyl 2-(2',4'-diisopropylbenzoyl)benzoate in an all-purpose cleaner

An all-purpose cleaner of the Fabuloso ® (registered trademark of Colgate-Palmolive, USA) type containing 0.3% of geranyl 2-(2',4'-diisopropylbenzoyl)benzoate was prepared. The all-purpose cleaner solution thus obtained was added to borosilicate flasks which were then irradiated for 3 hours in outdoor sunlight. The resulting solutions were then compared to the unperfumed all-purpose cleaner base, on a triangular blind test by a panel composed of 15 non-experts. The odd sample was the one containing the above precursor molecule.
The evaluation was carried out by sniffing on the flask.
From the 15 test persons, 14 correctly distinguished the perfumed sample from the unperfumed sample. They found that the odor note of the irradiated sample was floral, geraniol, citrus or citronellal, whereas the non-irradiated sample was found to be neutral, odorless or slightly oily.
When the odd sample was the one containing the unperfumed cleaner base, 10 of 15 panelists correctly distinguished the samples.
The release of geraniol from the 2-benzoyl benzoate used in the present embodiment and from the other benzoates synthesized occurrred in all types of all-purpose cleaners and is therefore not restricted to one type of these.

### Example 7

### Release of Polysantol ® from (E)-3,3-dimethyl-5-(2',2',3'-trimethyl-3'-cyclopenten-1-yl)-4-penten-2-yl 2-(2',4'-diisopropylbenzoyl)benzoate

The above-identified compound was dissolved in toluene in a concentration of 2.35 g/l and irradiated for 6 hours with a UV lamp. The amount of released Polysantol was measured by GC, and it was found that 35% of the theoretical amount of Polysantol had been released.

### Example 8

### Release of geraniol from a film of 2-(2',4'-diisopropylbenzoyl)benzoate deposed on tiles

0.8 G of an all-purpose cleaner of the Fabuloso ® type containing 0.3% of the title compound were evenly deposed on tiles of the size 10x10 cm. The liquid was allowed to evaporate, and the tiles were exposed to sunlight for 7 h in a covered petri dish. The tiles were then olfactively compared to tiles treated in the same way with the unperfumed cleaner base and exposed to sunlight on the same day and the same hours, on a triangular blind test by a panel composed of 15 non-experts, by sniffing on the petri dish. When the odd sample was the one containing the title compound, 14 of 15 panelists correctly distinguished the perfumed sample from the unperfumed sample. When the odd sample was the one containing the unperfumed base, the correct attribution was made by 9 of 15 panelists.

### Example 9

### Release of fragrant aldehydes and ketones from various citronellyl α-keto esters in solution or in the neat state

0.01 M solutions (5 ml) of the α-keto esters prepared as described in example 2, in toluene, acetonitrile or isopropanol, were prepared and irradiated with a xenon or a UV lamp or exposed to outdoor sunlight in 10 ml volumetric flasks. Samples in the neat state were also irradiated under the same conditions. Before irradiation in solution, 1 ml of a 0.01 M solution of decanol was added which served as internal standard for GC analysis. The results are found in the Table 6 below. Table 6 indicates the amount of released aldehyde or ketone in mol%, the amount of remaining starting material is indicated in brackets. It was also observed that olefins were released, from compounds **(11)** and **(12)** of example 2, together with release of citronellal.

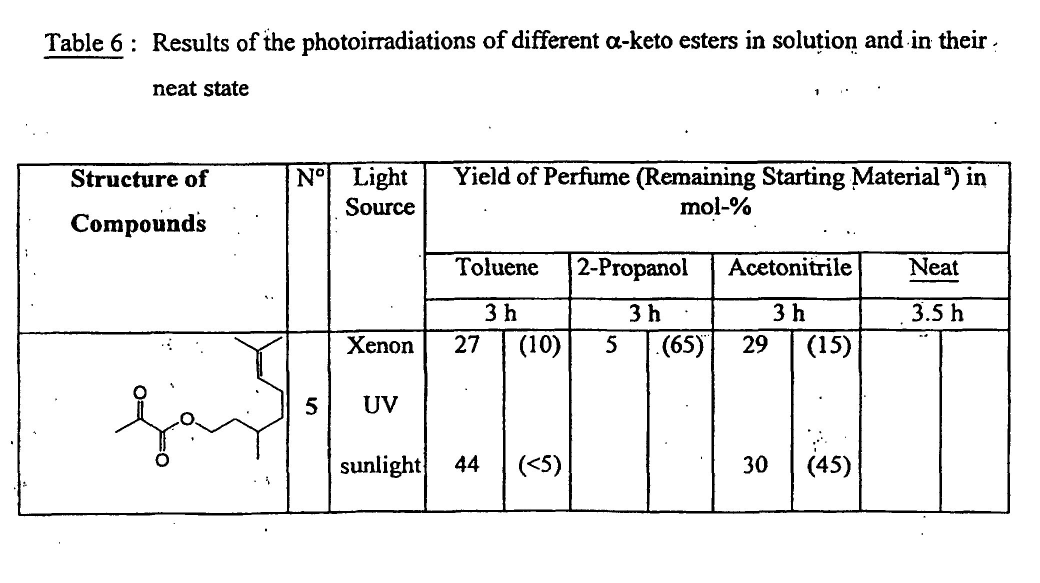

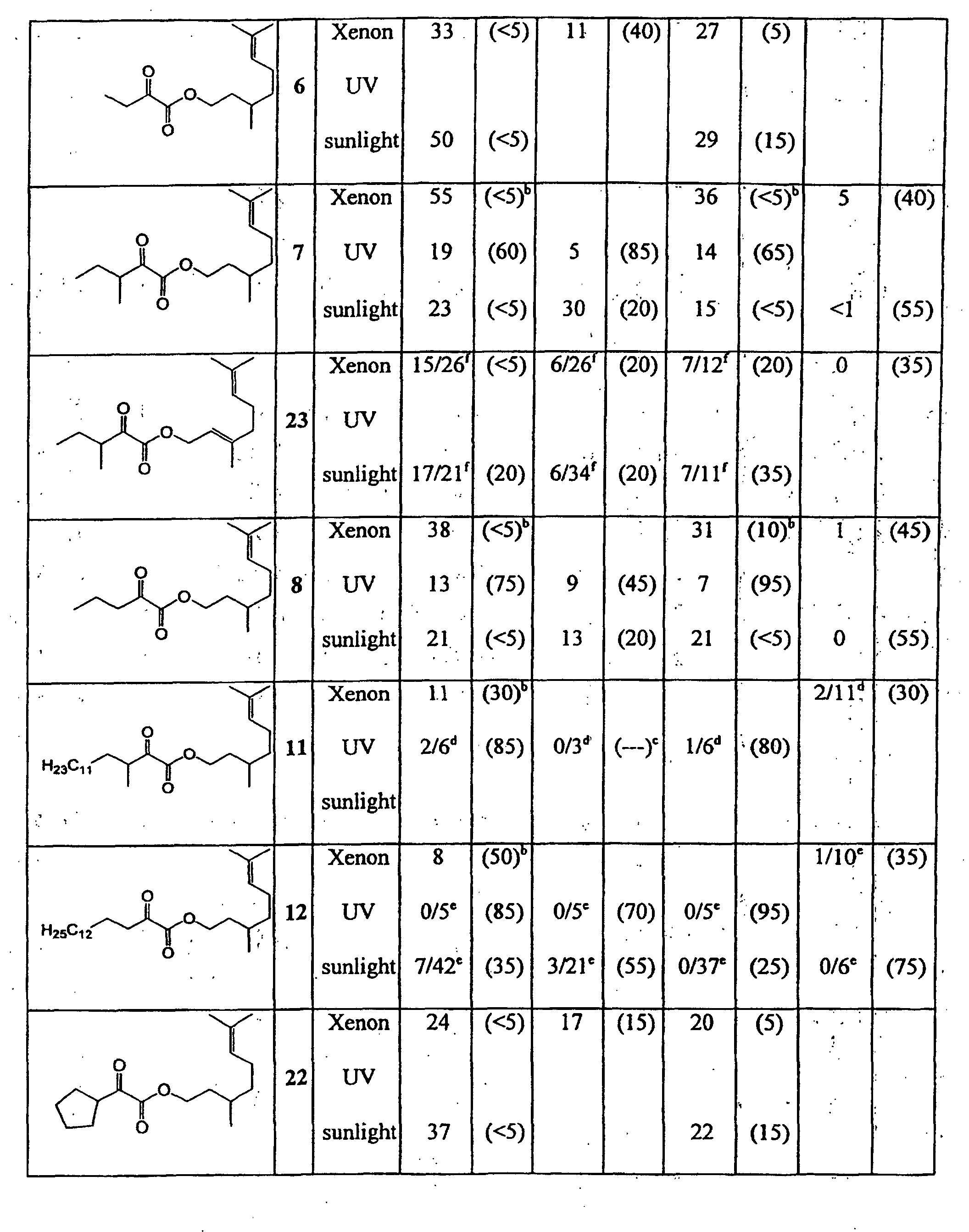

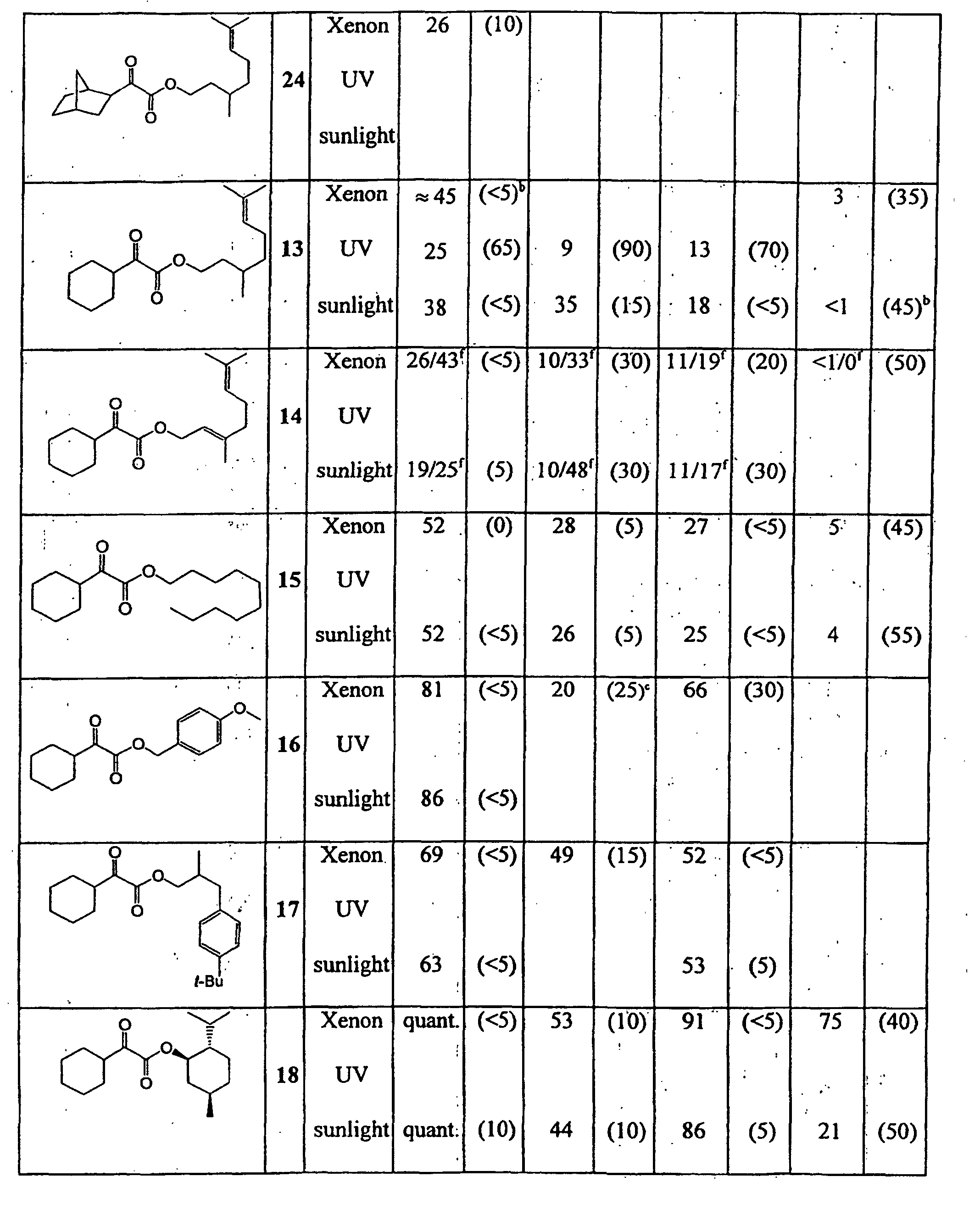

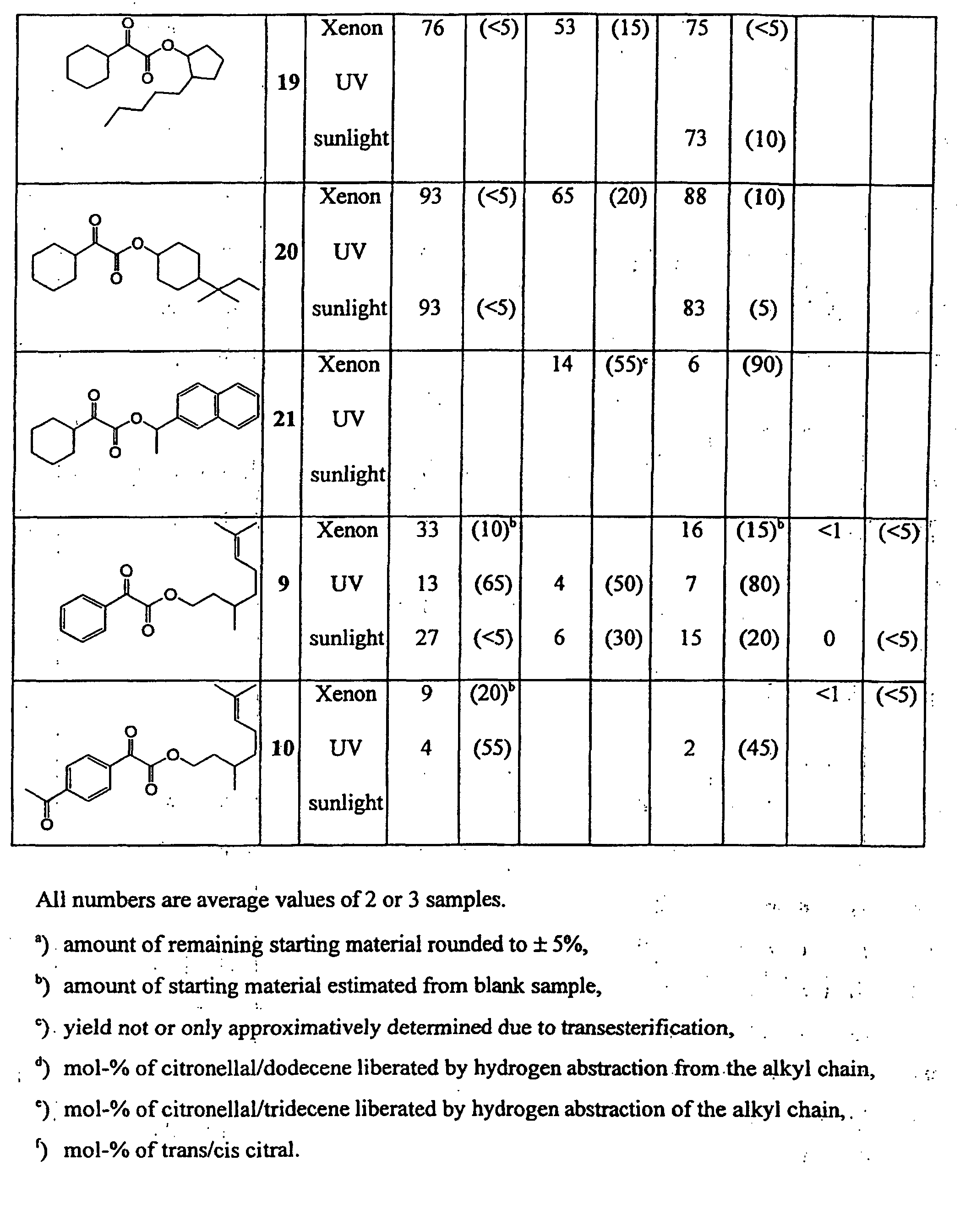

### Example 10

### Release of citronellal from various citronellyl α-keto esters in after-shave lotions

Compounds (7) and (8) of example 2 were each dissolved in an amount of 0.29 g in 19.54 g of a standard after-shave lotion base, under addition of a standard solubilizer (Cremophor RH40, BASF AG). For each of the compounds, three samples of 6 ml (one of which was wrapped in aluminium foil to serve as reference) were irradiated in 10 ml volumetric flasks for 3h with a xenon lamp. The irradiated samples were analyzed by HPLC using citronellal and the corresponding starting materials as external standards. The reference experiment (aluminium foil wrapped) showed no release of citronellal. The results obtained with the other samples are summarized in Table 7.

**Table 7 :**

| Results of the photoirradiations of α-keto esters in after-shave lotion | | |
|---|---|---|
| Compound N° | mol-% of citronellal liberated | mol-% of remaining * starting material |
| 7 | 12 | 36 |
| 8 | 2 | 53 |

| | | |
|---|---|---|
| * average of 2 samples | | |

### Example 11

### Release of citronellal or menthone from various citronellyl α-keto esters in a window cleaner and in an all-purpose cleaner

10-15 mg of the respective α-keto ester as specified in Table 8 below were weighed into 10 ml volumetric flasks. A solubilizer was added (Cremophor RH40, BASF AG for window cleaner, Triton X 100 (Rohm & Haas) for all-purpose cleaner), before adding 6 ml of the respective base, i.e. a standard type window cleaner, or a Fabuloso® (registered trademark of Colgate-Palmolive, USA) type all-purpose cleaner, and agitating until the solution became clear. For each irradiation series four samples were prepared for each compound, one of which, wrapped in aluminium foil, served as reference. All the samples were irradiated for 3, 6, or 15 h with either the Xenon or the UV lamp or exposed to outdoor sunlight. In all cases the formation of citronellal or menthone could be smelled after the photolysis. In order to quantify the amount of aldehyde or ketone (and of the remaining starting material) in the application base, the irradiated samples were subjected to GC analysis (extraction and on-column injection). For analysis, 1 g of NaCl was added and the samples were extracted with 3 ml of a 0.35 mM (50 mg/l) solution of undecane (used as internal standard) in *iso*-octane. The aqueous layer was re-extracted with 2 ml of the *iso*-octane solution and the two organic phases were combined and injected directly onto a GC column. The results obtained for the different bases are summarized in Table 8.

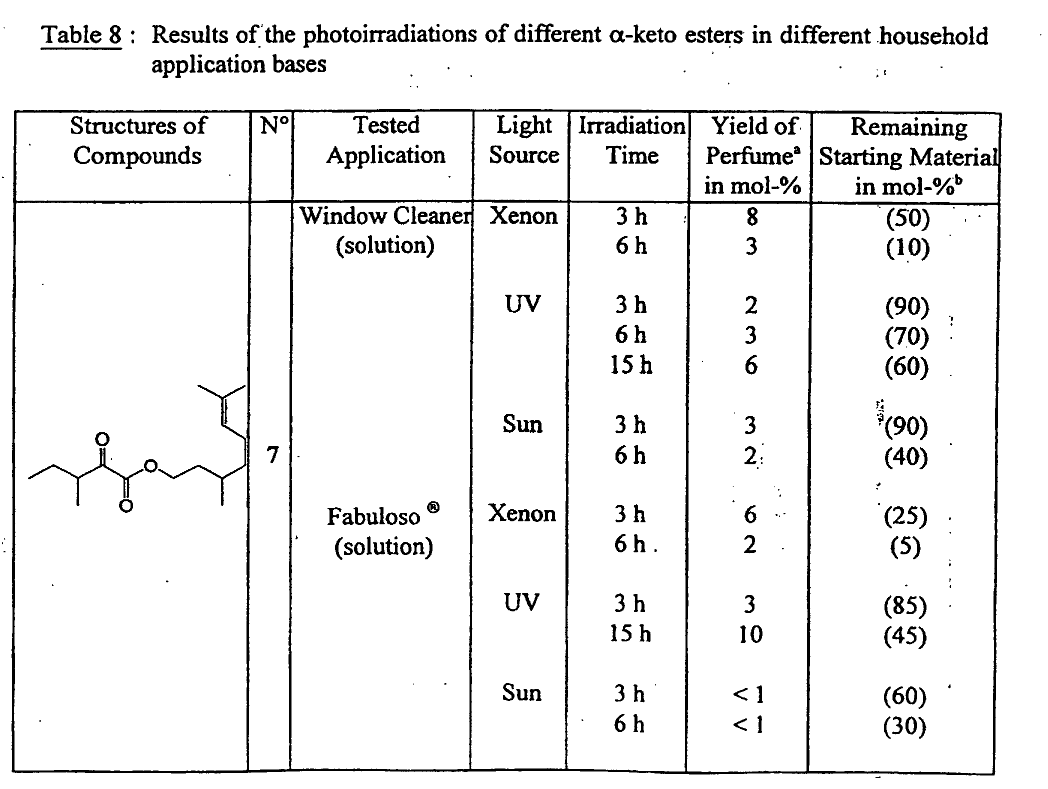

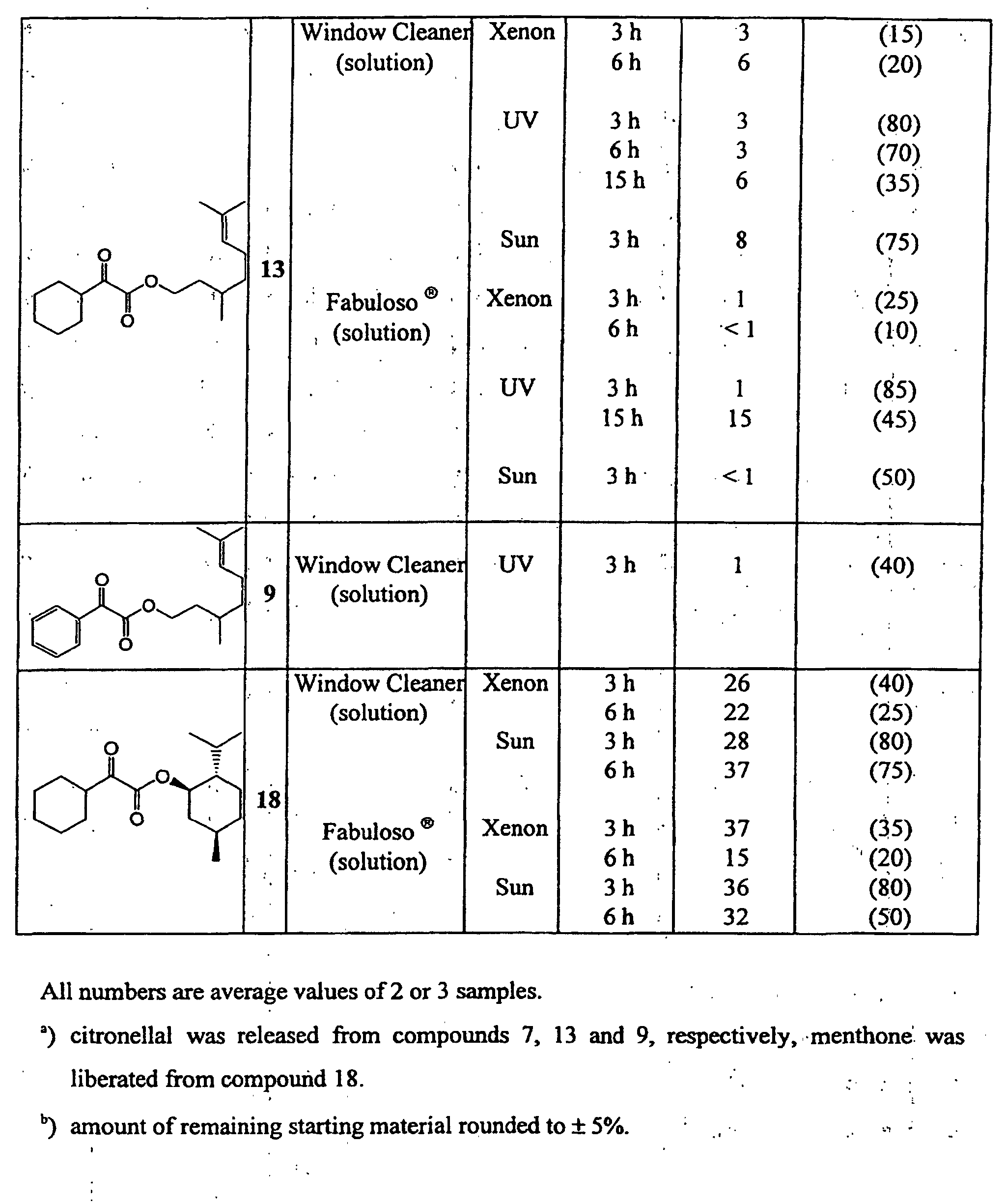

### Example 12

### Dynamic headspace analysis in all purpose cleaners (APC)

In order to follow the perfume release under more realistic application conditions, quantitative dynamic headspace analyses were carried out. The formation of citronellal from its precursor in an APC application was compared to the behaviour of free citronellal in the same base. Solutions of a base of the Fabuloso ® type containing either 0.3 mass-% of citronellal precursor **13** or 0.3 mass-% of pure citronellal (≈ 2 molar equivalents) were prepared and deposed in self-built 3.5 l *Pyrex ®* glass containers covered with a thin window glass plate. The chambers were exposed to outdoor sunlight for 6 h and continuously flushed with an air stream. Every hour the volatiles contained in the air stream were adsorbed on a *Tenax* cartridge (during 15 min) and the light intensity was measured. The amount of citronellal trapped on the cartridges was desorbed and quantified by GC analysis and are summarized in Table 9.

The amount of citronellal released increases with increasing light intensity and decreases when the intensity decreases, with the maximum of release being obtained shortly after the maximum of irradiation was measured. The amount of free citronellal, however, was found to decrease steadily with increasing time and, no dependency on the light intensity was observed.

**Table 9 :**

| Comparison of the dynamic headspace of free citronellal and citronellal released from precursor **13** in a Fabuloso ® type APC irradiated with outdoor sunlight. | | | |
|---|---|---|---|
| Time [h] | Free citronellal in base (0.3 mass-%) [ng l⁻¹] | Citronellal released from precursor **13** in base (0.3 mass-%) [ng l⁻¹] | Sunlight intensity [lux] |
| 1 | 154086 | 1579 | 38500 |
| 2 | 117735 | 4752 | 53500 |
| 3 | 67015 | 7475 | 64500 |
| 4 | 50632 | 7829 | 63000 |
| 5 | 33215 | 7297 | 52500 |
| 6 | 19757 | 5919 | 35000 |

The above described experiment was repeated using 0.3 mass-% of menthone precursor **18** or 0.15 mass-% of pure menthone (≈ 1 molar equivalent) in an APC application of the Fabuloso ® type. Again a dependency of perfume release of the irradiation intensity could be observed, see Table 10, whereas the amount of unprotected menthane decreased continuously over time. Working with molar equivalents instead of mass equivalents shows that the perfume concentration of both systems are in the same order of magnitude. At the beginning of the experiment the concentration of unprotected menthone is about three times stronger than the concentration of the perfume released from the precursor. At the end of the experiment the perfume released from the keto ester contributes more strongly than the free menthone.

**Table 10 :**

| Comparison of the dynamic headspace of free menthone and menthone released from precursor **18** in a Fabuloso ® type APC irradiated with outdoor sunlight. | | | |
|---|---|---|---|
| Time [h] | Free menthone in base (0.15 mass-%) [µg l⁻¹] | Menthone released from precursor **18** in base (0.3 mass-%) [µg l⁻¹] | Sunlight intensity [lux] |
| 0.5 | 94.6 | 33.1 | 53000 |
| 1.5 | 86.4 | 59.7 | 71000 |
| 2.5 | 81.5 | 70.0 | 86750 |
| 3.5 | 76.7 | 68.9 | 88500 |
| 4.5 | 64.2 | 63.3 | 80500 |
| 5.5 | 47.4 | 60.5 | 69250 |
| 6.5 | 39.1 | 48.1 | 53000 |

### Example 13

### Dynamic headspace analysis for the slow release on hair

In order to test the performance of the controlled photochemical release of perfumes in typical body care applications, 0.2 mass-% of precursor **13** dissolved in a leave-in hair conditioner of the standard type was sprayed four times on a hair curl (≈ 5 g weight) and irradiated in a glass tube for 3 h with a Xenon lamp. The hair curl had been washed beforehand with an unperfumed shampoo base and the amount of conditioner deposed on the hair was weighed precisely. A comparison experiment with 0.1 mass% (≈ 1 molar equivalent) of unprotected citronellal in the same base was carried out under identical conditions.

During irradiation, the glass tube was connected to a charcoal filter (for air decontamination) and a *Tenax* cartridge and continuously flushed with an air stream (80 ml/min, corresponding to 4 renewals of air/sampling). The diffusion of citronellal was monitored over a period of three hours and four samplings at t = 0, 1, 2 and 3 h were carried out. At each sampling, the citronellal diffusing from the hair was adsorbed onto a *Tenax* cartridge during 15 min, respectively. The cartridges were then thermally desorbed and the concentration of citronellal precisely quantified by GC (Table 11).

**Table 11 :**

| Comparison of the dynamic headspace of free citronellal and citronellal released from precursor **13** in a leave-in hair conditioner irradiated with a Xenon lamp. | | | |
|---|---|---|---|
| Time [h] | Free citronellal in hair conditioner (0.1 mass-%) [ng l⁻¹] | Citronellal released from precursor **13** in hair conditioner (0.2 mass-%) [ng l⁻¹] | Xenon light intensity [lux] |
| 0 | 20700 | 284 | 78000 |
| 1 | 435 | 394 | 86000 |
| 2 | 127 | 237 | 86500 |
| 3 | 39 | 151 | 87500 |

Table 11 illustrates that the concentration of unprotected citronellal decreases rapidly with time whereas the citronellal released from the precursor remains almost constant during the experiment with constant light intensity. After only one hour of irradiation the concentration of citronellal released from the precursor is as high as the concentration of the unprotected aldehyde, and thereafter remains higher than the concentration of the unprotected aldehyde.

### Example 14

### Dynamic headspace analysis for the slow release on cotton fabric

The release of citronellal from precursor **13** was compared to the diffusion of unprotected aldehyde on cotton fabric. For the study, precisely determined amounts of ethanolic solutions containing either 0.2 mass-% of **13** or 0.1 mass-% (≈ 1 molar equivalent) of unprotected citronellal, respectively, were sprayed four times on 4 x 20 cm cotton sheets, which had been washed beforehand with an unperfumed detergent base. The irradiation was carried out in a *Pyrex ®* glass tube for 3 h with a Xenon lamp as described above.

Again a rapid decrease of the released amount unprotected citronellal over time was observed, whereas the release of citronellal from the precursor remained constant with respect to the irradiation intensity, as illustrated in Table 12. The light dependence of the controlled perfume release was verified in a blank experiment. After only 3 h of irradiation comparable concentrations of citronellal were obtained either from the experiment with the free perfume or from release of the precursor compound.

**Table 12 :**

| Comparison of the dynamic headspace of free citronellal and citronellal released from precursor **13** on cotton sheets irradiated with a Xenon lamp. | | | |
|---|---|---|---|
| Time [h] | Free citronellal on cotton (0.1 mass-% in EtOH) [ng l⁻¹] | Citronellal released from precursor **13** on cotton (0.2 mass-% in EtOH) [ng l⁻¹] | Xenon light intensity [lux] |
| 0 - 0.25 | 3022 | 71 | 92500 |
| 1 - 1.25 | 1590 | 168 | 89250 |
| 2 - 2.25 | 469 | 150 | 80750 |
| 3 - 3.25 | 116 | 115 | 81750 |

### Example 15

### Slow release from cotton sheets treated with fabric softener

In a typical experiment, ten cotton towels were washed with an unperfumed, lipase free detergent powder and a fabric softener containing either 0.8 mass-% of keto ester **13** or 0.23 equivalents of the theoretically releasable unprotected aldehyde, respectively. The towels were washed at 40°C without prewashing cycle and dried in the dark overnight. Two towels of each type were irradiated with the above described UV lamp in one covered *Pyrex*® crystallizing dish with an approximative volume of 3.5 1 and compared to a set of non irradiated samples. After 3 h of irradiation the towels were analyzed by nine panelists. In all cases the irradiated towels with precursor **13** were characterized to give a fresh, floral, citrus type odor, and the average intensity was given the value 3 on an increasing scale starting at 0 and ending at 10. In the case of the unprotected citronellal or the two blank samples, the panelists detected only a weak odor with an intensity of 1 on the scale from 0 to 10.

The photoperfume precursor can therefore sucessfully be deposed on fabrics in a normal washing cycle, and the release of the desired perfume is detected in perceptible amounts upon irradiation of the dry fabric.

### Example 16

### Release of menthone from an all-purpose cleaner

An all-purpose cleaner of the Fabuloso ® type containing 0.3% of the compound **18** was prepared. This cleaner and the same cleaner without any perfume were placed into trapezoid flashes which were exposed to sunlight for 3 h (see also Example 11). The thus-obtained samples were then compared on a blind test by a panel of 15 non-experts. When the sample containing the photoperfume was the odd sample, 14 of the panelists correctly distinguished the samples. When the odd sample was the one containing the unperfumed base, 13 of the panelists correctly attributed the samples.

### Example 17

### Release of menthone from a window cleaner

A window cleaner of the type described in Example 11 containing 0.3% of the compound **18** was prepared. This cleaner and the same cleaner without any perfume were placed into trapezoid flashes which were exposed to sunlight for 3 h. The thus-obtained samples were then compared on a blind test by a panel of 15 non-experts. When the sample containing the photoperfume was the odd sample, 12 of the panelists correctly distinguished the samples. When the odd sample was the one containing the unperfumed base, 10 of the panelists correctly attributed the samples.

## Claims

1. Use as a perfuming ingredient of a 2-benzoyl benzoate or a 2-alkanoyl benzoate of formula 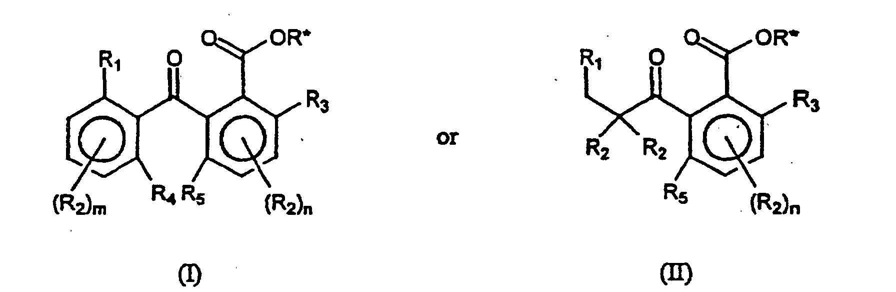 in which
R₁ represents hydrogen or a group of formula 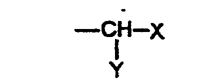 in which X and Y can be identical or different and represent, independently from each other, hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group or a group of formula 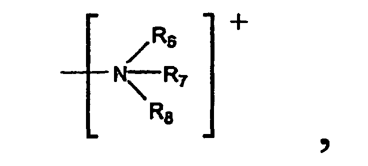 R₂ can be identical to R₁ or different from it and represents hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group, a group of formula 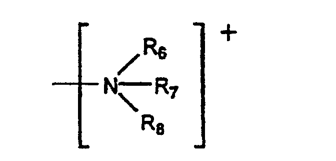 or a polyalcohol or polyether group ;
R₃ represents hydrogen, an alkyl or alkoxy group from C₁ to C₄, linear or branched, a OH group or a NH₂ group ;
R₄ and R₅, taken separately, have the meaning given above for R₁ and can be identical to or different from R₁ or from each other; or
R₄ and R₅, taken together, form a bridging group between the two aromatic rings, which bridging group can be a methylene or a keto group ;
m is an integer from 0 to 3 and n is an integer from 0 to 2; R₆ and R_{7,} taken separately, each represents hydrogen, an alkyl group from C₁ to C₄, an alcohol group having an alkyl chain from C₁ to C₁₂, or a phenyl group, or, R₆ and R₇, taken together with the nitrogen atom form a 5-membered or six-membered ring possibly containing another hetero atom ;
R₈ represents hydrogen, an alkyl group from C₁ to C₄, an alcohol group having an alkyl chain from C₁ to C₁₂ or a phenyl group ;
M represents hydrogen or an alkali metal ; and
R* is the organic part derived from a primary or secondary fragrant alcohol R*OH.

2. Use according to claim 1, wherein the 2-benzoyl benzoate is of formula 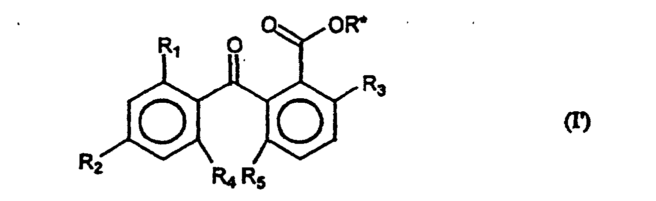 in which
R₁ is a branched alkyl group from C₃ to C₄ containing a secondary hydrocarbon group ;
R₂ is a branched alkyl group from C₃ to C₄ and is identical to R₁ ;
R₃ is hydrogen or a linear or branched alkyl group from C₁ to C₄;
R₄ is hydrogen or a linear or branched alkyl group from C₁ to C₄;
R₅ is hydrogen or a linear or branched alkyl group from C₁ to C₄ ;
R* is the organic part derived from a primary or secondary fragrant alcohol R*OH.

3. Use according to claim 1 or 2, wherein R₁ is an isopropyl group:

4. Use according to any of claims 1 to 3, wherein the fragrant alcohol R*OH from which is derived R* is geraniol, (E)-3,3-dimethyl-5-(2',2',3'-trimethyl-3'-cyclopenten-1'-yl)-4-penten-2-ol or phenethylol.

5. Use according to claim 1, wherein the 2-benzoyl benzoate is geranyl 2-benzoyl benzoate, geranyl 2-(2'-isopropylbenzyl)benzoate, geranyl 2-(2',4'-diisopropylbenzoyl)benzoate or (E)-3,3-dimethyl-5-(2',2',3'-trimethyl-3'-cyclopenten-1'-yl)-4-penten-1-yl 2-(2',4'-diiso-propyl-benzoyl)benzoate.

6. Use according to any of the preceeding claims, wherein there is added to the said 2-benzoyl benzoate a hydrogen radical source which is a solvent selected from the group consisting of primary or secondary aliphatic alcohols, aromatic alcohols, diols and polyols, ketones, esters, alkyl-substituted aromatic compounds, ethers, aminoalcohols and linear and branched hydrocarbons, provided that said solvents contain a linear alkyl group higher than ethyl or a branched secondary alkyl group.

7. Use according to claim 6, wherein the solvent is isopropanol, 1-dodecanol 2-tridecenol, butanol or amyl alcohol.

8. A 2-benzoyl benzoate or alkanoyl benzoate of formula 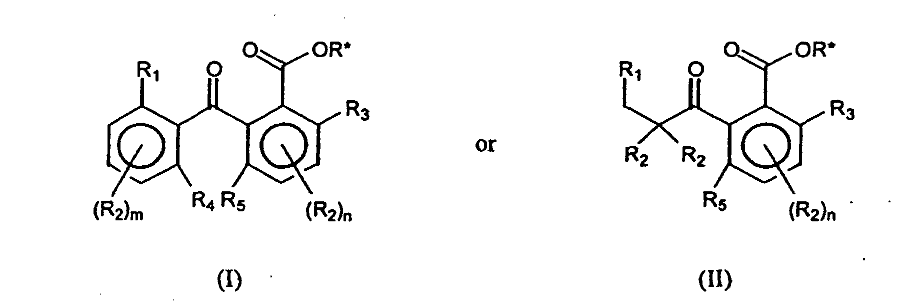 in which
R₁ represents hydrogen or a group of formula 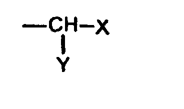 in which X and Y can be identical or different and represent, independently from each other, hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group or a group of formula 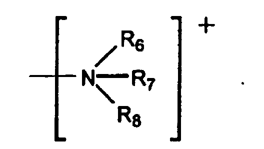 R₂ can be identical to R₁ or different from it and represents hydrogen, a linear or branched alkyl or alkoxy group from C₁ to C₁₂, a phenyl group which is optionally substituted, an olefinic group from C₂ to C₁₂, an alcohol group, a CO₂M group, a -NR₆R₇ group, a group of formula 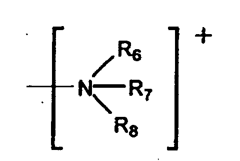 or a polyalcohol or polyether group ;
R₃ represents hydrogen, an alkyl or alkoxy group from C₁ to C₄, linear or branched, a OH group or a NH₂ group ;
R₄ and R₅, taken separately, have the meaning given above for R₁ and can be identical to or different from R₁ or from each other ; or
R₄ and R₅, taken together, form a bridging group between the two aromatic rings, which bridging group can be a methylene or a keto group ;
m is an integer from 0 to 3 and n is an integer from 0 to 2; R₆ and R_{7,} taken separately, each represents hydrogen, an alkyl group from C₁ to C₄, an alcohol group having an alkyl chain from C₁ to C₁₂, or a phenyl group, or, R₆ and R₇, taken together with the nitrogen atom form a 5-membered or six-membered ring possibly containing another hetero atom ;
R₈ represents hydrogen, an alkyl group from C₁ to C₄, an alcohol group having an alkyl chain from C₁ to C₁₂ or a phenyl group ;
M represents hydrogen or an alkali metal ; and
R* is the organic part derived from a primary or secondary fragrant alcohol R*OH ;
provided that geranyl 2-benzoyl benzoate is excluded.

9. A compound according to claim 8, obeying to formula 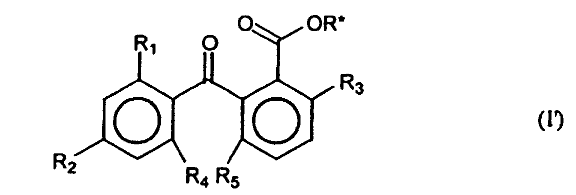 in which
R₁ is a branched alkyl group from C₃ to C₄ containing a secondary hydrocarbon group ;
R₂ is a branched alkyl group from C₃ to C₄ and is identical to R₁ ;
R₃ is hydrogen or a linear or branched alkyl group from C₁ to C₄;
R₄ is hydrogen or a linear or branched alkyl group from C₁ to C₄;
R₅ is hydrogen or a linear or branched alkyl group from C₁ to C₄;
R* is the organic part derived from a primary or secondary fragrant alcohol R*OH.

10. A compound according to claim 8 or 9, wherein R₁ is an isopropyl group.

11. A compound according to any of claims 8 to 10, wherein the fragrant alcohol R*OH from which R* is derived is geraniol, (E)-3,3-dimethyl-5-(2',2',3'-trimethyl-3'-cyclopenten-1'-yl)-4-penten-2-ol or phenethylol.

12. A compound according to claim 8 selected from the group consisting of geranyl 2-(2'-isopropylbenzoyl)benzoate, geranyl 2-(2',4'-diisopropylbenzoyl)benzoate or (E)-3,3-dimethyl-5-(2',2',3'-trimethyl-3'-cyclopenten-1'-yl)-4-penten-1-yl 2-(2',4'-diisopropylbenzoyl)benzoate.

13. Use, upon exposure to light, of an α-keto ester of formula 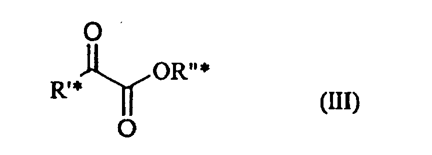 in which
R'* is hydrogen or a linear or branched, unsubstituted or substituted alkyl group or alkylene group from C₁ to C₃₅, an unsubstituted or substituted cycloalkyl group from C₃ to C₈, an unsubstituted or substituted phenyl group, wherein said alkyl, alkylene, cycloalkyl and phenyl groups may comprise one or several hetero atoms not directly linked to the α-keto group and selected from the group consisting of oxygen, nitrogen, phosphorous and sulfur, or
R'* is a substituted or unsubstituted, linear or branched alkyl group carrying an abstractable hydrogen in γ-position relative to the α-keto function and comprising a moiety from which is derived a fragrant compound containing an olefin function, such that said fragrant compound containing an olefin function is eliminated after abstraction of said γ-hydrogen atom ;
R"* is a methyl, ethyl or tert-butyl group or is the organic part of a primary or secondary alcohol R"OH from which is derived a fragrant aldehyde or ketone, and
wherein
at least one of the groups R'* and R"* is a group derived from a fragrant compound provided that decyl 2-oxopropanoate, (Z)-3-hexenyl-2-oxopropanoate and 2-ethyl-3-methylbutyl-2-oxopropanoate are excluded ; as a perfuming ingredient.

14. Use according to claim 13 wherein R"* is the organic part of a primary or secondary alcohol from which is derived a fragrant aldehyde or ketone and wherein R'* is a substituted or unsubstituted phenyl group, a cyclohexyl group, a cyclopentyl group, or a linear or branched alkyl group from C₁ to C₄, with the exception of a n-butyl group.

15. Use according to claim 14, wherein R'* is a phenyl group, a cyclohexyl group, a cyclopentyl group, a methyl group, an ethyl group, or an isopropyl group.

16. Use according to any of claims 13 to 15, wherein the fragrant aldehyde or ketone from which is derived the primary or secondary alcohol from which the organic part R"* is present in the α-keto ester as defined in formula (III) is citronellal, citral, hydroxycitronellal, methyl dihydrojasmonate, 4-(4-hydroxy-1-phenyl)-2-butanone, [3-(4-tert-butylphenyl)-2-methylpropanal), ortho- or para-anisaldehyde, menthone, 2-pentyl-1-cyclopentanone, 2-naphthalenyl-1-ethanone, 4-(1,1-dimethylpropyl)-1-cyclohexanone, benzyl acetone, or a saturated, unsaturated, linear or branched aldehyde from C₆ to C₁₃.

17. Use according to any of claims 13 to 16, wherein the fragrant compound containing an olefin function from which is derived R'* in formula (III) is Iinalool, rnyrcene, myrcenol, a 1,3,5-undecatriene, 9-decen-1-ol, or allyl heptanoate.

18. Perfuming composition or perfumed article, resulting from the use according to any of claims 1 to 7 and 13 to 17.

19. Perfuming composition or perfumed article according to claim 18 in the form of a perfume or a cologne, a bath or shower gel, a hair-care product, a cosmetic preparation, a body deodorant, a solid or liquid air-freshener, a detergent or a fabric softener, or a household product.

20. Perfumed article according to claim 18, in the form of an all purpose cleaner or an all purpose household cleaner, a window cleaner, a furniture polish, a fabric conditioner, softener or wash in form of a powder, a liquid or a tablet, a shampoo, a hair conditioner, a leave- in hair conditioner, or a hairspray.

21. α-Keto ester of formula 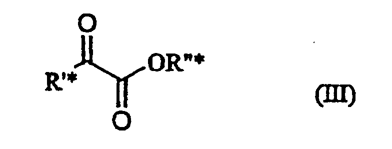 in which
R'* is a linear or branched, unsubstituted or substituted alkyl group or alkylene group from C₁ to C₃₅, an unsubstituted or substituted cycloalkyl group from C₃ to C₈, a substituted phenyl group, wherein said alkyl, alkylene, cycloalkyl and phenyl groups may comprise one or several hetero atoms not directly linked to the α-keto group and selected from the group consisting of oxygen, nitrogen, phosphorous and sulfur, or
R'* is a substituted or unsubstituted, linear or branched alkyl group carrying an abstractable hydrogen in γ-position relative to the α-keto function and comprising a moiety from which is derived a fragrant compound containing an olefin function, such that said fragrant compound containing an olefin function is eliminated after abstraction of said γ-hydrogen atom ;
R"* is a methyl, ethyl or tert-butyl group or is the organic part of a primary or secondary alcohol from which is derived a fragrant aldehyde or ketone, and wherein at least one of the groups R'* and R"* is a group derived from a fragrant compound ;
provided that R'* is not a methyl group,
R"* is not a menthyl or a benzyl group, and that
(-)-(1S,1R) 1,7,7-trimethylbicyclo[2.2.1]heptan-2-yl (4-methylphenyl)oxoacetate, butyl cyclohexylglyoxalate, 2'-hexenyl phenylglyoxylate, 3'- hexenyl phenylglyoxylate, 5'- hexenyl phenylglyoxylate, allyl phenylglyoxylate, 3'-methylbut-2'enyl phenylglyoxylate, 4'-methylpent-3'-enyl phenylglyoxylate, 1'5'-dimethylhex-4'-enyl phenylglyoxylate and hexyl (cyclohexyl)oxoacetate are excluded.

22. α-Keto ester according to claim 21 wherein R"* is the organic part of a primary or secondary alcohol from which is derived a fragrant aldehyde or ketone and in which R'* is a cyclohexyl group, a cyclopentyl group, or a linear or branched alkyl group from C₁ to C₄, with the exception of a n-butyl group.

23. α-Keto ester according to claim 22, wherein the alkyl group is a methyl, ethyl or isopropyl group.

24. α-Keto ester according to any of claims 21 to 23, wherein the fragrant aldehyde or ketone from which is derived the primary or secondary alcohol from which the organic part R"* is present in the α-keto ester as defined in formula (III) is citronellal, citral, hydroxycitronellal, methyl dihydrojasmonate, 4-(4-hydroxy-1-phenyl)-2-butanone, [3-(4-tert-butylphenyl)-2-methylpropanal], ortho- or par anisaldehyde, menthone, 2-pentyl-1-cyclopentanone, 2-naphthalenyl-1-ethanone, 4-(1,1-dimethyl)-1-cyclohexanone, benzyl acetone or a saturated, unsaturated, linear or branched aldehyde from C₆ to C₁₃.

25. α-Keto ester according to any of claims 21 to 24, wherein the fragrant compound containing an olefin function from which is derived R'* in formula (III) is linalool, myrcene, myrcenol, a 1,3,5-undecatriene, 9-decen-1-ol, or allyl heptanoate.

## Patentansprüche

1. Verwendung, als Duftbestandteil, eines 2-Benzoylbenzoats oder eines 2-Alkanoylbenzoats der Formel 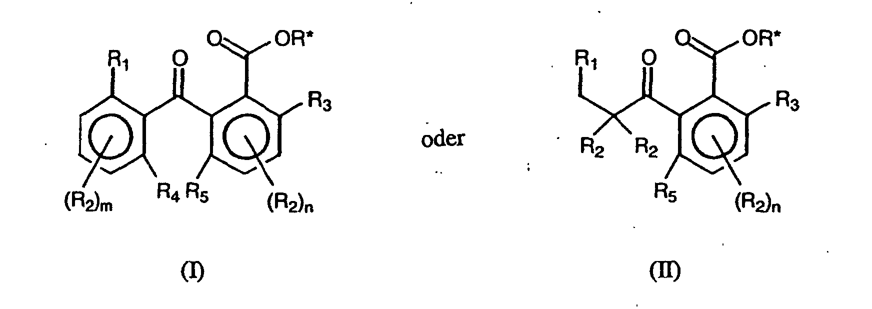 wobei
R₁ Wasserstoff oder eine Gruppe der Formel 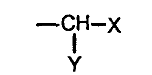 bedeutet, wobei X und Y identisch oder verschieden sein können und unabhängig voneinander Wasserstoff, eine lineare oder verzweigte C₁-C₁₂-Alkyl- oder - Alkoxygruppe, eine Phenylgruppe, die gegebenenfalls substituiert ist, eine C₂-C₁₂-Olefmgruppe, eine Alkoholgruppe, eine CO₂M-Gruppe, eine -NR₆R₇-Gruppe oder eine Gruppe der Formel 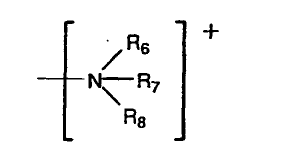 bedeuten,
R₂ mit R₁ identisch oder davon verschieden sein kann und Wasserstoff, eine lineare oder verzweigte C₁-C₁₂-Alkyl- oder -Alkoxygruppe, eine Phenylgruppe, die gegebenenfalls substituiert ist, eine C₂-C₂₂-Olefingruppe, eine Alkoholgruppe, eine CO₂M-Gruppe, eine -NR₆R₇-Gruppe, eine Gruppe der Formel 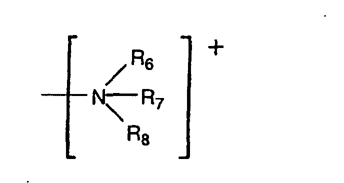 oder eine Polyalkohol- oder Polyethergruppe bedeutet;
R₃ Wasserstoff, eine lineare oder verzweigte C₁-C₄-Alkyl- oder -Alkoxygruppe, eine OH-Gruppe oder eine NH₂-Gruppe bedeutet;
R₄ and R₅ einzeln die oben für R₁ angegebene Bedeutung haben und mit R₁ identisch oder davon oder voneinander verschieden sein können; oder
R₄ and R₅ zusammen eine Brückengruppe zwischen den beiden aromatischen Ringen bilden, wobei die Brückengruppe eine Methylen- oder eine Ketogruppe sein kann;
m eine ganze Zahl von 0 bis 3 ist und n eine ganze Zahl von 0 bis 2 ist; R₆ and R₇ einzeln jeweils Wasserstoff, eine C₁-C₄-Alkylgruppe, eine Alkoholgruppe mit einer C₁-C₁₂-Alkylkette oder eine Phenylgruppe bedeuten oder R₆ and R₇ zusammen mit dem Stickstoffatom einen 5-gliedrigen oder 6-gliedrigen Ring bilden, der gegebenenfalls ein weiteres Heteroatom enthält;
R₈ Wasserstoff, eine C₁-C₄-Alkylgruppe, eine Alkoholgruppe mit einer C₁-C₁₂-Alkylkette oder eine Phenylgruppe bedeutet;
M Wasserstoff oder ein Alkalimetall bedeutet; und
R* der von einem primären oder sekundären duftenden Alkohol R*OH abgeleitete organische Teil ist.

2. Verwendung nach Anspruch 1, wobei das 2-Benzoylbenzoat die Formel 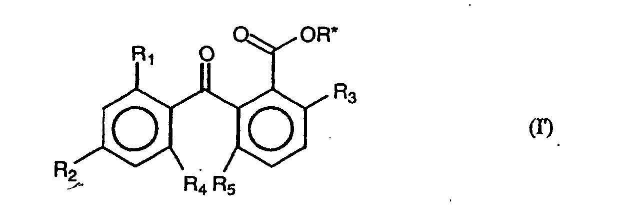 besitzt, wobei
R₁ eine verzweigte C₃-C₄-Alkylgruppe ist, die eine sekundäre Kohlenwasserstoffgruppe enthält;
R₂ eine verzweigte C₃-C₄-Alkylgruppe ist und mit R₁ identisch ist;
R₃ Wasserstoff oder eine lineare oder verzweigte C₁-C₄-Alkylgruppe ist;
R₄ Wasserstoff oder eine lineare oder verzweigte C₁-C₄-Alkylgruppe ist;
R₅ Wasserstoff oder eine lineare oder verzweigte C₁-C₄-Alkylgruppe ist;
R* der von einem primären oder sekundären duftenden Alkohol R*OH abgeleitete organische Teil ist.

3. Verwendung nach Anspruch 1 oder 2, wobei R₁ eine Isopropylgruppe ist.

4. Verwendung nach irgendeinem der Ansprüche 1 bis 3, wobei der duftende Alkohol R*OH, von dem R* abgeleitet ist, Geraniol, (E)-3,3-Dimethyl-5-(2',2',3'-trimethyl-3'cyclopenten-1'-yl)-4-penten-2-ol oder Phenethylol ist.

5. Verwendung nach Anspruch 1, wobei das 2-Benzoylbenzoat Geranyl-2-benzoylbenzoat, Geranyl-2-(2'-isopropylbenzoyl)benzoat, Geranyl-2-(2',4'diisopropylbenzoyl)benzoat oder (E)-3,3-Dimethyl-5-(2',2',3'-trimethyl-3'-cyclopenten-1'-yl)-4-penten-1-yl-2-(2',4'-diisopropyl-benzoyl)benzoat ist.

6. Verwendung nach irgendeinem der vorhergehenden Ansprüche, wobei dem 2-Benzoylbenzoat eine Wasserstoffradikalquelle zugesetzt wird, welche ein Lösungsmittel ist, ausgewählt aus der Gruppe bestehend aus primären oder sekundären aliphatischen Alkoholen, aromatischen Alkoholen, Diolen und Polyolen, Ketonen, Estern, alkylsubstituierten aromatischen Verbindungen, Ethern, Aminoalkoholen und linearen und verzweigten Kohlenwasserstoffen, mit der Einschränkung, dass die Lösungsmittel eine lineare Alkylgruppe höher als Ethyl oder eine verzweigte sekundäre Alkylgruppe enthalten.

7. Verwendung nach Anspruch 6, wobei das Lösungsmittel Isopropanol, 1-Dodekanol, 2-Tridecenol, Butanol oder Amylalkohol ist.

8. 2-Benzoylbenzoat oder -Alkanoylbenzoat der Formel 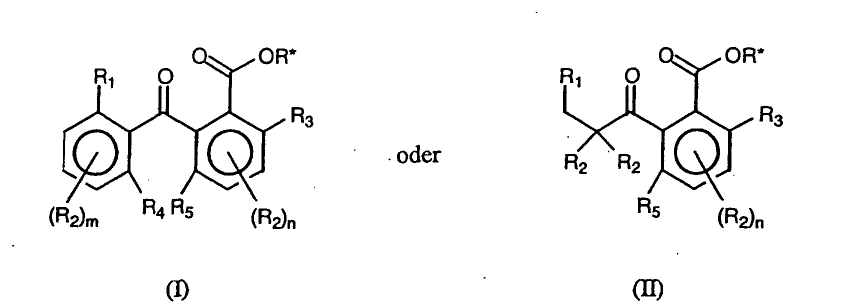 wobei
R₁ Wasserstoff oder eine Gruppe der Formel 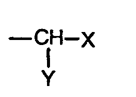 bedeutet, wobei X und Y identisch oder verschieden sein können und unabhängig voneinander Wasserstoff, eine lineare oder verzweigte C₁-C₁₂-Alkyl- oder - Alkoxygruppe, eine Phenylgruppe, die gegebenenfalls substituiert ist, eine C₂-C₁₂-Olefingruppe, eine Alkoholgruppe, eine CO₂M-Gruppe, eine -NR₆R₇-Gruppe oder eine Gruppe der Formel 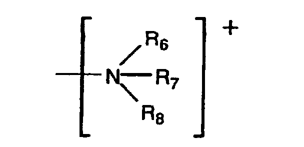 bedeuten,
R₂ mit R₁ identisch oder davon verschieden sein kann und Wasserstoff, eine lineare oder verzweigte C₁-C₁₂-Alkyl- oder -Alkoxygruppe, eine Phenylgruppe, die gegebenenfalls substituiert ist, eine C₂-C₁₂-Olefingruppe, eine Alkoholgruppe, eine CO₂M-Gruppe, eine -NR₆R₇-Gruppe, eine Gruppe der Formel 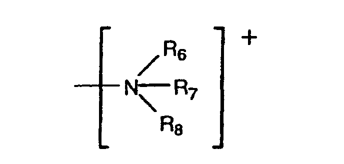 oder eine Polyalkohol- oder Polyethergruppe bedeutet;
R₃ Wasserstoff, eine lineare oder verzweigte C₁-C₄-Alkyl- oder -Alkoxygruppe, eine OH-Gruppe oder eine NH₂-Gruppe bedeutet;
R₄ and R₅ einzeln die oben für R₁ angegebene Bedeutung haben und mit R₁ identisch oder davon oder voneinander verschieden sein können; oder
R₄ and R₅ zusammen eine Brückengruppe zwischen den beiden aromatischen Ringen bilden, wobei die Brückengruppe eine Methylen- oder eine Ketogruppe sein kann;
m eine ganze Zahl von 0 bis 3 ist und n eine ganze Zahl von 0 bis 2 ist; R₆ and R₇ einzeln jeweils Wasserstoff, eine C₁-C₄-Alkylgruppe, eine Alkoholgruppe mit einer C₁-C₁₂-Alkylkette oder eine Phenylgruppe bedeuten oder R₆ and R₇ zusammen mit dem Stickstoffatom einen 5-gliedrigen oder 6-gliedrigen Ring bilden, der gegebenenfalls ein weiteres Heteroatom enthält;
R₈ Wasserstoff, eine C₁-C₄-Alkylgruppe, eine Alkoholgruppe mit einer C₁-C₁₂-Alkylkette oder eine Phenylgruppe bedeutet;
M Wasserstoff oder ein Alkalimetall bedeutet; und
R* der von einem primären oder sekundären duftenden Alkohol R*OH abgeleitete organische Teil ist;
mit der Einschränkung, dass Geranyl-2-benzoylbenzoat ausgenommen ist.

9. Verbindung nach Anspruch 9 entsprechend der Formel 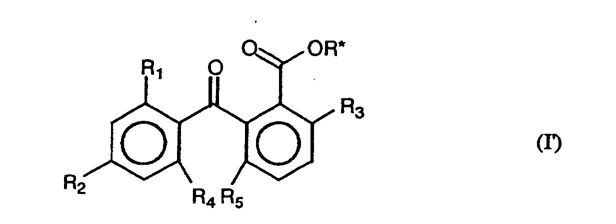 wobei
R₁ eine verzweigte C₃-C₄-Alkylgruppe ist, die eine sekundäre Kohlenwasserstoffgruppe enthält;
R₂ eine verzweigte C₃-C₄-Alkylgruppe ist und mit R₁ identisch ist;
R₃ Wasserstoff oder eine lineare oder verzweigte C₁-C₄-Alkylgruppe ist;
R₄ Wasserstoff oder eine lineare oder verzweigte C₁-C₄-Alkylgruppe ist;
R₅ Wasserstoff oder eine lineare oder verzweigte C₁-C₄-Alkylgruppe ist;
R* der von einem primären oder sekundären duftenden Alkohol R*OH abgeleitete organische Teil ist.

10. Verbindung nach Anspruch 8 oder 9, wobei R₁ eine Isopropylgruppe ist.

11. Verbindung nach irgendeinem der Ansprüche 8 bis 10, wobei der duftende Alkohol R*OH, von dem R* abgeleitet ist, Geraniol, (E)-3,3-Dimethyl-5-(2',2';3'-trimethyl-3'cyclopenten-1'-yl)-4-penten-2-ol oder Phenethylol ist.

12. Verbindung nach Anspruch 8, ausgewählt aus der Gruppe bestehend aus Geranyl-2-(2'isopropylbenzoyl)benzoat, Geranyl-2-(2',4'-diisopropylbenzoyl)benzoat oder (E)-3,3-Dimethyl-5-(2',2',3'-trimethyl-3'-cyclopenten-1'-yl)-4-penten-1-yl-2-(2',4'-diisopropylbenzoyl)benzoat.

13. Verwendung, nach Lichtexposition, eines α-Ketoesters der Formel 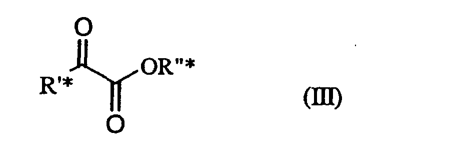 wobei
R'* Wasserstoff oder eine lineare oder verzweigte, unsubstituierte oder substituierte C₁-C₃₅-Alkyl- oder -Alkylengruppe, eine unsubstituierte oder substituierte C₃-C₈-Cycloalkylgruppe, eine unsubstituierte oder substituierte Phenylgruppe ist, wobei die Alkyl-, Alkylen-, Cycloalkyl- und Phenylgruppen ein oder mehrere Heteroatome umfassen können, die nicht direkt mit der α-Ketogruppe verknüpft sind und ausgewählt sind aus der Gruppe bestehend aus Sauerstoff, Stickstoff, Phosphor und Schwefel, oder
R'* eine substituierte oder unsubstituierte, lineare oder verzweigte Alkylgruppe ist, die in γ-Stellung zur α-Ketofunktion einen abspaltbaren Wasserstoff trägt und eine Komponente umfasst, von der eine duftende Verbindung abgeleitet ist, die eine Olefinfunktion enthält, so dass die eine Olefinfunktion enthaltende duftende Verbindung nach Abspaltung des γ-Wasserstoffatoms eliminiert wird;
R"* eine Methyl-, Ethyl- oder tert.-Butylgruppe ist oder der organische Teil eines primären oder sekundären Alkohols R"OH ist, von dem ein duftender Aldehyd oder ein duftendes Keton abgeleitet ist, und wobei
wenigstens eine der Gruppen R'* und R"* eine von einer duftenden Verbindung abgeleitete Gruppe ist, mit der Einschränkung, dass Decyl-2-oxopropanoat, (Z)-3-Hexenyl-2-oxopropanoat und 2-Ethyl-3-methylbutyl-2-oxopropanoat ausgenommen sind; als Duftbestandteil.

14. Verwendung nach Anspruch 13, wobei R"* der organische Teil eines primären oder sekundären Alkohols ist, von dem ein duftender Aldehyd oder ein duftendes Keton abgeleitet ist, und wobei R'* eine substituierte oder unsubstituierte Phenylgruppe, eine Cyclohexylgruppe, eine Cyclopentylgruppe oder eine lineare oder verzweigte C₁-C₄-Alkylgruppe mit Ausnahme einer n-Butylgruppe ist.

15. Verwendung nach Anspruch 14, wobei R'* eine Phenylgruppe, eine Cyclohexylgruppe, eine Cyclopentylgruppe, eine Methylgruppe, eine Ethylgruppe oder eine Isopropylgruppe ist.

16. Verwendung nach irgendeinem der Ansprüche 13 bis 15, wobei der duftende Aldehyd oder das duftende Keton, von dem der primäre oder sekundäre Alkohol abgeleitet ist, dessen organischer Teil R"* in dem wie in Formel III definierten α-Ketoester vorliegt, Citronellal, Citral, Hydroxycitronellal, Methyldihydrojasmonat, 4-(4-Hydroxy-1-phenyl)-2-butanon, [3-(4-tert.-Butylphenyl)-2-methylpropanal], ortho- oder para-Anisaldehyd, Menthon, 2-Pentyl-1-cyclopentanon, 2-Naphthalinyl-1-ethanon, 4-(1,1-Dimethylpropyl)-1-cyclohexanon, Benzylaceton oder ein gesättigter, ungesättigter, linearer oder verzweigter C₆-C₁₃-Aldehyd ist.

17. Verwendung nach irgendeinem der Ansprüche 13 bis 16, wobei die duftende Verbindung, die eine Olefinfunktion enthält, von der R'* in der Formel (III) abgeleitet ist, Linalool, Myrcen, Myrcenol, ein 1,3,5-Undecatrien, 9-Decen-1-ol oder Allylheptanoat ist.

18. Duftzusammensetzung oder parfümierter Artikel, der aus der Verwendung nach irgendeinem der Ansprüche 1 bis 7 und 13 bis 17 resultiert.

19. Duftzusammensetzung oder parfümierter Artikel nach Anspruch 18 in Form eines Parfüms oder von Kölnisch Wasser, eines Bade- oder Duschgels, eines Haarpflegeprodukts, eines Kosmetikpräparats, eines Körperdeodorants, eines festen oder flüssigen Lufterfrischers, eines Reinigungsmittels oder eines Textilweichspülers oder eines Haushaltsprodukts.

20. Parfümierter Artikel nach Anspruch 18 in Form eines Allzweckreinigers oder eines Allzweckhaushaltsreinigers, eines Fensterreinigers, einer Möbelpolitur, eines Gewebe-Conditioners, -weichspülers oder -waschmittels in Form eines Pulvers, einer Flüssigkeit oder einer Tablette, eines Shampoos, eines Haar-Conditioners, eines im Haar verbleibenden Conditioners oder eines Haarsprays.

21. α-Ketoester der Formel 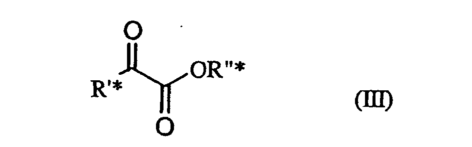 wobei
R'* eine lineare oder verzweigte, unsubstituierte oder substituierte C₁-C₃₅-Alkyloder -Alkylengruppe, eine unsubstituierte oder substituierte C₃-C₈-Cycloalkylgruppe, eine substituierte Phenylgruppe ist, wobei die Alkyl-, Alkylen-, Cycloalkyl- und Phenylgruppen ein oder mehrere Heteroatome umfassen können, die nicht direkt mit der α-Ketogruppe verknüpft sind und ausgewählt sind aus der Gruppe bestehend aus Sauerstoff, Stickstoff, Phosphor und Schwefel, oder
R'* eine substituierte oder unsubstituierte, lineare oder verzweigte Alkylgruppe ist, die in γ-Stellung zur α-Ketofunktion einen abspaltbaren Wasserstoff trägt und eine Komponente umfasst, von der eine duftende Verbindung abgeleitet ist, die eine Olefinfunktion enthält, so dass die eine Olefinfunktion enthaltende duftende Verbindung nach Abspaltung des γ-Wasserstoffatoms eliminiert wird;
R"* eine Methyl-, Ethyl- oder tert.-Butylgruppe ist oder der organische Teil eines primären oder sekundären Alkohols R"OH ist, von dem ein duftender Aldehyd oder ein duftendes Keton abgeleitet ist, und wobei wenigstens eine der Gruppen R'* und
R"* eine von einer duftenden Verbindung abgeleitete Gruppe ist;
mit der Einschränkung, dass R'* keine Methylgruppe ist,
R"* keine Menthyl- oder Benzylgruppe ist, und dass
(-)-(1S,1R)-1,7,7-Trimethylbicyclo[2.2.1]heptan-2-yl-(4-methylphenyl)oxoacetat, Butylcyclohexylglyoxalat, 2'-Hexenylphenylglyoxylat, 3'-Hexenylphenylglyoxylat, 5'-Hexenylphenylglyoxylat, Allylphenylglyoxylat, 3'-Methylbut-2'-enylphenylglyoxylat, 4'-Methylpent-3'-enyl-phenylglyoxylat, 1',5'-Dimethylbut-4'-enylphenylglyoxylat and Hexyl(cyclohexyl)oxoacetat ausgenommen sind.

22. α-Ketoester nach Anspruch 21, wobei R"* der organische Teil eines primären oder sekundären Alkohols ist, von dem ein duftender Aldehyd oder ein duftendes Keton abgeleitet ist und in dem R'* eine Cyclohexylgruppe, eine Cyclopentylgruppe oder eine lineare oder verzweigte C₁-C₄-Alkylgruppe mit Ausnahme einer n-Butylgruppe ist.

23. α-Ketoester nach Anspruch 22, wobei die Alkylgruppe eine Methyl-, Ethyl oder Isopropylgruppe ist.

24. α-Ketoester nach irgendeinem der Ansprüche 21 bis 23, wobei der duftende Aldehyd oder das duftende Keton, von dem der primäre oder sekundäre Alkohol abgeleitet ist, dessen organischer Teil R"* in dem wie in Formel III definierten α-Ketoester vorliegt, Citronellal, Citral, Hydroxycitronellal, Methyldihydrojasmonat, 4-(4-Hydroxy-1-phenyl)-2-butanon, [3-(4-tert.-Butylphenyl)-2-methylpropanal], ortho- oder para-Anisaldehyd, Menthon, 2-Pentyl-1-cyclopentanon, 2-Naphthalinyl-1-ethanon, 4-(1,1-Dimethylpropyl)-1-cyclohexanon, Benzylaceton oder ein gesättigter, ungesättigter, linearer oder verzweigter C₆-C₁₃-Aldehyd ist.

25. α-Ketoester nach irgendeinem der Ansprüche 21 bis 24, wobei die duftende Verbindung, die eine Olefinfunktion enthält, von der R'* in der Formel (III) abgeleitet ist, Linalool, Myrcen, Myrcenol, ein 1,3,5-Undecatrien, 9-Decen-1-ol oder Allylheptanoat ist.

## Revendications

1. Utilisation à titre d'ingrédient parfumant d'un 2-benzoyle benzoate ou d'un 2-alkanoyle benzoate de formule 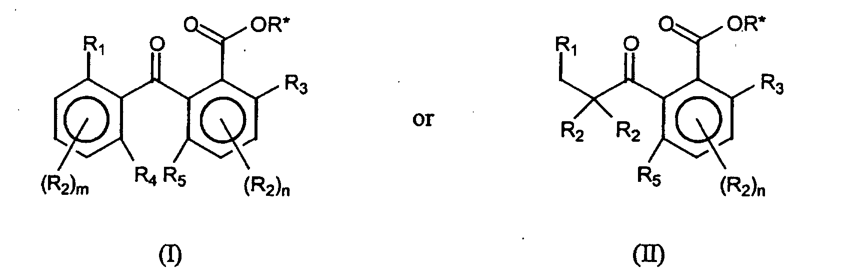 dans lesquelles
R₁ représente un hydrogène ou un groupe de formule 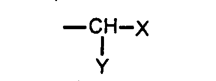 dans laquelle X et Y peuvent être identiques ou différents et représentent, indépendamment l'un de l'autre, un hydrogène, un groupe alkyle ou alkoxy linéaire
ou ramifié de C₁ à C₁₂, un groupe phényle facultativement substitué, un groupe oléfinique de C₂ à C₁₂, un groupe alcoolique, un groupe CO₂M, un groupe -NR₆R₇ ou un groupe of formule 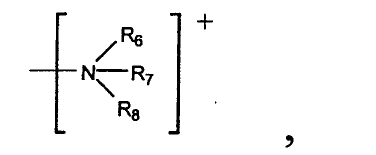 R₂ peut être identique à R₁ ou différent de celui-ci et représente un hydrogène, un groupe alkyle ou alkoxy linéaire ou ramifié de C₁ à C₁₂, un groupe phényle facultativement substitué, un groupe oléfinique de C₂ à C₁₂, un groupe alcoolique, un groupe CO₂M, un groupe -NR₆R₇, un groupe de formule 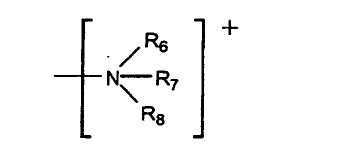 ou un polyalcool ou un polyéther ;
R₃ représente un hydrogène, un groupe alkyle ou alkoxy de C₁ à C₄, linéaire ou ramifié, un groupe OH ou un groupe NH₂ ;
R₄ et R₅, pris séparément, ont le sens donné plus haut pour R₁ et peuvent être identiques ou différents de R₁ ou l'un de l'autre ; ou
R₄ et R₅, pris ensemble, forment un pont entre les deux cycles aromatiques, lequel pont peut être un groupe méthylène ou un groupe céto ;
m est un entier de 0 à 3 et n est un entier de 0 à 2 ; R₆ et R₇, pris séparément, représentent chacun un hydrogène, un groupe alkyle de C₁ à C₄, un groupe alcoolique ayant une chaîne alkyle de C₁ à C₁₂, ou un groupe phényle, ou, R₆ et R₇, pris ensemble avec l'atome d'azote forment un cycle à 5 ou 6 membres pouvant contenir un autre hétéro atome ;
R₈ représente un hydrogène, un groupe alkyle de C₁ à C₄, un groupe alcoolique ayant une chaîne alkyle de C₁ à C₁₂ ou un groupe phényle ;
M représente un hydrogène ou un métal alcalin ; et
R* est la partie organique dérivée d'un alcool odorant primaire ou secondaire R*OH.

2. Utilisation selon la revendication 1, **caractérisée en ce que** le 2-benzoyle benzoate a pour formule 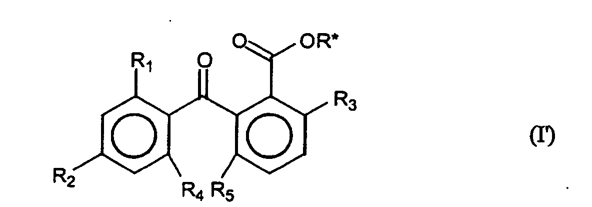 dans laquelle
R₁ est un groupe alkyle ramifié de C₃ à C₄ contenant un groupe hydrocarbure secondaire ;
R₂ est un groupe alkyle ramifié de C₃ à C₄ et est identique à R₁ ;
R₃ est un hydrogène ou un groupe alkyle linéaire ou ramifié de C₁ à C₄;
R₄ est un hydrogène ou un groupe alkyle linéaire ou ramifié de C₁ à C₄;
R₅ est un hydrogène ou un groupe alkyle linéaire ou ramifié de C₁ à C₄ ;
R* est la partie organique dérivée d'un alcool odorant primaire ou secondaire R*OH.

3. Utilisation selon la revendication 1 ou 2, **caractérisée en ce que** R₁ est un groupe isopropyle.

4. Utilisation selon l'une des revendications 1 à 3, **caractérisée en ce que** l'alcool odorant R*OH dont est dérivé R* est le géraniol, le (E)-3,3-diméthyl-5-(2',2',3'-triméthyl-3'-cyclopentèn-1'-yle)-4-pentèn-2-ol ou le phénéthylol.

5. Utilisation selon la revendication 1, **caractérisée en ce que** le 2-benzoyle benzoate est le 2-benzoyle benzoate de géranyle, le 2-(2'-isopropylbenzyl)benzoate de géranyle, le 2-(2',4'-diisopropylbenzoyl)benzoate de géranyle ou le 2-(2',4'-diiso-propyl-benzoyl)benzoate de (E)-3,3-diméthyl-5-(2',2',3'-triméthyl-3'-cyclopentèn-1'-yle)-4-pentèn-1-yle.

6. Utilisation selon l'une des revendications précédentes, **caractérisée en ce que** l'on ajoute audit 2-benzoyl benzoate une source radicalaire d'hydrogène qui est un solvant choisi dans le groupe constitué d'alcools aliphatiques primaires ou secondaires, d'alcools aromatiques, de diols et polyols, de cétones, d'esters, de composés aromatiques substitués par des groupements alkyles, d'éthers, d'aminoalcools et d'hydrocarbures linéaires et ramifiés, étant entendu que lesdits solvants contiennent un groupement alkyle linéaire plus long qu'un éthyle ou qu'un groupement alkyle secondaire ramifié.

7. Utilisation selon la revendication 6, **caractérisée en ce que** le solvant est de l'isopropanol, du 1-dodécanol 2-tridécénol, du butanol ou de l'alcool amylique.

8. 2-Benzoyl benzoate ou alkanoyl benzoate de formule 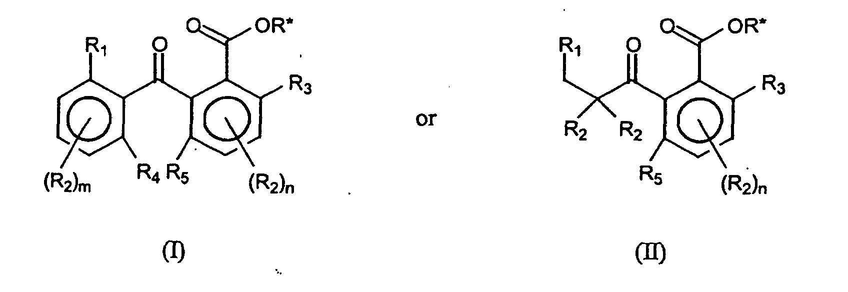 dans lesquelles
R₁ représente un hydrogène ou un groupe de formule 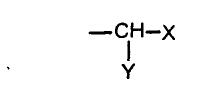 dans laquelle X et Y peuvent être identiques ou différents et représentent, indépendamment l'un de l'autre, un hydrogène, un groupe alkyle ou alkoxy linéaire
ou ramifié de C₁ à C₁₂, un groupe phényle facultativement substitué, un groupe oléfinique de C₂ à C₁₂, un groupe alcoolique, un groupe CO₂M, un groupe -NR₆R₇ ou un groupe de formule 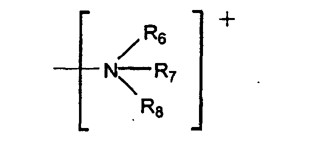 R₂ peut être identique à R₁ ou différent de celui-ci et représente un hydrogène, un groupe alkyle ou alkoxy linéaire ou ramifié de C₁ à C₁₂, un groupe phényle facultativement substitué, un groupe oléfinique de C₂ à C₁₂, un groupe alcoolique, un groupe CO₂M, un groupe -NR₆R₇, un groupe de formule 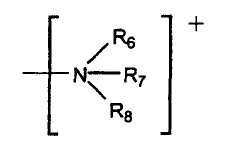 ou un polyalcool ou polyéther ;
R₃ représente un hydrogène, un groupe alkyle ou alkoxy de C₁ à C₄, linéaire ou ramifié, un groupe OH ou un groupe NH₂ ;
R₄ et R₅, pris séparément, ont le sens donné plus haut pour R₁ et peuvent être identiques ou différents de R₁ ou l'un de l'autre ; ou
R₄ et R₅, pris ensemble, forment un pont entre les deux cycles aromatiques, lequel pont peut être un groupe méthylène ou céto ;
m est un entier de 0 à 3 et n est un entier de 0 à 2 ; R₆ et R₇, pris séparément, représentent chacun un hydrogène, un groupe alkyle de C₁ à C₄, un groupe alcoolique ayant une chaîne alkyle de C₁ à C₁₂, ou un groupe phényle, ou, R₆ et R₇, pris ensemble avec l'atome d'azote forment un cycle à 5 ou 6 membres pouvant contenir un autre hétéro atome ;
R₈ représente un hydrogène, un groupe alkyle de C₁ à C₄, un groupe alcoolique ayant une chaîne alkyle de C₁ à C₁₂ ou un groupe phényle ;
M représente un hydrogène ou un métal alcalin ; et
R* est la partie organique dérivée d'un alcool odorant primaire ou secondaire R*OH ;
étant entendu que le 2-benzoyl benzoate de géranyle est exclu.

9. Composé selon la revendication 8, obéissant à la formule 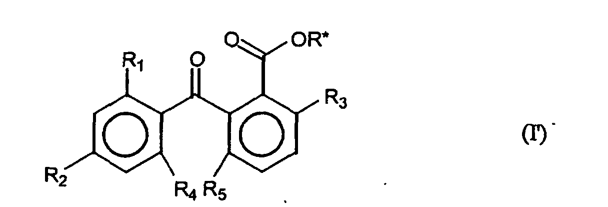 dans laquelle
R₁ est un groupe alkyle ramifié de C₃ à C₄ contenant un groupe hydrocarbure secondaire ;
R₂ est un groupe alkyle ramifié de C₃ à C₄ et est identique à R₁ ;
R₃ est un hydrogène ou un groupe alkyle linéaire ou ramifié de C₁ à C₄;
R₄ est un hydrogène ou un groupe alkyle linéaire ou ramifié de C₁ à C₄;
R₅ est un hydrogène ou un groupe alkyle linéaire ou ramifié de C₁ à C₄ ;
R* est la partie organique dérivée d'un alcool odorant primaire ou secondaire R*OH.

10. Composé selon la revendication 8 ou 9, caractérisé en ce que-R₁ est un groupe isopropyle.

11. Composé selon l'une des revendications 8 à 10, **caractérisé en ce que** l'alcool odorant R*OH dont R* est dérivé est le géraniol, le (E)-3,3-diméthyl-5-(2',2',3'-triméthyl-3'-cyclopentèn-1'-yle)-4-pentèn-2-ol ou le phénéthylol.

12. Composé selon la revendication 8 choisi dans le groupe constitué du 2-(2'-isopropylbenzoyle)benzoate de géranyle, du 2-(2',4'-diisopropylbenzoyle)benzoate de géranyle ou du 2-(2',4'-diisopropylbenzoyl)benzoate de (E)-3,3-diméthyl-5-(2',2',3'-triméthyl-3'-cyclopentèn-1'-yle)-4-pentèn-1-yle.

13. Utilisation, sous exposition à la lumière, d'un α-céto ester de formule 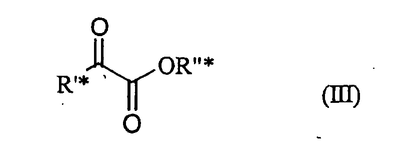 dans laquelle
R'* est un hydrogène ou un groupe alkyle ou alkylène linéaire ou ramifié, non substitué ou substitué de C₁ à C₃₅, un groupe cycloalkyle non substitué ou substitué de C₃ à C₈, un groupe phényle non substitué ou substitué, lesdits groupes alkyle, alkylène, cycloalkyle et phényle pouvant comprendre un ou plusieurs hétéro atomes non directement liés au groupe α-céto et choisis dans le groupe constitué de l'oxygène, l'azote, le phosphore et le soufre ou
R'* est un groupe alkyle substitué ou non substitué, linéaire ou ramifié portant un hydrogène susceptible d'être arraché en position γ par rapport à la fonction α-céto et comprenant une entité dont est dérivé un composé odorant contenant une fonction oléfine, de façon à ce que ledit composé odorant contenant une fonction oléfine soit éliminé après que ledit atome d'hydrogène en position γ a été arraché ;
R"* est un groupe méthyle, éthyle ou tert-butyle ou est la partie organique d'un alcool primaire ou secondaire R"OH dont est dérivé un aldéhyde odorant ou une cétone odorante,
et dans laquelle
au moins l'un des groupes R'* et R"* est un groupe dérivé d'un composé odorant, étant entendu que le 2-oxopropanoate de décyle, le 2-oxopropanoate de (Z)-3-hexenyle et le 2-oxopropanoate de 2-éthyl-3-méthylbutyle sont exclus.

14. Utilisation selon la revendication 13 **caractérisée en ce que** R"* est la partie organique d'un alcool primaire ou secondaire dont est dérivé un aldéhyde odorant ou d'une cétone odorante et **caractérisée en ce que** R'* est un groupe phényle substitué ou non substitué, un groupe cyclohexyle, un groupe cyclopentyle, ou un groupe alkyle linéaire ou ramifié de C₁ à C₄, à l'exception d'un groupe n-butyle.

15. Utilisation selon la revendication 14, **caractérisée en ce que** R'* est un groupe phényle, un groupe cyclohexyle, un groupe cyclopentyle, a un groupe méthyle, un groupe éthyle, ou un groupe isopropyle.

16. Utilisation selon l'une des revendications 13 à 15, **caractérisée en ce que** l'aldéhyde odorant ou la cétone odorante dont est dérivé l'alcool primaire ou secondaire dont la partie organique R"* est présente dans l'α-céto ester tel que défini à la formule (III) est le citronellal, le citral, l'hydroxycitronellal, le dihydrojasmonate de méthyle, la 4-(4-hydroxy-1-phényle)-2-butanone, le [3-(4-tert-butylphényl)-2-méthylpropanal], ortho- or para-anisaldéhyde, la menthone, la 2-pentyl-1-cyclopentanone, la 2-naphthalényl-1-éthanone, la 4-(1,1-diméthylpropyle)-1-cyclohexanone, la benzyle acétone, ou un aldéhyde saturé, insaturé, linéaire ou ramifié de C₆ à C₁₃.

17. Utilisation selon l'une des revendications 13 à 16, **caractérisée en ce que** le composé odorant contenant une fonction oléfine dont est dérivé R'* dans la formule (III) est le linalool, le myrcène, le myrcénol, un 1,3,5-undecatriène, le 9-décèn-1-ol, ou l'heptanoate d'allyle.

18. Composition parfumante ou article parfumé, résultant d'une utilisation selon l'une des revendications 1 à 7 et 13 à 17.

19. Composition parfumante ou article parfumé selon la revendication 18 sous la forme d'un parfum ou d'une eau de Cologne, d'un gel de bain ou de douche, d'un produit de traitement des cheveux, d'une préparation cosmétique, d'un déodorant corporel, d'un déodorant d'air ambiant solide ou liquide, d'un détergent ou d'un adoucissant textile, ou d'un produit ménager.

20. Article parfumé selon la revendication 18, sous forme d'un nettoyant multi-usages ou d'un nettoyant ménager multi-usages, d'un nettoyant pour vitres, d'un produit astiquant pour meuble, d'un produit traitant pour textile, d'une poudre adoucissante ou lavante, d'une tablette liquide ou solide, d'un shampoing, d'un après-shampoing, d'un après-shampoing traitant, ou d'un spray pour les cheveux.

21. α-Céto ester de formule 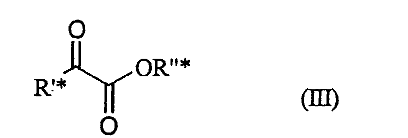 dans laquelle
R'* est un groupe alkyle ou alkylène linéaire ou ramifié, non substitué ou substitué de C₁ à C₃₅, un groupe cycloalkyle non substitué ou substitué de C₃ à C₈, un groupe phényle substitué, lesdits groupes alkyle, alkylène, cycloalkyle et phenyle pouvant comprendre un ou plusieurs hétéro atomes non directement liés au groupe α-céto et sélectionnés dans le groupe constitué de l'oxygène, l'azote, le phosphore et le soufre,
ou
R'* est un groupe alkyle substitué ou non substitué, linéaire ou ramifié, portant un hydrogène susceptible d'être arraché en position γ par rapport à la fonction α-céto et comprenant une entité dont est dérivé un composé odorant contenant une fonction oléfine, de telle façon à ce que ledit composé odorant contenant une fonction oléfine est éliminé après que ledit atome d'hydrogène en position γ a été arraché ;
R"* est un groupe méthyle, éthyle ou tert-butyle ou est la partie organique d'un alcool primaire ou secondaire dont est dérivé un aldéhyde odorant ou une cétone odorante ;
étant entendu que R'* n'est pas un groupe méthyle,
R"* n'est pas un groupe menthyle ou benzyle, et que
le (4-méthylphényl)oxoacétate de (-)-(1S,1R) 1,7,7-triméthylbicyclo[2.2.1]heptan-2-yle, le cyclohexylglyoxalate de butyle, le phénylglyoxylate de 2'-hexenyle, le phénylglyoxylate de 3'- hexenyle, le phénylglyoxylate de 5'- hexenyle, le phénylglyoxylate d'allyle, le phénylglyoxylate de 3'-méthylbut-2'ényle, le phénylglyoxylate de 4'-méthylpent-3'-ényle, le phénylglyoxylate de 1'5' diméthylhex-4'-ényle et le (cyclohexyl)oxoacetate d'hexyle sont exclus.

22. α-Céto ester selon la revendication 21 **caractérisé en ce que** R"* est la partie organique d'un alcool primaire ou secondaire dont est dérivé un aldéhyde odorant ou une cétone odorante et **caractérisé en ce que** R'* est un groupe cyclohexyle, un groupe cyclopentyle, ou un groupe alkyle linéaire ou ramifié de C₁ à C₄, à l'exception du groupe n-butyle.

23. α-Céto ester selon la revendication 22, **caractérisé en ce que** le groupe alkyle est un groupe méthyle, éthyle ou isopropyle.

24. α-Céto ester selon l'une des revendications 21 à 23, **caractérisé en ce que** l'aldéhyde odorant ou la cétone odorante dont est dérivé l'alcool primaire ou secondaire dont la partie organique R"* est présente dans l'α-céto ester tel que défini à la formule (III) est le citronellal, le citral, l'hydroxycitronellal, le dihydrojasmonate de méthyle, la 4-(4-hydroxy-1-phényl)-2-butanone, le [3-(4-tert-butylphényl)-2-méthylpropanal], l'ortho- ou para-anisaldéhyde, la menthone, la 2-pentyl-1-cyclopentanone, la 2-naphthalényl-1-éthanone, la 4-(1,1-diméthyl)-1-cyclohexanone, la benzyle acétone ou un aldéhyde, saturé, insaturé, linéaire ou ramifié de C₆ à C₁₃.

25. α-Céto ester selon l'une des revendications 21 à 24, **caractérisé en ce que** le composé odorant contenant une fonction oléfine dont est dérivé R'* dans la formule (III) est le linalool, le myrcène, le myrcénol, un 1,3,5-undécatriène, le 9-décèn-1-ol, ou l'heptanoate d'allyle.
